# EUROPEAN PATENT APPLICATION

(11) **EP 4 186 526 A1**
(43) Date of publication of application: **31.05.2023**
(21) Application number: 21820949.2
(22) Date of filing: 07.06.2021
(51) Int. Cl.: A61K 45/06, A61K 31/506, A61K 31/517, A61K 31/5377, A61K 39/395, A61P 15/00, A61P 35/00, A61P 37/02, A61P 43/00, C07K 16/30, C07K 16/40, C12N 1/15, C12N 1/19, C12N 1/21, C12N 5/10, C12N 15/13, C12N 15/63

(54) **AGENT FOR REVERSING RESISTANCE TO ANTICANCER DRUGS**

(30) Priority: 08.06.2020 JP 2020099449; 02.04.2021 JP 2021063781
(71) Applicant: Y'S AC Co., Ltd., Tokyo 150-0021 (JP); Juntendo Educational Foundation, Tokyo 113-8421 (JP)
(72) Inventor: MORIMOTO, Chikao, Tokyo 156-0043 (JP); OHNUMA, Kei, Tokyo 141-0032 (JP); HATANO, Ryo, Yokohama-shi, Kanagawa 220-0042 (JP); ITOH, Takumi, Nishitokyo-shi, Tokyo 188-0004 (JP); KANEKO, Yutaro, Tokyo 150-0021 (JP)
(74) Representative: Barker Brettell LLP
(86) International application number: PCT/JP2021/021576
(87) International publication number: WO 2021/251340

(57) **Abstract**

Provided is the present invention focusing on roles of IL-26 present in humans but not in mice by characterizing molecular mechanisms involved in interspecies differences in terms of efficacy of EGFR-TKI. The present inventors found that human cancers are exposed to IL-26 in a tumor microenvironment (TME), activating the EGFR-TKI bypass pathway, while suppression of IL-26 overcomes EGFR-TKI resistance in cancers. Furthermore, the present inventors identified EphA3 as a new functional receptor for IL-26 of TNBC. Therefore, the present invention provides a pharmaceutical composition for use in combination with an anticancer drug for treating cancer in a patient, the pharmaceutical composition containing a drug that reverses resistance to the anticancer drug by IL-26 as an active ingredient. Furthermore, the present invention provides a humanized anti-IL-26 antibody, and a therapeutic or preventive agent for refractory immune disorders or cancer using the antibody.

## Description

### CROSS-REFERENCE TO RELATED APPLICATION

This international application claims priority based on Japanese Patent Application No. 2020-099449 filed with the Japanese Patent Office on June 8, 2020 and Japanese Patent Application No. 2021-063781 filed with the Japanese Patent Office on April 2, 2021, the entire contents of Japanese Patent Application No. 2020-099449 and Japanese Patent Application No. 2021-063781 are incorporated in the present description by reference.

### TECHNICAL FIELD

The present invention relates to a technical field of reversing resistance to anticancer drugs in cancer patients.

### BACKGROUND ART

IL-26 was first identified as AK155 in Herpes saimiri infected T-cells (Non-Patent Document 1). Human IL-26, a member of the IL-10 cytokine family, is produced mainly by Th1, Th17, or NK cells, and regulates T-cells, monocytes, NK cells, synoviocytes, fibroblasts, and bacterial pathogens in inflammatory lesions (Non-Patent Documents 2 to 6). IL-26 is composed of 171 amino acids with a calculated molecular weight of about 19 kDa, and exists as a 36 kDa homodimer in human serum (Non-Patent Document 1).

It has been previously reported by the present inventors that IL-26 produced and secreted by CD4+ effector T-cells activates fibroblasts for collagen production via its functional receptor IL-20RA/IL-10RB, and plays an important role in development of chronic graft-versus-host disease (cGVHD) pulmonary fibrosis (Non-Patent Document 4).

Recently, it has been shown by the present inventors that IL-26 act directly on endothelial cells and have a profound effect on angiogenesis and immune cell recruitment in inflammatory lesions of the skin, thereby inducing exacerbation of inflammation in inflammatory skin lesions including psoriasis (Patent Document 1, Non-Patent Documents 5 and 7). Other reports indicate that IL-26 activated keratinocytes in atopic dermatitis and was exacerbated by increased production of Th17 and Th2-related cytokines in keratinocytes (Non-Patent Document 8). IL-26 have also been reported to have antibacterial effects by stimulation of various immune cells and killing of bacteria by membrane pore formation (Non-Patent Document 4). Therefore, it has been suggested that IL-26 plays an important role as a mediator of local inflammation.

Furthermore, a recent report suggests that IL-26 is involved in cancer biology including gastric cancer and hepatocellular carcinoma. In gastric cancer, IL-26 promoted cell proliferation and survival through activation of STAT1 and STAT3 signalings, which are downstream pathways of the IL-20RA/IL-10RB receptor (Non-Patent Document 9). It has been reported that an expression level of IL-26 is a poor prognostic factor in hepatocellular carcinoma patients who have undergone surgical resection (Non-Patent Document 10). In pancreatic cancer, it has been reported that exosome miR-3607-3p secreted from NK cells suppressed the development of cancer and malignant transformation by targeting IL-26 (Non-Patent Document 11). However, little is known about the function of IL-26 in breast cancer.

Importantly, human IL-26 is conserved in several vertebrate species but has not been found in rodents including mice and rats (Non-Patent Documents 2 and 12).

Previous reports have shown that IL-26 binds to the cell-surface heterodimeric receptor IL-20RA/IL-10RB, leading to downstream signaling cascade events via STAT3 phosphorylation (Non-Patent Documents 13 and 14).

Human ephrin type-A receptor 3 (EphA3) is a 110 kDa protein composed of 983 amino acids, and belongs to the Ephrin receptor subfamily of the Eph receptor tyrosine kinase (RTK) family. A structure of EphA3 is well conserved between humans and mice, with about 96% homology and 88% similarity. EphA3 is composed of an N-terminal globular ligand-binding domain in the extracellular region, a regulatory juxtamembrane domain having two tyrosine residues, a tyrosine kinase domain, and a PDZ-binding motif in the intracellular cytoplasmic region (Non-Patent Document 15). EphA3 regulates cell-cell interactions and plays a role in development and tissue formation in living bodies (Non-Patent Document 16). EphA3 was first identified as a cell surface antigen of pre-B lymphoblastic leukemia cell line (Non-Patent Document 17) and was found to be an antigen of melanoma cells identified by lytic CD4+ T-cells (Non-Patent Document 18).

Previous reports have shown that the preferential binding ligand for EphA3 is Ephrin A5 (Non-Patent Documents 15 and 19). In a downstream signaling of EphA3, membrane-bound Ephrin A5 induces clustering and tyrosine phosphorylation of EphA3, followed by activation of GTPase or ERK and dephosphorylation of AKT, leading to reorganization of cytoskeleton, cell retraction, and differentiation (Non-Patent Documents 20 to 24). In contrast, binding of unbound soluble ligand to EphA3 causes dephosphorylation of EphA3 by recruitment of protein tyrosine phosphatases and auto-inhibition of their RTK domains, leading to cell growth and proliferation (Non-Patent Documents 15, 25, and 26).

EphA3 is overexpressed in various tumor cells and is involved in maintenance of tumor initiating cells in glioblastoma and leukemia (Non-Patent Documents 16 and 20). Frequent somatic mutations of EphA3 as a receptor tyrosine kinase have been shown in various metastatic cancers (Non-Patent Document 27). Re-expression of wild-type EphA3 in a human lung cancer line including mutant EphA3 leads to a decrease in AKT phosphorylation and leads to tumor apoptosis and growth inhibition (Non-Patent Document 28). Furthermore, a decrease in EphA3 levels leads to suppression of cell proliferation in glioblastoma multiforme (Non-Patent Documents 29 and 30). In breast cancer, it has been reported that an expression level of EphA3 varies depending on the stage, and EphA3 is highly expressed in lymph node metastasis but is not expressed in a primary tumor (Non-Patent Document 31). However, little is known about potential roles of EphA3 in pathophysiology of breast cancer, including breast cancer without expression of estrogen receptor (ER), progesterone receptor (PR), and human epidermal growth factor receptor 2 (HER2) (Non-Patent Document 32).

A subtype of breast cancer without expression of ER, PR, and HER2 has been classified as triple negative breast cancer (TNBC) (Non-Patent Document 33). TNBC accounts for 15 to 20% of breast cancer cases, has low responsiveness to various anticancer drugs or a short reaction period, has a very poor prognosis, and is the most invasive subtype (Non-Patent Documents 34 to 36). Although many molecular targeted therapies have been developed against breast cancer, they are generally not effective against TNBC (Non-Patent Document 37). Signaling via the epidermal growth factor receptor (EGFR) is activated in various cancers, such as non-small cell lung cancer (NSCLC) and colorectal cancer (CRC). Anti-EGFR therapies such as EGFR tyrosine kinase inhibitors (EGFR-TKIs) have been successful in treating these cancers (Non-Patent Document 38). On the other hand, EGFR is highly expressed in most TNBC, and a significant effect of EGFR-TKI on TNBC has been shown in a non-clinical model (Non-Patent Documents 39 and 40), but the result of a clinical trial of EGFR-TKI on TNBC was unexpected (Non-Patent Documents 41 to 43).

### PRIOR ART DOCUMENTS

### PATENT DOCUMENTS

Patent Document 1: Japanese Unexamined Patent Application Publication No. 2019 -99492

### NON-PATENT DOCUMENTS

Non-Patent Document 1: Knappe A et al., J Virol 2000, 74(8): 3881-3887.
Non-Patent Document 2: Fickenscher H et al., Int Immunopharmacol 2004, 4(5): 609-613.
Non-Patent Document 3: Stephen-Victor E et al., PLoS Pathog 2016, 12(6): e 1005624.
Non-Patent Document 4: Ohnuma K et al., J Immunol 2015, 194(8): 3697-3712.
Non-Patent Document 5: Itoh T et al., J Invest Dermatol 2019, 139(4): 878-889.
Non-Patent Document 6: Collins PL et al., J Immunol 2010, 185(3): 1492-1501.
Non-Patent Document 7: Ohnuma K et al., Crit Rev Immunol 2016, 36(3): 239-267.
Non-Patent Document 8: Kamijo H et al., J Invest Dermatol 2020, 140(3): 636-644 e632.
Non-Patent Document 9: You W et al., PLoS One 2013, 8(5): e63588.
Non-Patent Document 10: Xi ZF et al., 2019, 18(3): 242-248.
Non-Patent Document 11: Sun H et al., Front Immunol 2019, 10(2819): 2819.
Non-Patent Document 12: Donnelly RP et al., Cytokine Growth Factor Rev 2010, 21(5): 393-401.
Non-Patent Document 13: Hor S et al., J Biol Chem 2004, 279(32): 33343-33351.
Non-Patent Document 14: Sheikh F et al., J Immunol 2004,172(4): 2006-2010.
Non-Patent Document 15: Janes PW, Atlas of Genetics and Cytogenetics in Oncology and Haematology 2017(5).
Non-Patent Document 16: Boyd AW et al., Nat Rev Drug Discov 2014, 13(1): 39-62.
Non-Patent Document 17: Boyd AW et al., J Biol Chem 1992, 267(5): 3262-3267.
Non-Patent Document 18: Chiari R et al., Cancer Res 2000, 60(17): 4855-4863.
Non-Patent Document 19: Lackmann M et al., Proc Natl Acad Sci USA 1996, 93(6): 2523-2527.
Non-Patent Document 20: Janes PW et al., Growth Factors 2014, 32(6): 176-189.
Non-Patent Document 21: Davis S et al., Science 1994, 266(5186): 816-819.
Non-Patent Document 22: Lackmann M et al., J Biol Chem 1998, 273(32): 20228-20237.
Non-Patent Document 23: Clifford N et al., J Cell Biochem 2008, 105(5): 1250-1259.
Non-Patent Document 24: Aoki M et al., J Biol Chem 2004, 279(31): 32643-32650.
Non-Patent Document 25: Pasquale EB, Nat Rev Cancer 2010, 10(3): 165-180.
Non-Patent Document 26: Nievergall E et al., J Cell Biol 2010,191(6): 1189-1203.
Non-Patent Document 27: Lisabeth EM et al., Biochemistry 2012, 51(7): 1464-1475.
Non-Patent Document 28: Zhuang G et al., J Natl Cancer Inst 2012, 104(15): 1182-1197.
Non-Patent Document 29: Day BW et al., Cancer Cell 2013, 23(2): 238-248.
Non-Patent Document 30: Qazi MA et al., Cancer Res 2018, 78(17): 5023-5037.
Non-Patent Document 31: Vecchi M et al., Oncogene 2008, 27(15): 2148-2158.
Non-Patent Document 32: Kou CJ et al., Biomed Res Int 2018, 2018: 7390104.
Non-Patent Document 33: Garrido-Castro AC et al., Cancer Discov 2019, 9(2): 176-198.
Non-Patent Document 34: Dent R et al., Clin Cancer Res 2007, 13(15 Pt 1): 4429-4434.
Non-Patent Document 35: Foulkes WD et al., N Engl J Med 2010, 363(20): 1938-1948.
Non-Patent Document 36: Bianchini G et al., Nat Rev Clin Oncol 2016, 13(11): 674-690.
Non-Patent Document 37: Nakhjavani M et al., J Breast Cancer 2019, 22(3): 341-361.
Non-Patent Document 38: Ciardiello F et al., N Engl J Med 2008, 358(11): 1160-1174.
Non-Patent Document 39: Liang YJ et al., Oncotarget 2017, 8(29): 47454-47473.
Non-Patent Document 40: McLaughlin RP et al., Breast Cancer Res 2019, 21(1): 77.
Non-Patent Document 41: Nakai K et al., Am J Cancer Res 2016, 6(8): 1609-1623.
Non-Patent Document 42: Costa R et al., Cancer Treat Rev 2017, 53: 111-119.
Non-Patent Document 43: Ali R et al., Signal Transduct Target Ther 2017, 2(1): 16042.

### SUMMARY OF THE INVENTION

The precise molecular mechanism of action of IL-26 in cancer biology, including identification of functional receptors for IL-26 in cancer cells and downstream signaling phenomena thereof, has not yet been elucidated. Furthermore, due to a lack of IL-26 in mice and rats, a lack of a suitable mouse non-clinical model has prevented the understanding of the roles thereof in cancer biology of IL-26. The present inventors were able to investigate the role of IL-26 in EGFR-TKI-resistant TNBC by using hIL-26Tg mice.

Understanding the molecular mechanism involved in the observed differences for the efficacy of EGFR-TKI in TNBC between humans and mice may lead to therapeutic approaches to overcome EGFR-TKI resistance in TNBC. The present inventors have shown that IL-26, which is not present in rodents but is present in humans, plays an important role in cell proliferation of EGFR-TKI-treated TNBC via activation of an EGFR bypass pathway. The interspecies difference in IL-26 expression has prevented development of suitable mouse models to assess unknown problems such as EGFR-TKI sensitivity and TNBC resistance. To address this important problem, the present inventors have successfully defined a molecular mechanism involved in resistance via IL-26 to EGFR-TKI and established that IL-26 is an appropriate therapeutic target for TNBC. In other words, by revealing the mechanism involved in EGFR-TKI resistance via IL-26 in TNBC, the contradiction in the interspecies differences between the mouse model and human subjects observed in EGFR-TKI treatment of TNBC was resolved.

While a sensitivity of EGFR-TKI is closely related to the status of EGFR gene mutation in NSCLS (Lynch TJ et al., N Engl J Med 2004, 350(21): 2129-2139.), it is considered that activating mutations in EGFR gene are not clinically relevant in TNBC (Nakai K et al. (2016) supra, Costa R et al. (2017) supra). The cell lines used in the studies described in the present description constitutively expressed EGFR without EGFR gene mutations, indicating that EGFR-TKI resistance via IL-26 in TNBC is not associated with specific gene mutations of EGFR. In addition, the present inventors have found that IL-26 is secreted by tumor-infiltrating immune cells including CD4+ T-cells and macrophages.

Furthermore, the present inventors have found that exogenous IL-26 inhibits EGFR-TKI-induced suppression of cell proliferation and TNBC growth. The present inventors have found that blocking of IL-26 by anti-IL-26 monoclonal antibody (mAb) abolished IL-26 dependent EGFR-TKI resistance of TNBC. The present inventors have newly found EphA3 as a novel receptor for IL-26 of TNBC, and have found that its involvement in the induction of a downstream signaling cascade of AKT and JNK phosphorylation promotes cell proliferation even under EGFR-TKI treatment. Immunohistochemical assays of TNBC tissue samples have revealed that IL-26 is mainly expressed in TILs and that EphA3 is detected in tumor cells. These findings provide new information on the mechanism of EGF-TKI resistance via the IL-26-EphA3 axis in TNBC, indicating that IL-26 is expected as a new therapeutic target for TNBC.

The TNBC cell lines used in the studies described in the present description did not express IL-20RA, suggesting that there may be IL-26 receptors other than IL-20RA/IL-10RB in TNBC cells. The present inventors have identified EphA3 as a new functional receptor for IL-26 in TNBC.

In the experiments described in the present description, exogenous soluble IL-26 induced dephosphorylation of EphA3 in TNBC and then phosphorylation of JNK and AKT, thereby inducing TNBC proliferation even under EGFR-TKI treatment conditions. Although EphA3 related molecular alterations were associated with IL-26 alone, IL-26 separate treatment did not alter TNBC tumor growth. Rather, the effect of IL-26 on TNBC tumor growth has been seen under conditions of EGFR-TKI exposure. These findings indicate that IL-26 is a new functional ligand of EphA3 that can regulate cell growth and proliferation in TNBC. Furthermore, TNBC tumor suppression has been achieved in the presence of EGFR-TKI agents after blocking the IL-26-EphA3 interaction. Three different anti-IL-26 mAbs, clones 20-3, 31-4 and 69-10, recognizing different epitopes from each other, restored a gefitinib inhibitory effect on cell proliferation. Accordingly, it is shown that each of the anti-IL-26 mAb clones 20-3, 31-4 and 69-10 recognized the epitope of the binding domain of EphA3 to IL-26. Also, a mixture of four different anti-IL-26 mAbs clearly abolished the inhibitory effect of IL-26 on gefitinib-induced suppression of cell proliferation, the extent of which was comparable to that of anti-IL-26 pAb. These findings indicated that this mixture of 4 different anti-IL-26 mAbs fully recognized an entire epitope that is the binding domain of EphA3 to IL-26. Accordingly, it was strongly suggested that targeting IL-26-EphA3 may be an effective therapeutic strategy to overcome EGFR-TKI resistance of TNBC.

Furthermore, a major side effect associated with EGFR-TKI antitumor therapy is skin toxicity (Giovannini M et al., J Oncol 2009, 2009:849051.). Inhibition of EGFR signaling by TKIs causes early differentiation and induces inflammation and apoptosis, skin atrophy, telangiectasia and photosensitivity in the dermal and epidermal regions (Lacouture ME, Nat Rev Cancer 2006, 6(10): 803-812.). The present inventors have recently shown that IL-26 plays an important role in angiogenesis and leukocyte recruitment. By controlling excessive angiogenesis observed in inflammatory skin lesions by the anti-IL-26 mAb and neutralizing IL-26, skin inflammatory processes such as psoriasis and irritable dermatitis were reduced (Itoh T et al., (2019) supra, Hatano R et al. (2019) supra). From these findings, anti-IL-26 therapy in combination with EGFR-TKI may effectively suppress TNBC proliferation and reduce skin toxicity.

The present invention has been achieved in a way of focusing on roles of IL-26 present in humans but not in mice by characterizing the molecular mechanisms involved in the interspecies differences in the efficacy of EGFR-TKI in TNBC. The present inventors have found that human TNBC are exposed to IL-26 in the tumor microenvironment (TME), activating EGFR-TKI bypass pathway, while suppression of IL-26 overcomes EGFR-TKI resistance in TNBC. This was further confirmed by a tumor-bearing mouse model utilizing human IL-26 transgenic (hIL-26Tg) mice. Furthermore, the present inventors identified EphA3 as a new functional receptor for IL-26 of TNBC.

### EFFECTS OF THE INVENTION

The present invention demonstrates the importance of IL-26 for TNBC EGFR-TKI resistance, and provides a new cancer therapeutic strategy including inhibition of interaction between IL-26 and EphA3 and a combination of EGFR-targeted agents. In particular, the present invention contributes to the realization of treatment of EGFR-TKI-resistant cancer.

### BRIEF DESCRIPTION OF THE DRAWINGS

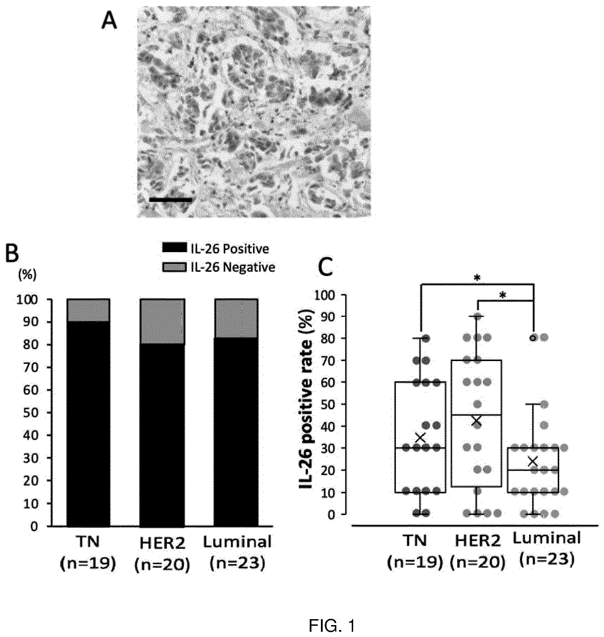

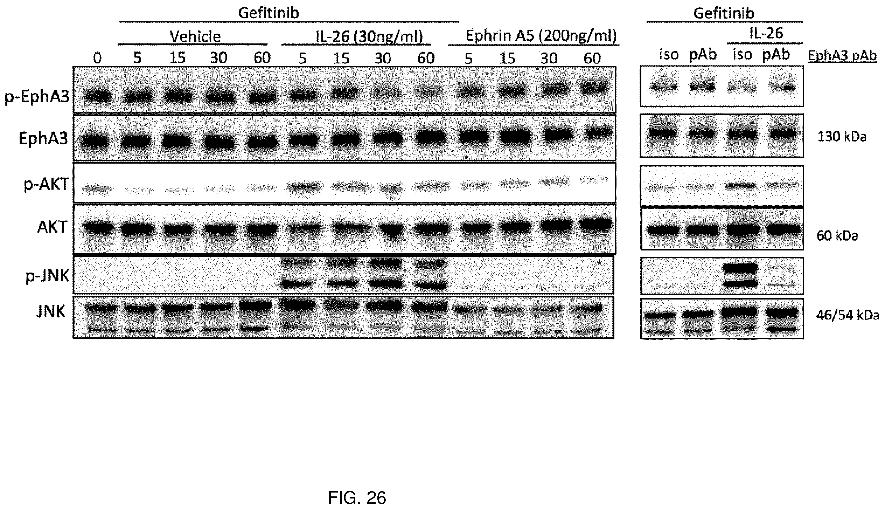

FIG. 1A is a representative image of TNBC cases with high expression of IL-26 protein among results of staining TNBC tissue specimens with anti-human IL-26 mAb (n=19). All immunohistochemically stained specimens were counterstained with hematoxylin. IL-26 was detected in tumor-infiltrating lymphocytes. Original magnification, 100x. A scale bar represents 50 µm. FIG. 1B and FIG. 1C are both subtypes of breast cancer tissue specimens that were stained with anti-human IL-26 mAb (TNBC (n=19), HER2 (n=20), and Luminal (n=23)). The IL-26 stained in the cytoplasm of the stromal immune cells were semi-quantitatively measured in increments of 10%. A proportion of IL-26 positive in each subtype of breast cancer was significantly higher than a proportion of IL-26 negative (FIG. 1B). The proportion of IL-26 positive stromal immune cells in TNBC and HER2 subtypes was higher than in the Luminal subtype (FIG. 1C). *p < 0.05.

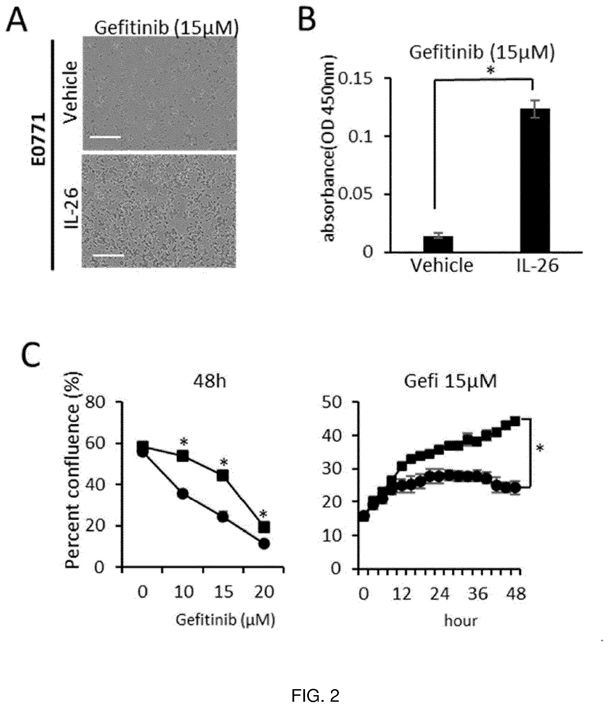

FIG. 2 shows the results of treating E0771 cells (1×10⁴) with gefitinib at various concentrations for 48 hours together with either IL-26 or a solvent control (vehicle). FIG. 2A shows a phase contrast micrograph of E0771 cells (1×10⁴) after culturing for 48 hours with either IL-26 (lower panel) or a solvent control (vehicle; upper panel) in the presence of gefitinib (15 µM). The number of cells after IL-26 treatment was clearly increased (lower panel). 100x. Scale bar = 100 µm. The data shown is a representative image of five independent experiments showing similar results. FIG. 2B is a graph showing the results of an MTT assay performed in the experiment of FIG. 2A. A vertical axis represents absorbance (OD450 nm) at 450 nm. Cell proliferation was significantly increased due to IL-26 treatment. Data was expressed as a mean value ± S.D. of triplicate experiments. *P < 0.001. FIG. 2C is a graph showing the results of a cell proliferation assay of the experiment of FIG. 2A. The vertical axis represents a percent confluence of cells (%). Black squares indicate the results of the control group, and black circles indicate the results of the IL-26 administration group. Data was expressed as a mean value ± S.D. of triplicate experiments. Left graph in FIG. 2C shows the percent confluence of cells after 48 hours from gefitinib treatment at various concentrations. The horizontal axis represents a gefitinib concentration (µM). IL-26 treatment significantly increased cell proliferation in the presence of gefitinib at various concentrations (10, 15, or 20 µM) (black squares) (*P < 0.001). Right graph in FIG. 2C shows changes in cell confluence up to 48 hours after gefitinib (15 µM) treatment. The horizontal axis represents the time elapsed from the addition of gefitinib (hour). A significant increase in cell proliferation with gefitinib of 15 µM and IL-26 treatment was observed over time (*P < 0.001) (black squares).

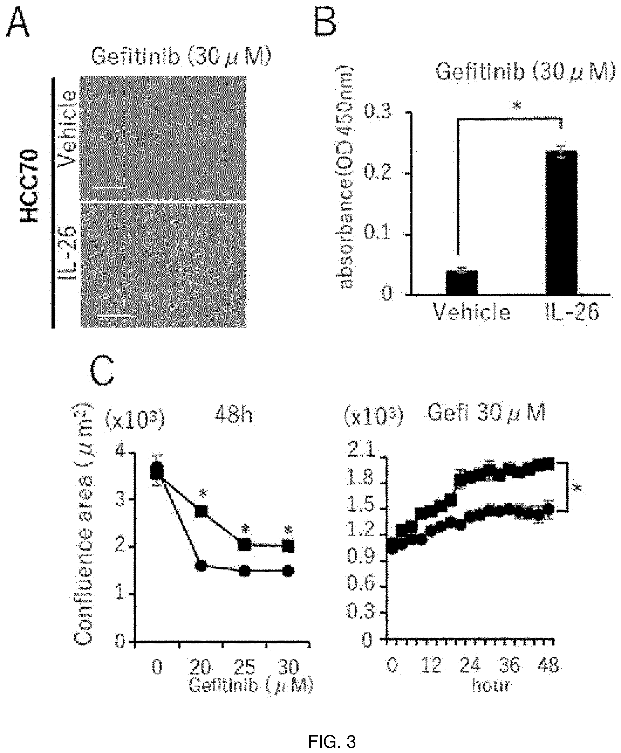

FIG. 3 shows the results of treating HCC70 cells with gefitinib at various concentrations for 48 hours together with either IL-26 or a solvent control (vehicle). FIG. 3A shows a phase contrast micrograph of HCC70 cells after culturing for 48 hours with either IL-26 (lower panel) or a solvent control (vehicle; upper panel) in the presence of gefitinib (30 µM). The number of cells after IL-26 treatment was clearly increased (lower panel). 100x. Scale bar = 100 µm. The data shown is a representative image of five independent experiments showing similar results. FIG. 3B is a graph showing the results of an MTT assay performed in the experiment of FIG. 3A. The vertical axis represents absorbance (OD450 nm) at 450 nm. Cell proliferation was significantly increased by IL-26 treatment (*P < 0.001). Data was expressed as a mean value ± S.D. of triplicate experiments. FIG. 3C is a graph showing the results of the cell proliferation assay of the experiment of FIG. 3A. The vertical axis represents the percent confluence of cells (%). The black circles indicate the results of the control group, and black squares indicate the results of the IL-26 administration group. Data was expressed as a mean value ± S.D. of triplicate experiments. Left graph in FIG. 3C shows the percent confluence of cells after 48 hours from gefitinib treatment at various concentrations. The horizontal axis represents the gefitinib concentration (µM). IL-26 treatment significantly increased the cell proliferation in the presence of gefitinib at various concentrations (20, 25, or 30 µM) (black squares) (*P < 0.001). Right graph in FIG. 3C shows changes in cell confluence up to 48 hours after gefitinib (30 µM) treatment. The horizontal axis represents the time elapsed from the addition of gefitinib (hour). A significant increase in cell proliferation after gefitinib of 30 µM and IL-26 treatment was observed over time (*P < 0.001) (black squares).

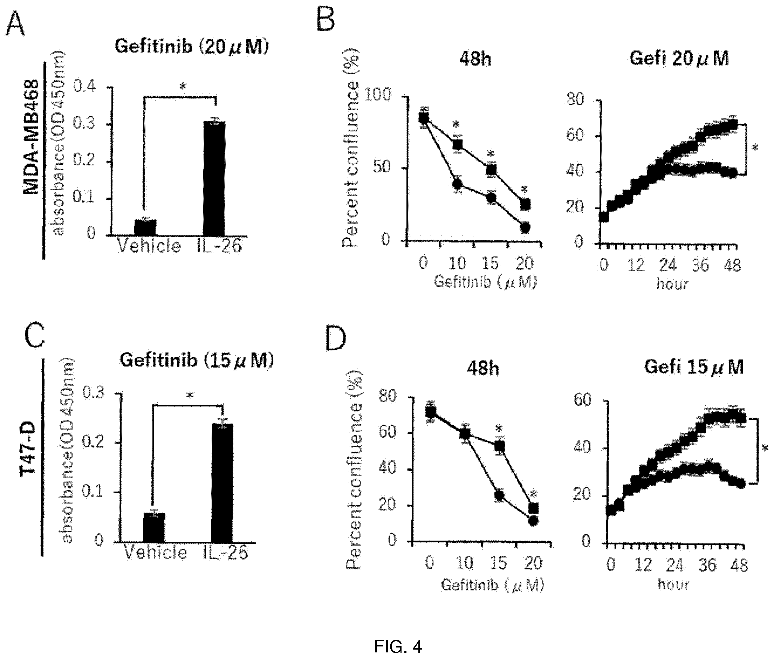

FIG. 4 shows the results of treating MDA-MB468 cells (A and B) or T47D cells (C and D) with gefitinib at various concentrations for 48 hours together with either IL-26 or a solvent control (vehicle). Data was expressed as a mean value ± S.D. of triplicate experiments. *P < 0.001. FIG. 4A is a graph showing the results of an MTT assay after culturing MDA-MB468 cells together with either IL-26 or a solvent control (vehicle) in the presence of gefitinib for 48 hours. The vertical axis represents absorbance (OD450 nm) at 450 nm. Cell proliferation was significantly increased by IL-26 treatment. FIG. 4B is a graph showing the results of the cell proliferation assay in the experiment of FIG. 4A. The vertical axis represents the percent confluence of cells (%). The black circles indicate the results of the control group, and black squares indicate the results of the IL-26 administration group. Left graph in FIG. 4B shows the percent confluence of cells after 48 hours from gefitinib treatment at various concentrations. The horizontal axis represents the gefitinib concentration (µM). IL-26 treatment significantly increased MDA-MB468 cell proliferation in the presence of gefitinib at various concentrations (10, 15, or 20 µM) (black squares) (*P < 0.001). Right graph in FIG. 4B shows changes in cell confluence up to 48 hours after gefitinib (30 µM) treatment. The horizontal axis represents the time elapsed from the addition of gefitinib (hour). A significant increase in cell proliferation after gefitinib of 20 µM and IL-26 treatment was observed over time (*P < 0.001) (black squares). FIG. 4C shows the results of the MTT assay following incubation of T47D cells with gefitinib in the presence of IL-26 or a solvent control (vehicle) for 48 hours. Cell proliferation was significantly increased after IL-26 treatment (*P < 0.001). FIG. 4D is a graph showing the results of the cell proliferation assay of the experiment of FIG. 4C. The vertical axis represents the percent confluence of cells (%). The black circles indicate the results of the control group, and black squares indicate the results of the IL-26 administration group. Left graph in FIG. 4D shows the percent confluence of cells after 48 hours from gefitinib treatment at various concentrations. The horizontal axis represents the gefitinib concentration (µM). IL-26 treatment significantly increased T47D cell proliferation in the presence of gefitinib at various concentrations (10, 15, or 20 µM) (black squares) (*P < 0.001). Right graph in FIG. 4D shows changes in cell confluence up to 48 hours after gefitinib (30 µM) treatment. The horizontal axis represents the time elapsed from the addition of gefitinib (hour). A significant increase in cell proliferation after gefitinib of 15 µM and IL-26 treatment was observed over time (*P < 0.001) (black squares).

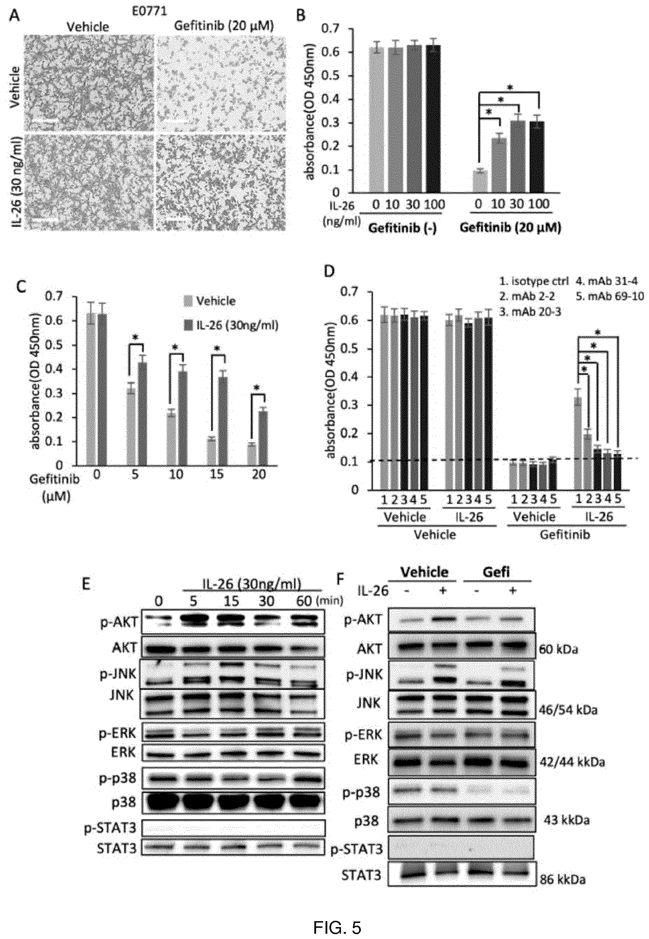

FIG. 5A shows a phase contrast micrograph of E0771 cells (1×10⁴) after culturing for 48 hours with IL-26 or a control vehicle in the presence or absence of gefitinib. Original magnification, 100x. The scale bar represents 100 µm. The data is a representative image of five independent experiments showing similar results. FIG. 5B is a graph showing the results of the MTT assay of E0771 cells treated with the indicated dose of IL-26 for 48 hours in the presence or absence of gefitinib (20 µM). IL-26 dose dependently enhanced the proliferation of E0771 cells in the presence of gefitinib. *p < 0.01. Representative data from five independent experiments was presented as a mean value ± standard deviation of triplicate samples. Similar results were obtained in each experiment. FIG. 5C is a graph showing the results of the MTT assay of IL-26 (30 ng/ml) treated E0771 cells in the presence of gefitinib at varying doses (5, 10, 15, or 20 µM). IL-26 significantly inhibited the suppression of cell proliferation by gefitinib (*p < 0.01), and this phenomenon was significantly observed with gefitinib of 15 µM or 20 µM. Representative data from three independent experiments was presented as a mean value ± standard deviation of triplicate samples. Similar results were obtained in each experiment. FIG. 5D is a graph showing the results of the MTT assay of E0771 cells treated with IL-26 (30 ng/ml) and/or gefitinib (20 µM) for 48 hours in the presence of an anti-IL-26 neutralizing mAb or isotype control mouse IgG (20 µg/ml each). Broken lines indicate reference values when gefitinib and the vehicle were added. The blocking of IL-26 with anti-IL-26 mAb clearly reversed the inhibitory effect of IL-26 on gefitinib-induced suppression of cell proliferation. Representative data from three independent experiments was presented as a mean value ± standard deviation of triplicate samples. Similar results were obtained in each experiment. FIG. 5E is a photograph showing the results of Western blot analysis using an anti-phosphorylated AKT antibody, an anti-phosphorylated JNK antibody, an anti-phosphorylated ERK antibody, an anti-phosphorylated p38 antibody, and an anti-phosphorylated STAT3 antibody after stimulating E0771 cells for the time indicated by IL-26 (30 ng/ml), and re-blotting with an anti-pan-AKT antibody, an anti-pan-JNK antibody, an anti-pan-ERK antibody, an anti-pan-p38 antibody, and an anti-pan-STAT3 antibody. The addition of IL-26 caused phosphorylation of AKT and JNK but not activation of STAT3 in E0771 cells. For the representative data from five independent experiments, similar results were obtained in each experiment. FIG. 5F is a photograph showing the results of Western blot analysis after stimulating E0771 cells with IL-26 (30 ng/ml) for 15 minutes in the presence or absence of gefitinib (20 µM). IL-26 activated the AKT and JNK signals of E0771 cells eve in the presence of gefitinib. For the representative data from five independent experiments, similar results were obtained in each experiment.

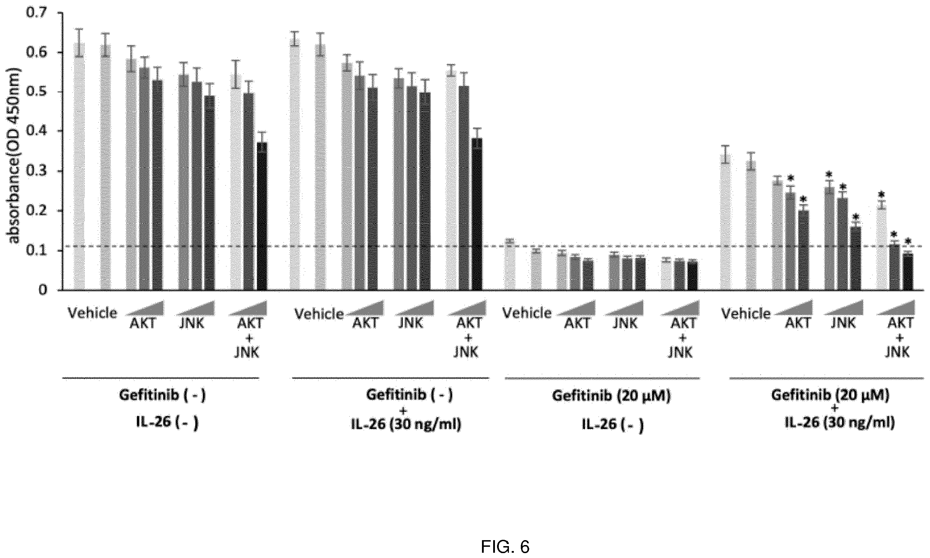

FIG. 6 is a graph showing the results of the MTT assay of E0771 cells treated with IL-26 and/or gefitinib for 48 hours in the presence or absence of a signal inhibitor (an AKT inhibitor, a JNK inhibitor, or a combination of AKT inhibitor and JNK inhibitor) at various concentrations. The broken lines indicate the reference values to which gefitinib and the vehicle were added. Both the AKT inhibitor and the JNK inhibitor partially inhibited the proliferation of E0771 cells in the absence of gefitinib. The AKT inhibitor and the JNK inhibitor had little effect on the proliferation of E0771 cells treated with gefitinib alone. The combination of AKT inhibitor and JNK inhibitor almost completely reversed the inhibitory effect of IL-26 on the gefitinib-induced suppression of cell proliferation, and suppressed cell proliferation to a level equivalent to that of administration of gefitinib alone in the absence of IL-26. *p < 0.01. Representative data from three independent experiments was presented as a mean value ± standard deviation of triplicate samples. Similar results were obtained in each experiment.

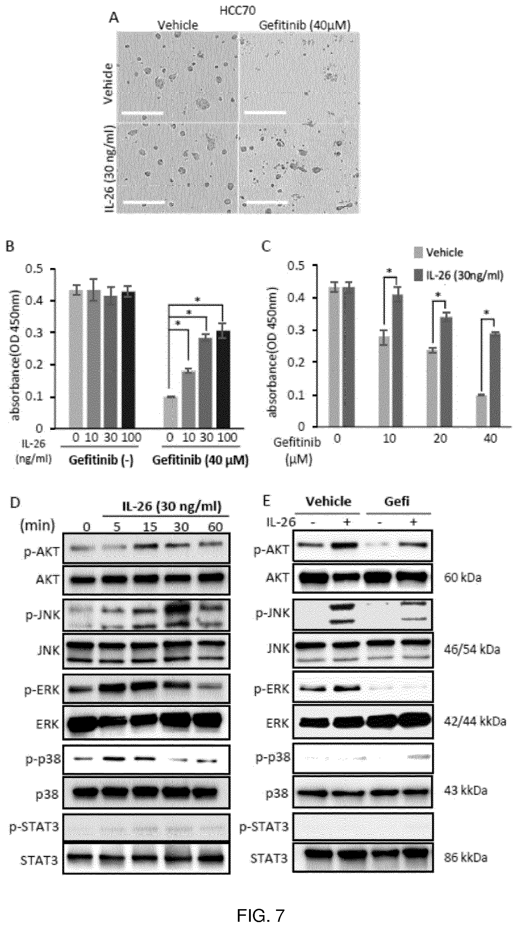

FIG. 7A shows a phase contrast micrograph of HCC70 (1×10⁴) after culturing for 48 hours with IL-26 or a control vehicle in the presence or absence of gefitinib. Original magnification, 100x. The scale bar represents 100 µm. The data is a representative image of five independent experiments showing similar results. FIG. 7B is a graph showing the results of the MTT assay of HCC70 cells treated with the indicated dose of IL-26 for 48 hours in the presence or absence of gefitinib (40 µM). IL-26 dose dependently enhanced the proliferation of HCC70 cells in the presence of gefitinib. *p < 0.01. Representative data from five independent experiments was presented as a mean value ± standard deviation of triplicate samples. Similar results were obtained in each experiment. FIG. 7C is a graph showing the results of the MTT assay of IL-26 (30 ng/ml) treated HCC70 cells in the presence of gefitinib at varying doses (10, 20, or 40 µM). IL-26 significantly inhibited the suppression of cell proliferation due to gefitinib (*p < 0.01), and this phenomenon was significantly observed with gefitinib of 40 µM. Representative data from three independent experiments was presented as a mean value ± standard deviation of triplicate samples. Similar results were obtained in each experiment. FIG. 7D is a photograph showing the results of stimulating HCC70 cells for the time indicated by IL-26 (30 ng/ml), then performing Western blot analysis using an anti-phosphorylated AKT antibody, an anti-phosphorylated JNK antibody, an anti-phosphorylated ERK antibody, an anti-phosphorylated p38 antibody, and an anti-phosphorylated STAT3 antibody, and re-blotting with an anti-pan-AKT antibody, an anti-pan-JNK antibody, an anti-pan-ERK antibody, an anti-pan-p38 antibody, and an anti-pan-STAT3 antibody. The addition of IL-26 caused the phosphorylation of AKT and JNK but not activation of STAT3 in HCC70 cells. For the representative data from five independent experiments, similar results were obtained in each experiment. FIG. 7E is a photograph showing the result of Western blot analysis after stimulating HCC70 cells with IL-26 (30 ng/ml) for 15 minutes in the presence or absence of gefitinib (40 µM). IL-26 activated the AKT and JNK signals of HCC70 cells even in the presence of gefitinib. For the representative data from five independent experiments, similar results were obtained in each experiment.

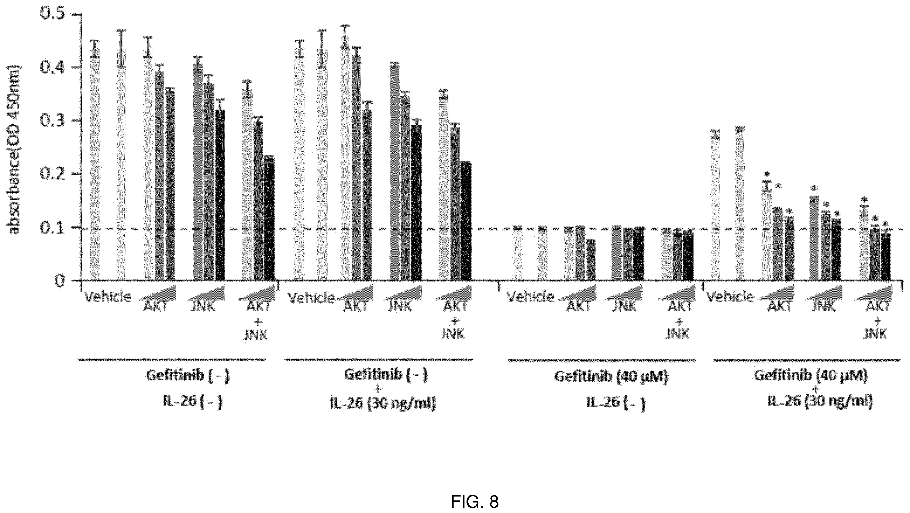

FIG. 8 is a graph showing the results of the MTT assay of HCC70 cells treated with IL-26 and/or gefitinib for 48 hours in the presence or absence of the signal inhibitor (the AKT inhibitor, the JNK inhibitor, or a combination of the AKT inhibitor and the JNK inhibitor) at various concentrations. The broken lines indicate the reference values to which gefitinib and the vehicle were added. Both the AKT inhibitor and the JNK inhibitor partially inhibited the proliferation of HCC70 cells in the absence of gefitinib. The AKT inhibitor and JNK inhibitor had little effect on the proliferation of HCC70 cells treated with gefitinib alone. The combination of AKT inhibitor and JNK inhibitor almost completely reversed the inhibitory effect of IL-26 on the gefitinib-induced suppression of cell proliferation, and suppressed cell proliferation to a level equivalent to that of administration of gefitinib alone in the absence of IL-26. *p < 0.01. Representative data from three independent experiments was presented as a mean value ± standard deviation of triplicate samples. Similar results were obtained in each experiment.

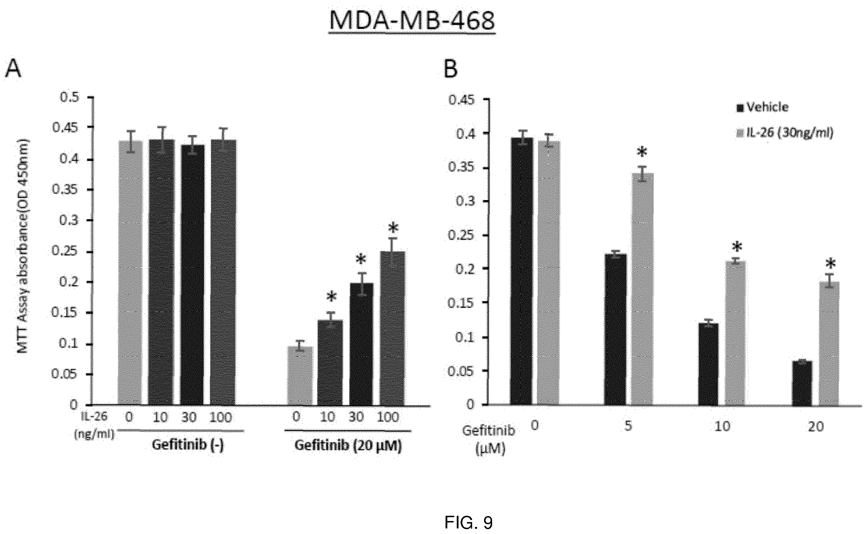

FIG. 9A is a graph showing the results of the MTT assay of MDA-MB468 cells treated with the indicated doses of IL-26 for 48 hours in the presence or absence of gefitinib (20 µM). IL-26 dose dependently enhanced the proliferation of MDA-MB468 cells in the presence of gefitinib. *p < 0.01. Representative data from three independent experiments was presented as a mean value ± standard deviation of triplicate samples. Similar results were obtained in each experiment. FIG. 9B is a graph showing the results of the MTT assay of MDA-MB468 cells treated with IL-26 (30 ng/ml) in the presence of gefitinib at varying doses (5, 10, or 20 µM). IL-26 significantly inhibited the suppression of cell proliferation by gefitinib (*p < 0.01). Representative data from three independent experiments was presented as a mean value ± standard deviation of triplicate samples. Similar results were obtained in each experiment.

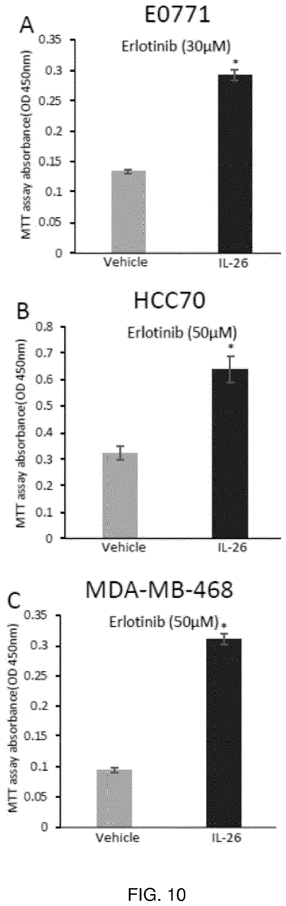

FIG. 10 confirmed that the exogenous IL-26 activated the bypass pathway of erlotinib in TNBC. It is a graph showing the results of the MTT assay after treatment of E0771 cells, HCC70 cells, or MDA-MB-468 cells with either IL-26 (30 ng/ml) or a solvent control (vehicle) for 48 hours with erlotinib of 30 µM or 50 µM. The vertical axis represents absorbance at 450 nm (MTT assay absorbance (OD450 nm)). The exogenous IL-26 inhibited the inhibitory effect of erlotinib on cell proliferation of E0771 cells, HCC70 cells, and MDA-MB-468 cells at levels similar to those observed in gefitinib. Representative data of three independent experiments with similar results is shown as a mean value ± S.D. of the triplicate experiments. *p < 0.01 was determined by one-way ANOVA and unpaired t test.

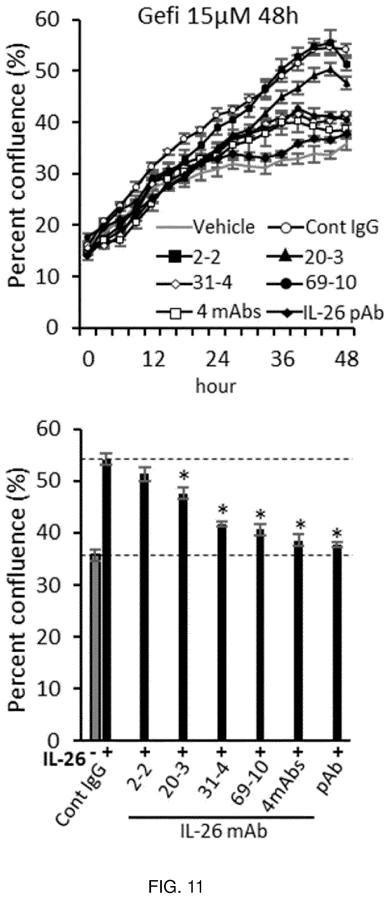

FIG. 11 shows E0771 cells that were cultured in the presence of gefitinib of 15 µM and IL-26 together with anti-IL-26 mAb (clones 2-2, 20-3, 31-4, and 69-10) alone or a mixture thereof, or control IgG (10 µg/ml) (Cont IgG). In the presence of gefitinib of 15 µM, cells to which IL-26 and antibodies were not added were used as vehicles. It is a graph showing results of cell proliferation assays. The vertical axis represents the percent confluence of cells (%). Data was expressed as a mean value ± S.D. of triplicate experiments. The upper panel shows the time series of changes in the percent confluence of cells (the horizontal axis shows the time elapsed from drug treatment (hour)). The lower panel shows the percent confluence of cells after 48 hours of culture. Three different anti-IL-26 mAbs, clones 20-3, 31-4 and 69-10, significantly reversed the effect of IL-26 on gefitinib-induced suppression of cell proliferation. Furthermore, a mixture of four different anti-IL-26 mAbs (2.5 µg/ml each) clearly abolished the IL-26 effect on gefitinib-induced suppression of cell proliferation to a comparable extent to that of anti-IL-26 pAb.

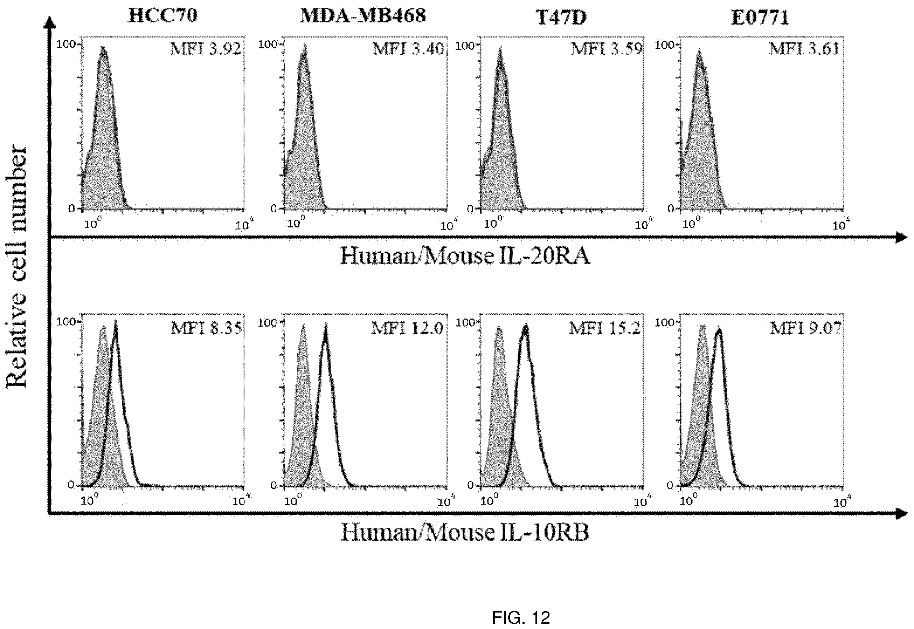

FIG. 12 shows the results of confirming that IL-20RA is not expressed in breast cancer cell lines. The results of detecting cell surface protein expression of IL-20RA and IL-10RB in human and mouse TNBC cells are shown. HCC70, MDA-MB468, T47D, or E0771 cells were stained with APC-labeled mouse anti-human IL-20RA mAb, rabbit anti-mouse IL-20RA pAb, and PE-labeled donkey anti-rabbit IgG pAb, PE-labeled mouse anti-human IL-10RB mAb, APC-labeled rat anti-mouse IL-10RB mAb, or isotype control, and analyzed by flow cytometry. The data is displayed as histograms of the IL-20RA intensity (upper diagram) and the IL-10RB intensity (lower diagram), and a gray area of each histogram indicates isotype control data. Representative histograms and representative mean fluorescence intensity (MFI) data of three independent experiments with similar results are shown.

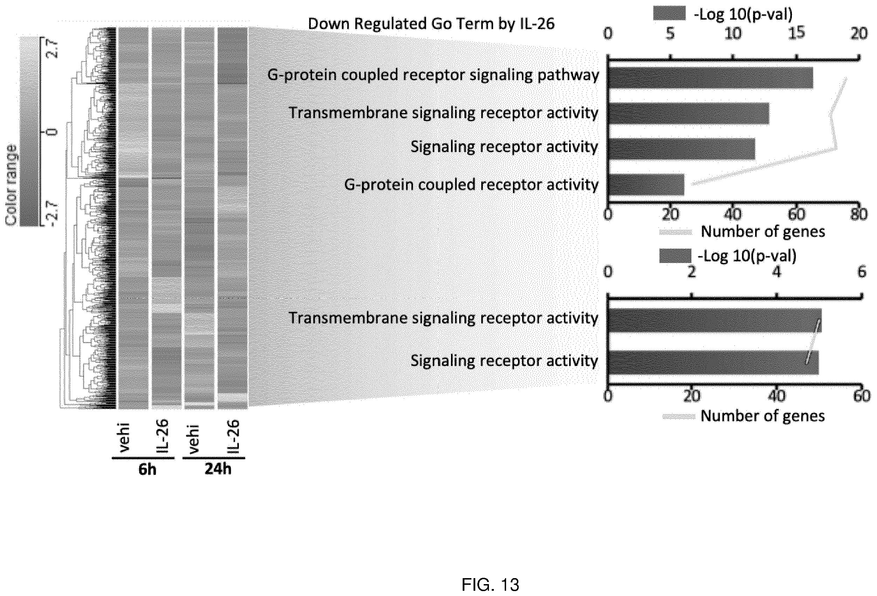

FIG. 13 shows E0771 cells that were treated with the exogenous IL-26 (30 ng/ml) or a control vehicle (vehi) for 6 or 24 hours, and total RNA was isolated and subjected to DNA microarray analysis. Left in FIG. 13 is a heat map (magnification >= 2.0) of 960 genes differentially expressed between E0771 cells treated with IL-26 and E0771 cells treated with vehicle that was constructed by a hierarchical cluster analysis using Cluster 3.0 software, and the results were displayed in the TreeView program. Right in FIG. 13 is GO terms that were greatly downregulated in IL-26 induced genes. Genes associated with transmembrane signaling receptor activity and signaling receptor activity were downregulated in E0771 cells treated with IL-26 for 6 and 24 hours. In addition, IL-26 significantly reduced the expression level of EphA3, one of the genes corresponding to these GO terms.

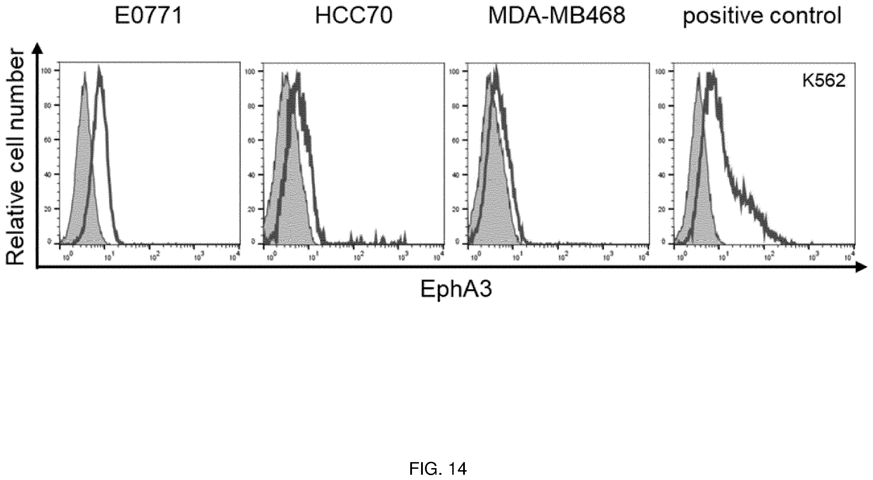

FIG. 14 shows a histogram of results of staining and measuring E0771 cells, HCC70 cells, MDA-MB468 cells, and indicated positive control cells with an antibody against EphA3. The gray area of each histogram indicates the isotype control data. Representative data of three independent experiments is shown, and similar results were obtained in each experiment.

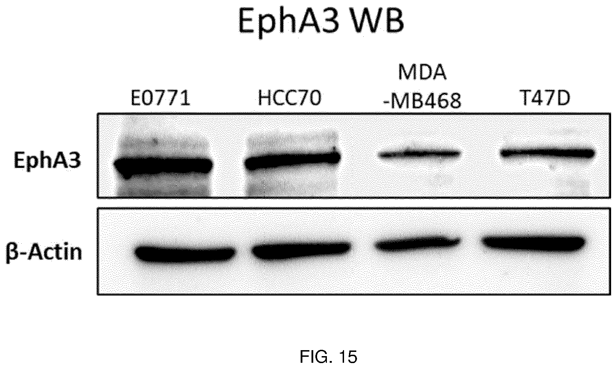

FIG. 15 shows the results of examining the expression of EphA3 in breast cancer cell lines that were used in the examples. The expression of EphA3 in breast cancer cell lines was detected by immunoblotting. A photograph of results of performing Western blot using an anti-EphA3 antibody and an anti-β-actin antibody is shown. The expression of EphA3 was confirmed in all breast cancer cell lines. The data shown indicates representative photographs of three independent experiments showing similar results.

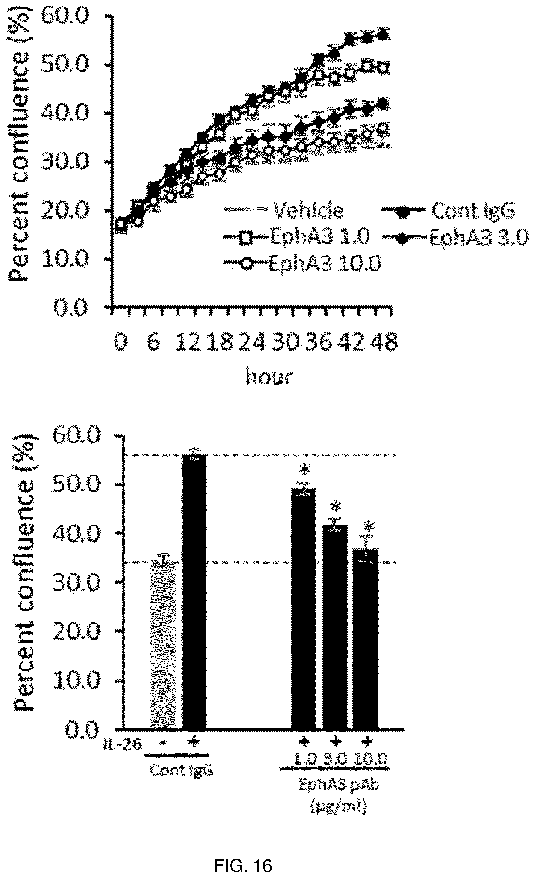

FIG. 16 shows E0771 cells that were cultured in the presence of ani-EphA3 pAb or control IgG in the presence of IL-26 and gefitinib in the same manner as in FIG. 11. The top graph shows the time series of changes in the percent confluence of cells. The vertical axis represents a percent confluence of cells (%), and the horizontal axis represents the elapsed time (hour) from drug treatment. The black circles indicate the results of the control IgG administration group, white squares, black diamonds, and white circles indicate the results of the anti-EphA3 polyclonal antibody 1.0 µg/ml, 3.0 µg/ml, and 10.0 µg/ml administration groups, respectively. The lower graph shows the percent confluence of cells after 48 hours of culture. In a dose-dependent anti-EphA3 pAb, blocking of EphA3 resulted in an inhibitory effect on IL-26 dependent cell proliferation in the presence of gefitinib. Data was expressed as a mean value ± S.D. of triplicate experiments. *P < 0.001.

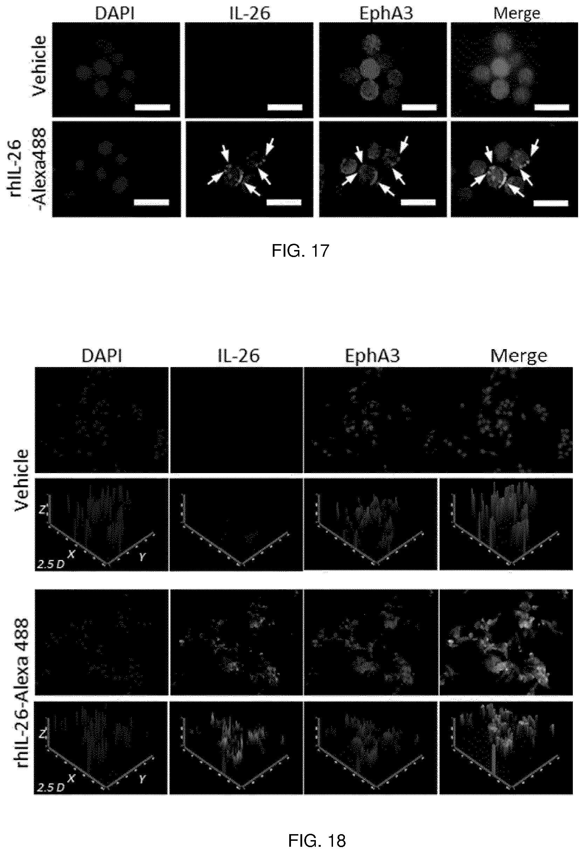

FIG. 17 is a photograph showing the results of immunostaining E0771 cells with rhIL-26-Alexa 488 (green), followed by anti-mouse EphA3 pAb (red) and DAPI (blue). A green staining with 11-26 was clearly fused with a red staining with a cell surface EphA3 (arrow in the lower photograph). 200x. Scale bar = 20 µm. A representative image of five independent experiments showing similar results is shown.
FIG. 18 shows a reconstructed 2.5D image of the intensity of fluorescence peaks of the image of FIG. 17. 2.5D intensity plot of EphA3, IL-26, and DAPI signals represents the absolute signal intensity of each pixel. Merged views and individual channels were shown. The 2.5D intensity plot showed that EphA3 and IL-26 were distributed almost similarly and coexisted frequently at edges. These images show EphA3/IL-26 interaction in E0771 cells. 200x. A representative image of five independent experiments showing similar results is shown.

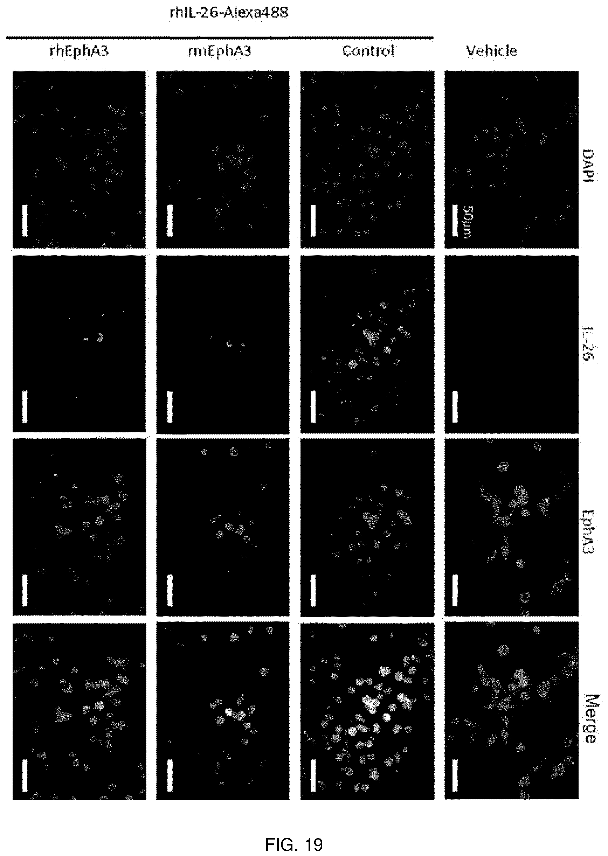

FIG. 19 is an image obtained by immunofluorescence staining EphA3 and IL-26 in E0771 cells in the presence or absence of human or mouse soluble EphA3. The immunofluorescence staining of E0771 cells was performed using anti-EphA3 pAb and rhIL-26-Alexa 488. EphA3 positive cells are shown in red and IL-26 are shown in green. All sections were stained with DAPI to mark nuclei (blue). Human and mouse soluble EphA3 inhibited EphA3/IL-26 interaction. Representative images are shown. 200x. Scale bar = 50 µm. A representative image of five independent experiments showing similar results is shown.

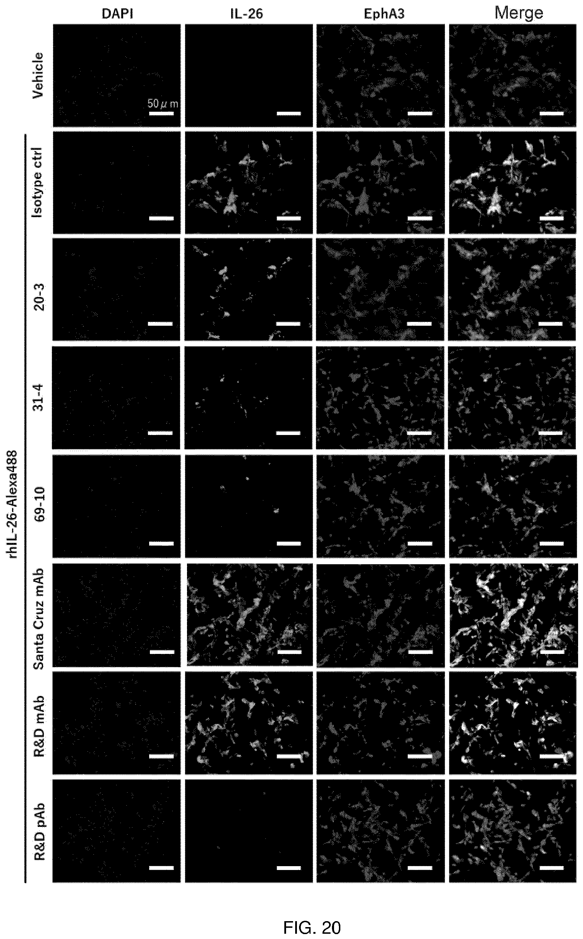

FIG. 20 is an image obtained by immunofluorescence staining EphA3 and IL-26 in E0771 cells in the presence or absence of anti-IL-26 mAb (clones 20-3, 31-4, and 69-10) or commercially available anti-IL-26 mAb (Santa Cruz and R&D Systems), anti-IL-26 pAb (R&D Systems), or control IgG (10 µg/ml) (Isotype ctrl). The immunofluorescence staining of E0771 cells was performed using anti-EphA3 pAb and rhIL-26-Alexa 488. The EphA3 positive cells are shown in red and IL-26 are shown in green. All sections were stained with DAPI to mark nuclei (blue). Anti-IL-26 mAb (clones 31-4 and 69-10) and anti-IL-26 pAb clearly inhibited the EphA3/IL-26 interaction. On the other hand, the commercially available anti-IL-26 mAb 2 clone hardly inhibited the EphA3/IL-26 interaction. Representative images are shown. 200x. Scale bar = 50 µm. Representative images of three independent experiments showing similar results are shown.

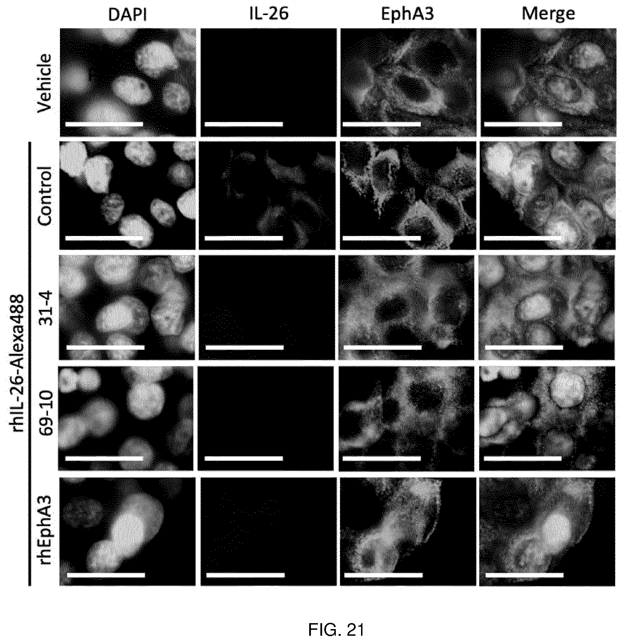

FIG. 21 shows HCC70 cells that were treated with recombinant human IL-26 (rhIL-26-Alexa488 (green)) (30 ng/ml) labeled with Alexa Fluor 488 in the presence of anti-IL-26 mAb (clone 31-4 or 69-10), recombinant human EphA3-Ig (rhEphA3), or control IgG (50 µg/ml each) for 1 hour, and then immunostained with anti-mouse EphA3 pAb (red) and DAPI (blue). IL-26 merged with EphA3 on the cell surface. On the other hand, anti-IL-26 mAbs and rhEphA3-Ig clearly inhibited the interaction between IL-26 and EphA3. Original magnification 200x. Scale bar = 20 µm. Representative images of three independent experiments with similar results are shown.

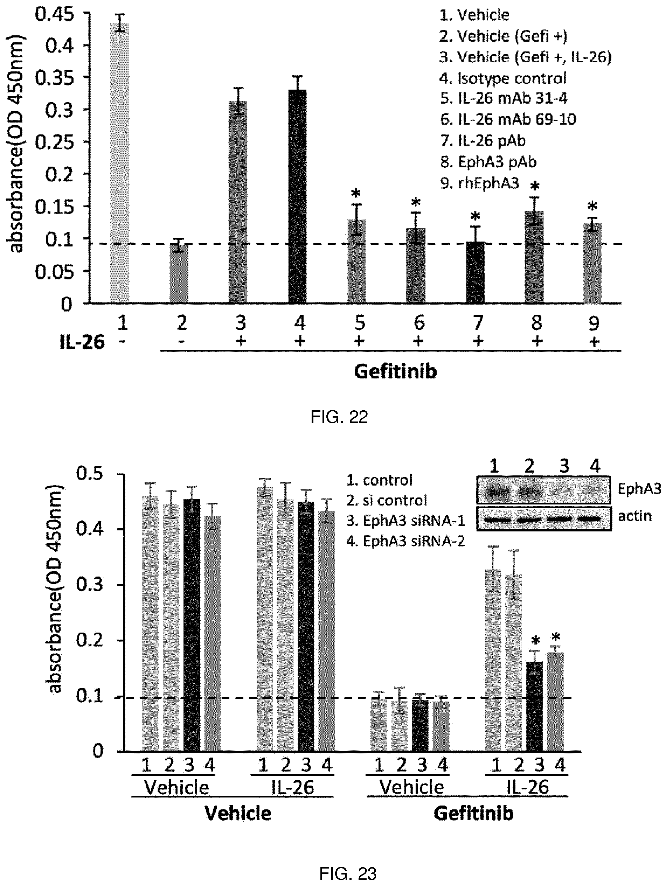

FIG. 22 is a graph showing the results of evaluating the cell proliferation of HCC70 cells treated with IL-26 (30 ng/ml) and gefitinib (40 µM) for 48 hours in the presence of indicated Abs or rhEphA3-Ig (50 µg/ml each) by the MTT assay. The broken lines indicate results of cells to which gefitinib and the vehicle were added as the reference values. All reagents including anti-IL-26 mAb and pAb, anti-EphA3 pAb and rhEphA3-Ig potently blocked the inhibitory effect of IL-26 on suppression of cell proliferation by gefitinib. Representative data from three independent experiments was presented as a mean value ± standard deviation of triplicate samples. Similar results were obtained in each experiment. *p < 0.01.
FIG. 23 shows HCC70 cells that were transfected with siRNA, and stimulated with IL-26 (30 ng/ml) for 48 hours in the presence or absence of gefitinib (40 µM). The expression level of EphA3 was confirmed by Western blotting. The cell proliferation was assessed by the MTT assay. The broken lines indicate the reference values for gefitinib + vehicle. Treatment with EphA3-siRNA in the presence of gefitinib significantly reduced the proliferation of IL-26 stimulated HCC70 cells as compared to control siRNA, although in the absence of gefitinib, siRNA directed against EphA3 did not affect the proliferation of either vehicle-treated HCC70 cells or IL-26 treated HCC70 cells. Representative data from three independent experiments was presented as a mean value ± standard deviation of triplicate samples. Similar results were obtained in each experiment. *p < 0.01.

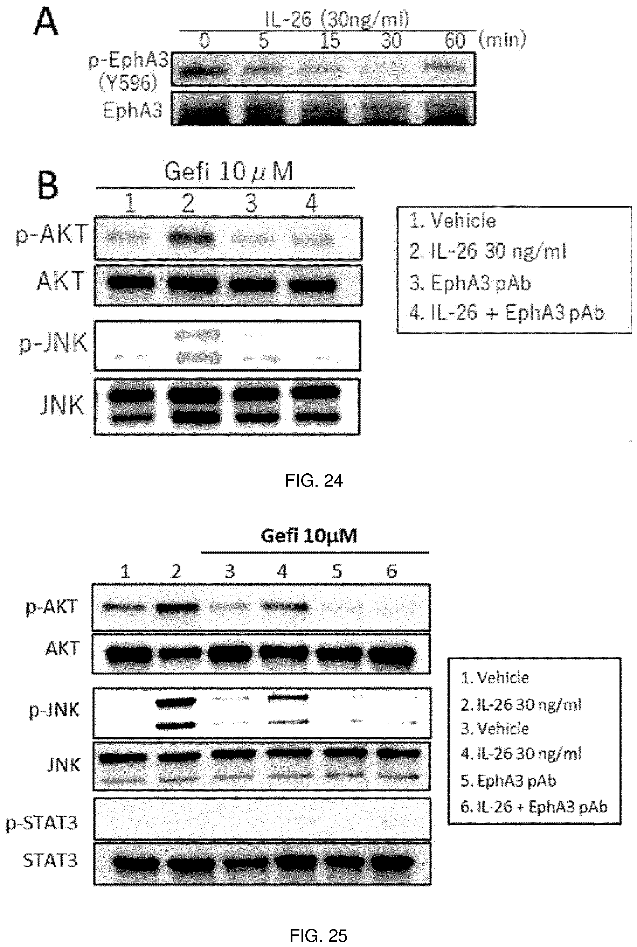

FIG. 24A shows E0771 cells that were treated with 30 ng/ml of IL-26 for 0, 5, 15, 30, or 60 minutes (min), and then subjected to Western blot analysis using anti-phosphorylated EphA3 and anti-EphA3 antibodies. The exogenous IL-26 induced dephosphorylation of EphA3. Representative data of three independent experiments showing similar results is shown. FIG. 24B shows E0771 cells that were stimulated with IL-26, anti-EphA3 pAb, IL-26 + anti-EphA3 pAb, or control IgG (vehicle) together with gefitinib (20 µM) for 5 minutes. Thereafter, a Western blot analysis was performed using an anti-phosphorylated AKT antibody and an anti-phosphorylated JNK antibody and, and re-blotted with an anti-pan AKT antibody and an anti-pan JNK antibody and. Treatment of E0771 cells with the exogenous IL-26 in the presence of gefitinib resulted in the phosphorylation of AKT and JNK (lanes 1 to 2), which was reversed by treatment with the anti-EphA3 receptor pAb (lane 4). Representative data of three independent experiments showing similar results is shown.
FIG. 25 shows HCC70 cells that were stimulated with EphA3 pAb (10 µg/ml) or isotype control mAb IL-26 in the presence or absence of gefitinib (40 µM) for 15 minutes. Treatment of HCC70 cells with the exogenous IL-26 in the presence of gefitinib resulted in the phosphorylation of AKT and JNK (lanes 3 to 4). This phosphorylation was counteracted by treatment with anti-EphA3 pAb (lane 6). Stimulation of HCC70 with IL-26 in the presence of gefitinib resulted in marked phosphorylation of AKT and JNK, whereas AKT and JNK phosphorylation with IL-26 stimulation was inhibited by EphA3 pAb. Phosphorylation of each protein was detected by immunoblotting. Representative data of three independent experiments showing similar results is shown.
Left in FIG. 26 shows HCC70 cells that were treated with IL-26 (30 ng/ml) or Ephrin A5 (200 ng/ml) in the presence of gefitinib (40 µM) for an indicated period, subjected to Western blot analysis using an anti-phosphorylated EphA3 antibody, an anti-phosphorylated AKT antibody, and an anti-phosphorylated JNK antibody, and reblotted using an anti-pan-EphA3 antibody, an anti-pan-AKT antibody, and an anti-pan-JNK antibody. IL-26 activated the AKT and JNK signals of HCC70 cells even in the presence of gefitinib, while inducing dephosphorylation for EphA3. In contrast, Ephrin A5 slightly enhanced the phosphorylation of AKT, but had little effect on the phosphorylation of JNK and EphA3. Right in FIG. 26 shows HCC70 cells that were treated with gefitinib (40 µM) and IL-26 (30 ng/ml) in the presence of anti-EphA3 pAb or isotype control IgG (50 µg/ml each) for 15 minutes and then subjected to Western blot analysis. The effect of IL-26 shown in the left panel was partially reversed by treatment with anti-EphA3 pAb. Representative data of three independent experiments is shown, and similar results were obtained in each experiment.

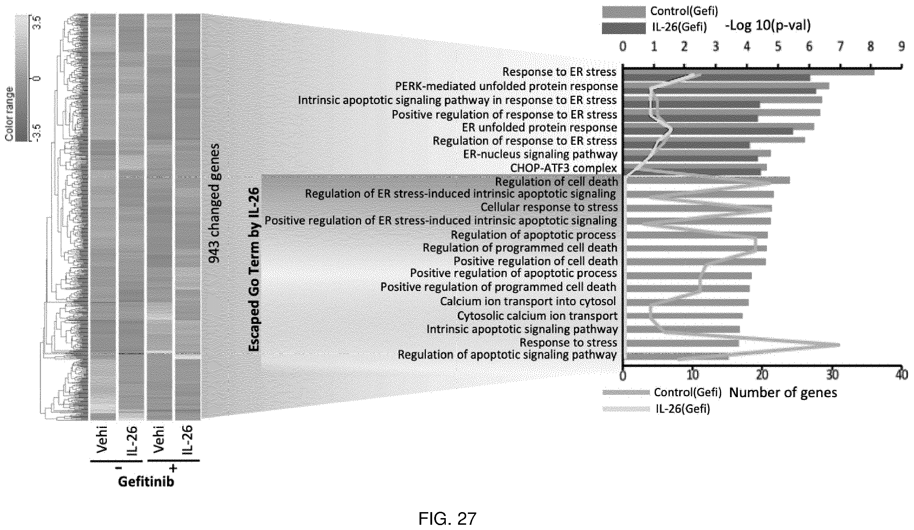

FIG. 27 shows total RNA that was isolated from HCC70 cells treated with IL-26 (30 ng/ml) or control vehicle (vehi) for 3 hours in the presence or absence of gefitinib (40 µM), and subjected to DNA microarray analysis. Left in FIG. 27 shows a heat map (magnification >= 2.0) of 943 genes differentially expressed between HCC70 cells treated with gefitinib and IL-26 and HCC70 cells treated with gefitinib alone that was constructed by the hierarchical cluster analysis. Right in FIG. 27 shows GO terms of genes greatly increased under induction by gefitinib and suppression by IL-26. Gene ontology (GO) analysis showed that the GO terms of endoplasmic reticulum stress and cell death signaling were significantly induced in HCC70 cells treated with gefitinib and the vehicle. In contrast, combination treatment of gefitinib with IL-26 significantly reduced the various GO terms of endoplasmic reticulum stress-induced apoptotic signaling.

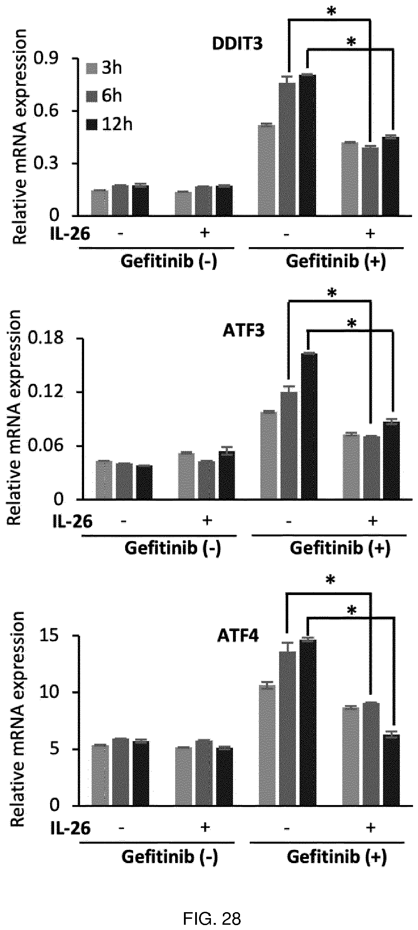

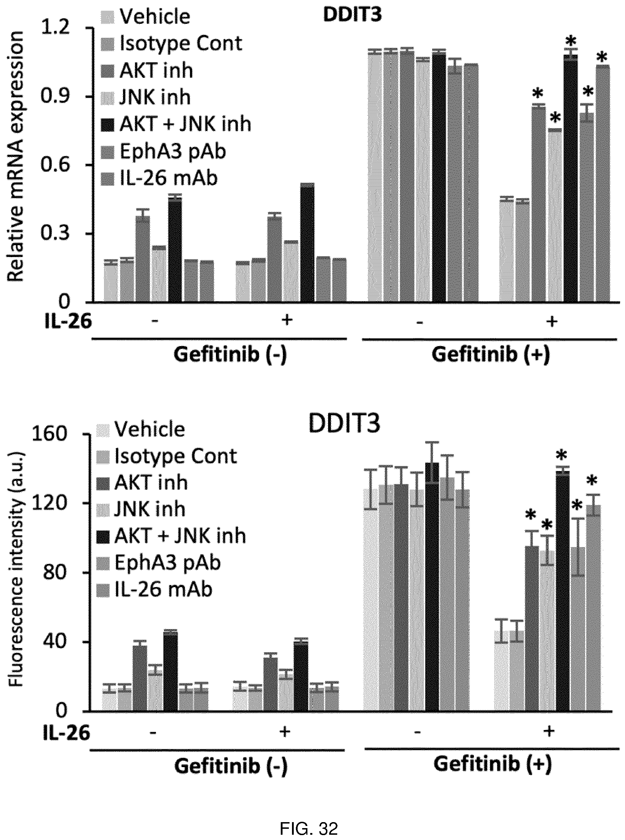

FIG. 28 shows HCC70 cells treated with IL-26 (30 ng/ml) in the presence or absence of gefitinib (40 µM) for an indicated period that were subjected to qRT-PCR analysis. The mRNA expression levels of DDIT3, ATF3, and ATF4 in HCC70 cells were significantly increased after gefitinib treatment, and IL-26 clearly decreased these expression levels in gefitinib-treated HCC70 cells, particularly at 6 and 12 hours. Representative data from three independent experiments with similar results is shown as a mean value ± standard deviation of triplicate samples (as in FIGS. 32 to 35).

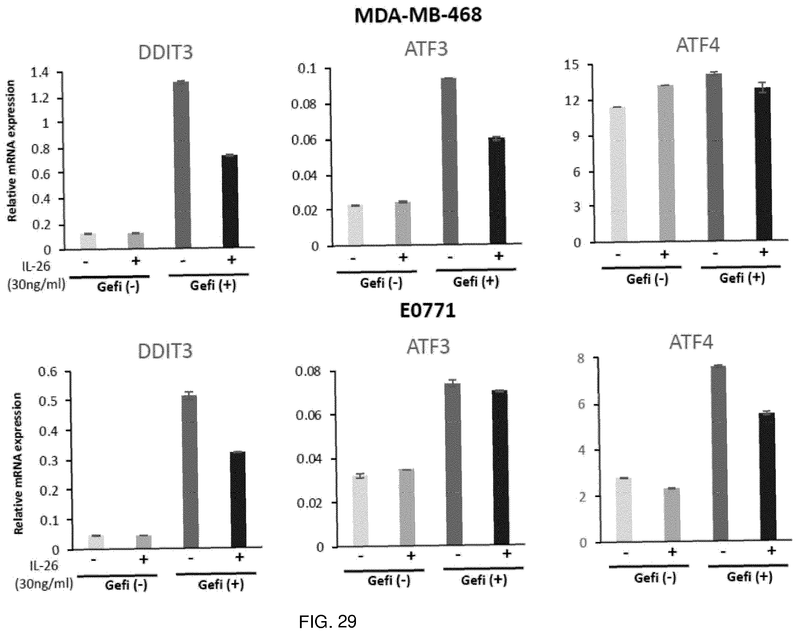

FIG. 29 shows human TNBC MDA-MB-468 cells and mouse TNBC E0771 cells that were treated with IL-26 (30 ng/ml) in the presence or absence of gefitinib (20 µM) for 6 hours. Total RNA was isolated and cDNA was synthesized with oligo (dT) primers. The mRNA expression levels were quantified by real-time RT-PCR and normalized to hypoxanthine phosphoribosyltransferase 1 (HPRT1) expression levels. The mRNA expression levels of DDIT3 in MDA-MB-468 and E0771 cells were significantly increased after gefitinib treatment, whereas IL-26 clearly reduced the DDIT3 expression levels of gefitinib-treated MDA-MB-468 and E0771. *p < 0.01. Representative data from three independent experiments is shown as a mean value ± standard deviation of triplicate samples. Similar results were obtained in each experiment.

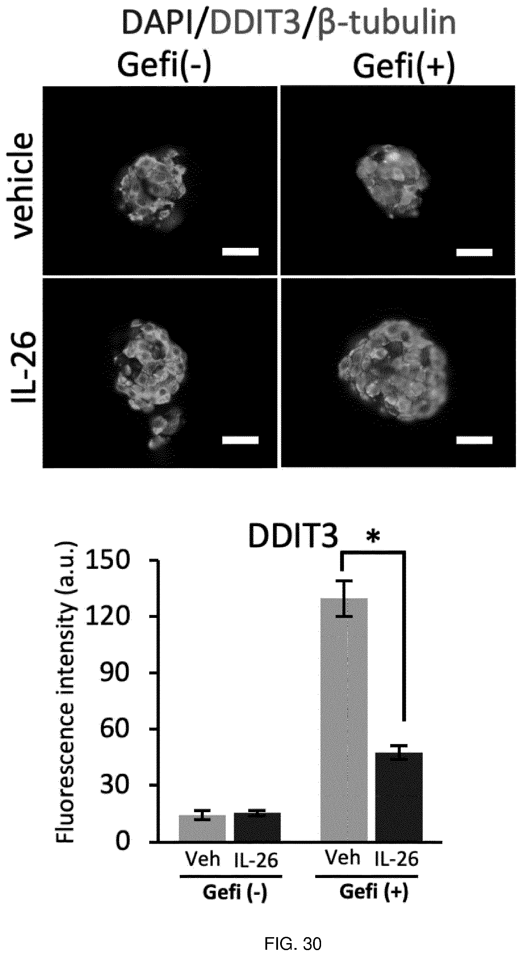

FIG. 30 shows HCC70 cells treated with IL-26 (30 ng/ml) for 24 hours in the presence or absence of gefitinib (40 µM) that were immunostained with anti-human DDIT3 pAb, β-tubulin, and DAPI. Original magnification, 400x. The scale bar indicates 20 µm.

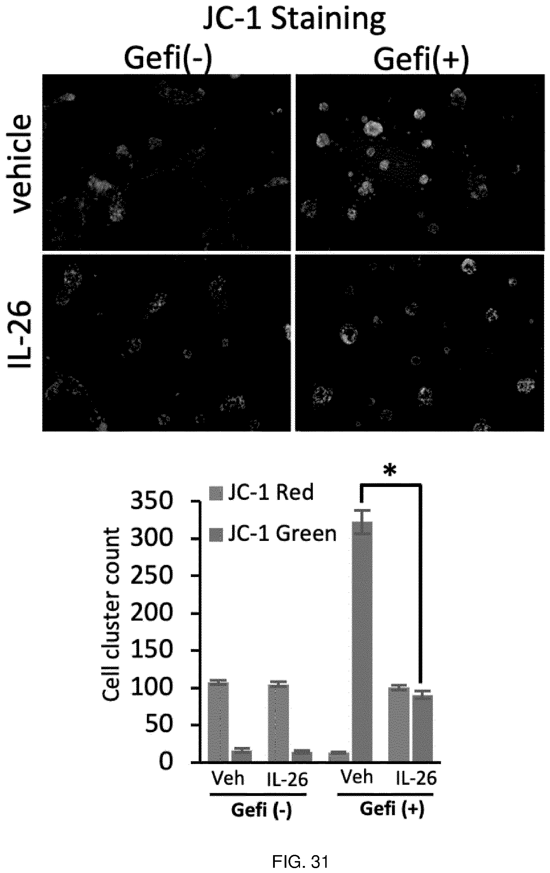

FIG. 31 shows HCC70 cells treated with IL-26 (30 ng/ml) for 24 hours in the presence or absence of gefitinib (40 µM) that were JC-1 stained. A red fluorescence, indicating a conserved mitochondrial membrane potential (ΔΨm), was observed in HCC70 cells treated with vehicle or IL-26 in the absence of gefitinib. A green fluorescence signal, an indicator of mitochondrial membrane depolarization, was significantly observed in HCC70 cells treated with the vehicle in the presence of gefitinib. Depolarization of the mitochondrial membrane was significantly reduced in HCC70 cells due to treatment with IL-26 even in the presence of gefitinib. Stained cell clusters were quantified using Image-J software (NIH). *p < 0.01.
Upper in FIG. 32 shows HCC70 cells that were treated with IL-26 (30 ng/ml) and/or gefitinib (40 µM) for 6 hours in the presence of vehicle, signal inhibitor, anti-EphA3 pAb, or anti-IL-26 mAb (clone 69-10) (50 µg/ml each). The mRNA expression levels were quantified by qRT-PCR. *p < 0.01. Lower in FIG. 32 shows HCC70 cells that were treated with IL-26 (30 ng/ml) and/or gefitinib (40 µM) for 24 hours in the presence of a vehicle, a signal inhibitor, an anti-EphA3 pAb, or an anti-IL-26 mAb (clone 69-10) (50 µg/ml each). Expression of DDIT3 was detected by immunofluorescence staining.

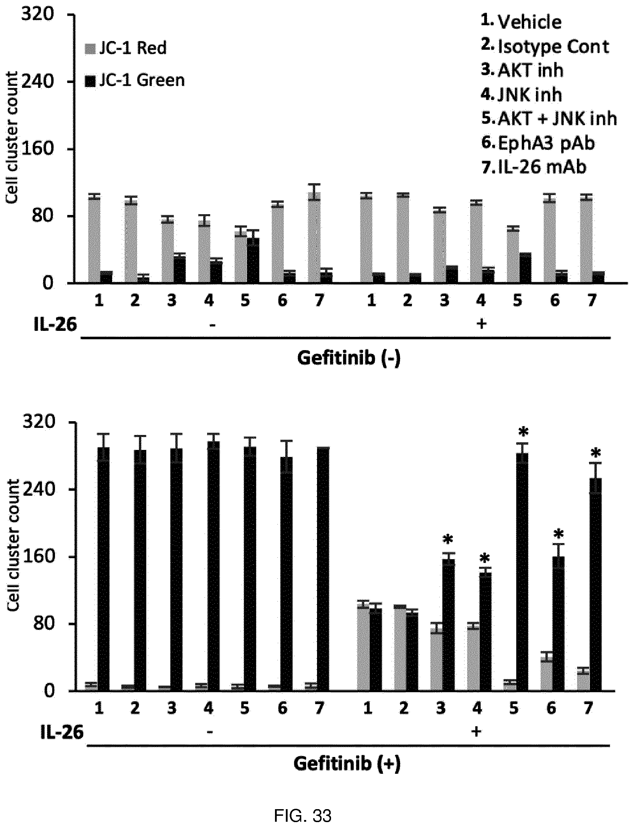

FIG. 33 shows HCC70 cells that were treated with IL-26 (30 ng/ml) and/or gefitinib (40 µM) for 24 hours in the presence of vehicle, signal inhibitor, anti-EphA3 pAb, or anti-IL-26 mAb (clone 69-10) (50 µg/ml each). The mitochondrial membrane potential (ΔΨm) was analyzed with JC-1 staining. The fluorescence intensity and stained cell clusters were quantified using Image-J software (NIH). *p < 0.01.

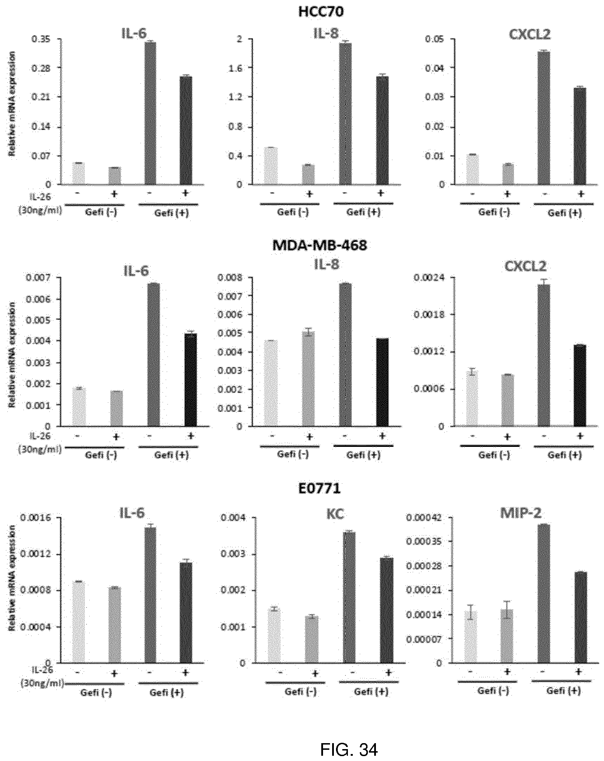

FIG. 34 shows HCC70 cells, MDA-MB-468, and E0771 cells were stimulated with IL-26 (30 ng/ml) for 6 hours in the presence or absence of gefitinib (40 µM for HCC70, 20 µM for MDA-MB-468 and E0771). Total RNA was isolated and cDNA was synthesized with oligo (dT) primers. The mRNA expression levels of IL-6, IL-8, CXCL2 (MIP-2) and CXCL1 (KC) were quantified by real-time RT-PCR and normalized to the expression level of hypoxanthine phosphoribosyltransferase 1 (HPRT1). The gefitinib treatment significantly enhanced the mRNA expression levels of IL-6, IL-8, and CXCL2 of HCC70 and MDA-MB-468, and the mRNA expression levels of IL-6, MIP-2, and KC of E0771. *p < 0.01.

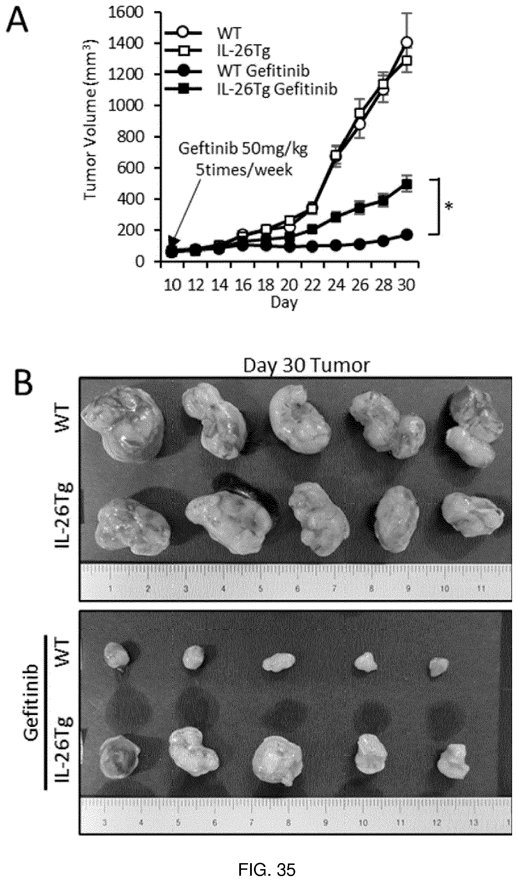

FIG. 35 shows the results of examining the effect of IL-26 in the presence of EGFR-TKI on TNBC tumor growth in vivo. E0771 cells (5×10⁵) and Matrigel were injected subcutaneously into a flank of hIL-26Tg (IL-26Tg) or littermate control mice (WT) (n=8, respectively). gefitinib was administered by oral gavage (50 mg/kg) once a day, five times a week after ten days from the inoculation of E0771 cells. FIG. 35A is a graph showing the change in tumor volume over time. The vertical axis represents a tumor volume (mm³), and the horizontal axis represents the number of days elapsed from tumor transplantation (day). Tumor growth was clearly suppressed by oral gavage of gefitinib compared to WT mice (white circles) and IL-26Tg (white squares). In the gefitinib-administered WT mice (black circles), significantly increased tumor growth was observed as compared with the gefitinib-administered IL-26Tg mice (black squares). Data was expressed as a mean value ± SEM. *P < 0.001. FIG. 35B is a macroscopic photograph of tumors resected 30 days after inoculation. It was observed that tumor growth was significantly increased in gefitinib-administered IL-26Tg mice (lower part of lower panel) compared to gefitinib-administered WT mice (upper part of lower panel). Representative images of eight independent experiments showing similar results are shown.

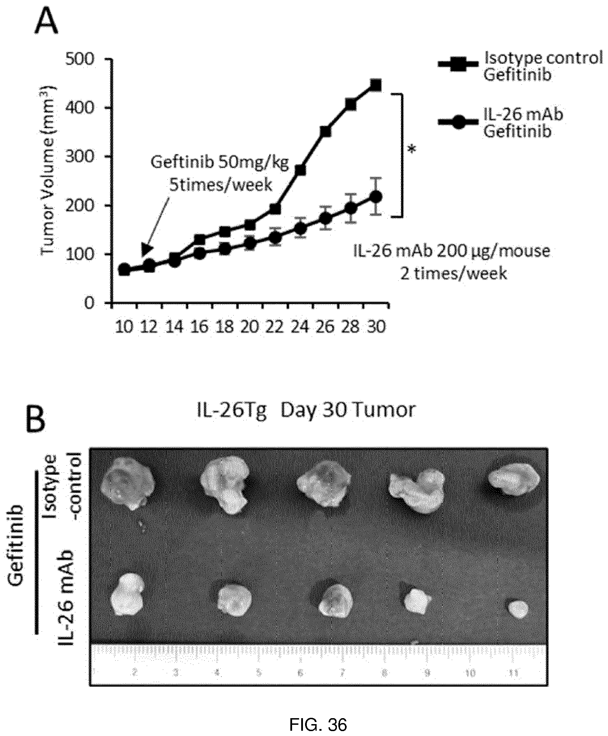

FIG. 36 shows hIL-26Tg mice (n=8) that were transplanted with E0771 cells in the same manner as in FIG. 35, and treated with gefitinib. From 10 days after E0771 inoculation, an anti-IL-26 mAb (clone 69-10) or a mouse IgG isotype control (200 µg/dose each) was intraperitoneally injected once a day, twice a week. FIG. 36A is a graph showing the change in tumor volume over time. The vertical axis represents a tumor volume (mm³), and the horizontal axis represents the number of days elapsed from tumor transplantation (day). The tumor growth was significantly suppressed by anti-IL-26 mAb treatment (black circles). Data was expressed as a mean value ± SEM. *P < 0.001. FIG. 36B is a macroscopic photograph of tumors resected 30 days after inoculation. A significant reduction in tumor growth was observed in the treatment using anti-IL-26 mAb with gefitinib (lower part of the photograph) than in the treatment using isotype control with gefitinib (upper part of the photograph). Representative images of eight independent experiments showing similar results are shown.

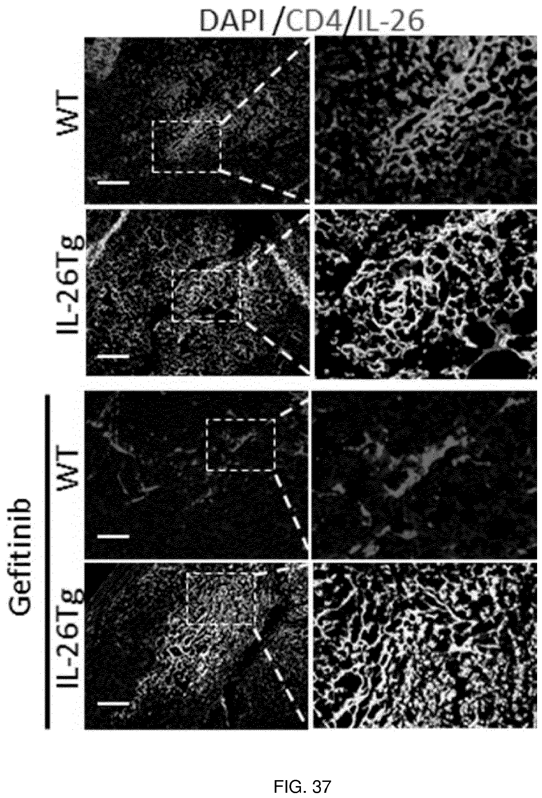

FIG. 37 is a photograph showing the results of immunohistochemical staining of a tumor specimen resected 14 days after E0771 inoculation. DAPI was stained in blue, CD4 in red, and IL-26 in green. The green staining with IL-26 was seen in CD4+ cells stained with red. 100x. Scale bar = 100 µm. Representative images of eight independent experiments showing similar results are shown.

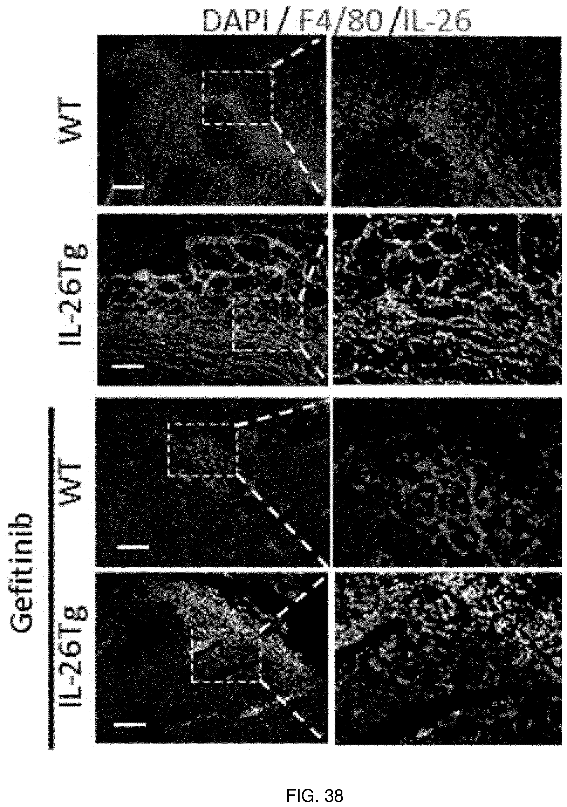

FIG. 38 is a photograph showing the results of immunohistochemical staining of a tumor specimen resected 14 days after E0771 inoculation. DAPI was stained in blue, F4/80 in red, and IL-26 in green. The green staining with IL-26 was seen in F4/80+ cells stained with red. 100x. Scale bar = 100 µm. Representative images of eight independent experiments showing similar results are shown.

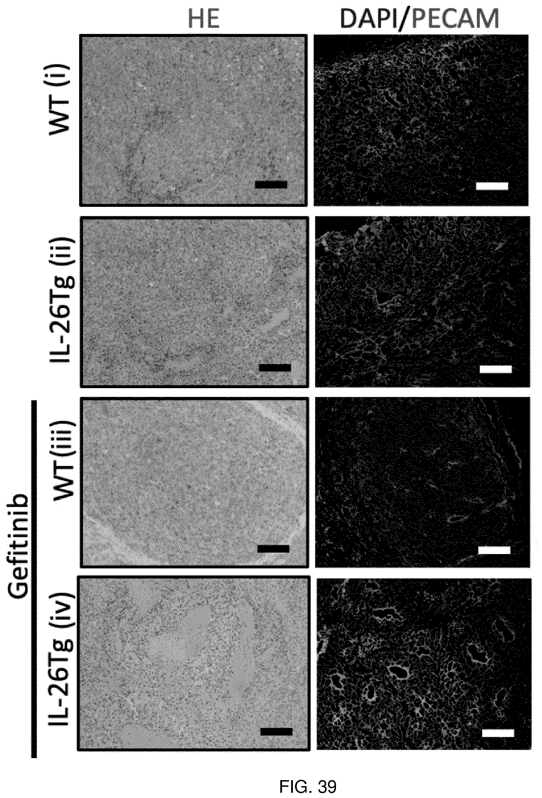

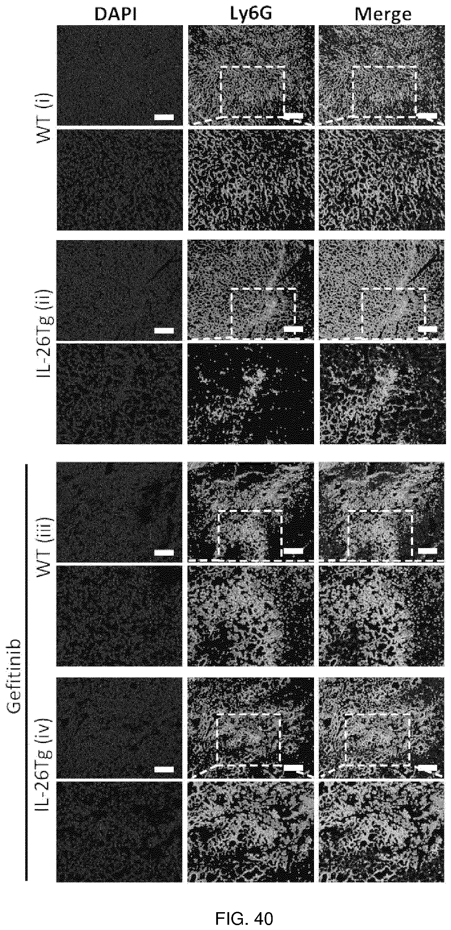

FIG. 39 is a photograph of H&E staining (left) and immunostaining using an anti-PECAM antibody (right) of a tumor specimen resected 14 days after E0771 inoculation. PECAM-positive blood vessels were significantly increased in gefitinib-treated hIL-26Tg mice (iv) than in gefitinib-treated WT mice (iii) or vehicle-treated hIL-26Tg mice (ii). The same applies to FIGS. 40 to 42. Original magnification, 100x. The scale bar indicates 100 µm. The inserted picture shows a high-magnification image of the box area. The images shown are representative images of eight mice in each group with similar results.
FIG. 40 is a photograph of immunostaining using an anti-Ly6G antibody of a tumor specimen resected 14 days after E0771 inoculation. In both the gefitinib-treated WT mice (iii) and the hIL-26Tg mice (iv), the number of Ly6G+ granulocytes infiltrating TME was significantly increased compared to the vehicle-treated WT mice (i) or the hIL-26Tg mice (ii). No IL-26 was detected in the WT mice treated with vehicle (i) or gefitinib (iii).

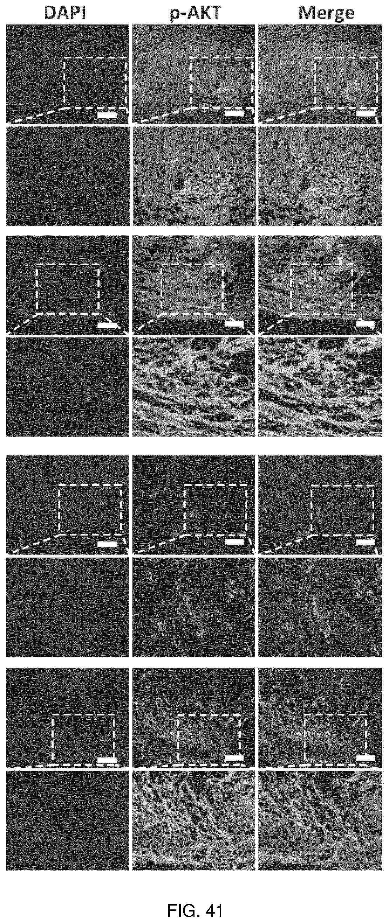

FIG. 41 is a photograph of immunostaining using an anti-p-AKT antibody of a tumor specimen resected 14 days after E0771 inoculation. The phosphorylation of AKT in tumor cells of the vehicle-treated hIL-26Tg mice (ii) was enhanced compared to the vehicle-treated WT mice (i). The phosphorylation of AKT in tumor cells was strongly suppressed in the gefitinib-treated WT mice (iii), whereas phosphorylated AKT was clearly observed in the hIL-26Tg mice in spite of the gefitinib treatment (iv).

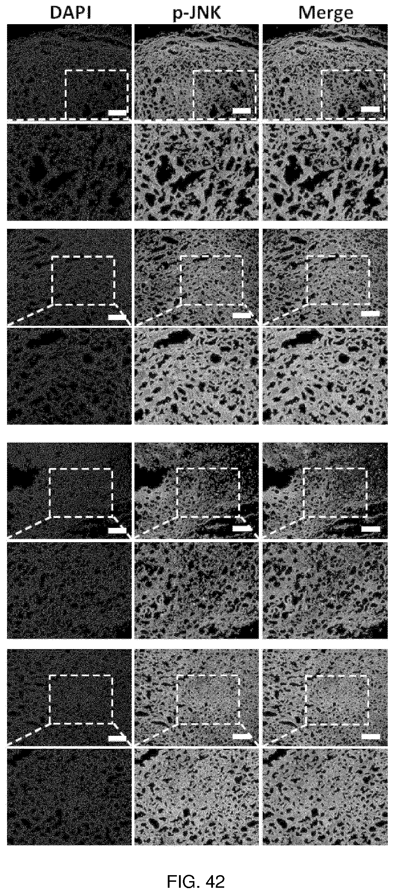

FIG. 42 is a photograph of immunostaining using an anti-p-JNK antibody of a tumor specimen resected 14 days after E0771 inoculation. The phosphorylation of JNK in tumor cells of the vehicle-treated hIL-26Tg mice (ii) was enhanced compared to the vehicle-treated WT mice (i). The phosphorylation of JNK in tumor cells was strongly suppressed in the gefitinib-treated WT mice (iii), while in the hIL-26Tg mice, liphosphorylated JNK was clearly observed in spite of the gefitinib treatment (iv).

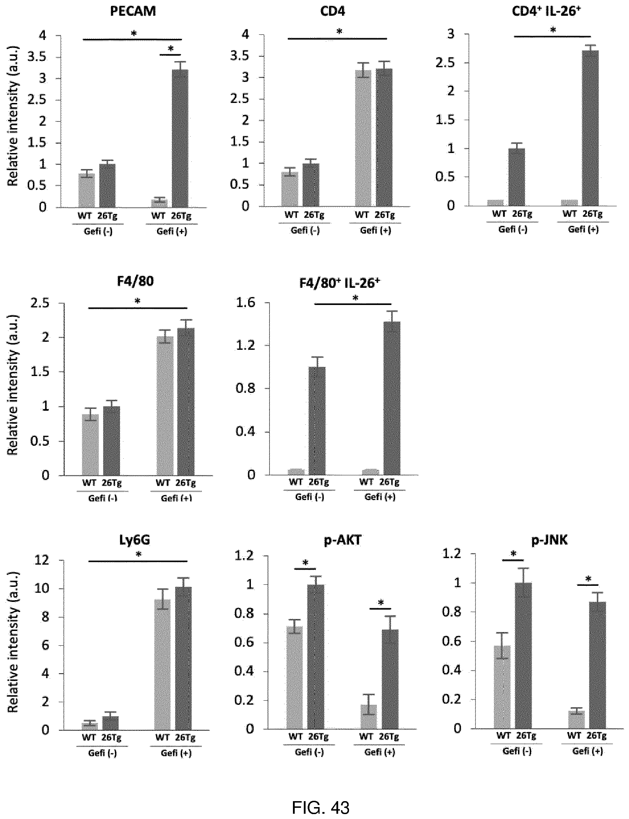

FIG. 43 shows Relative fluorescence intensities from eight mice in each group that were compared to the vehicle-treated WT mice (i), the gefitinib-treated WT mice (iii) or the hIL-26Tg mice (iv) and the vehicle-treated WT mice (ii), and the data as a mean value ± S.E. (*p < 0.01).

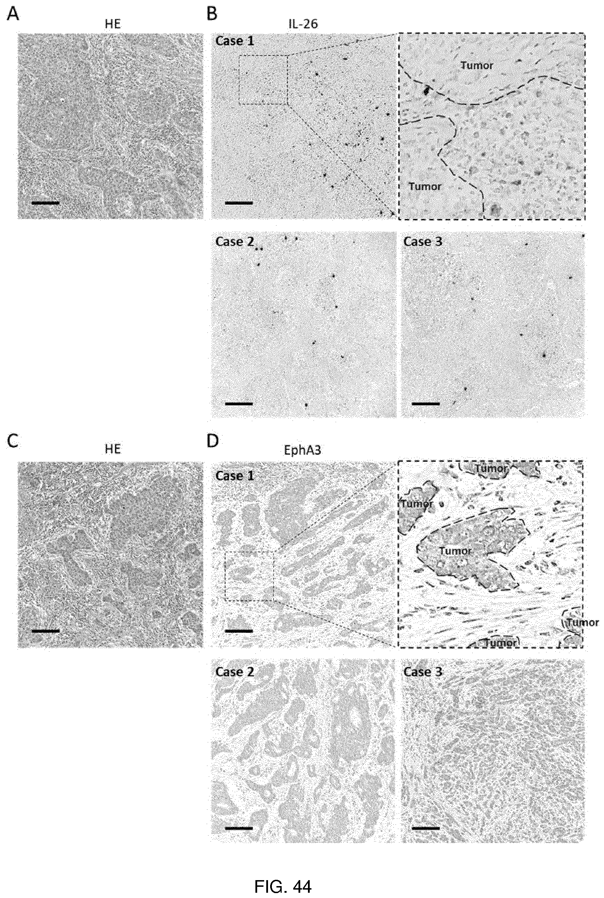

FIG. 44A and FIG. 44B show TNBC tissue specimens that were stained with H&E staining (A) and anti-human IL-26 mAb (B). All immunohistochemistry specimens were counterstained with hematoxylin. Representative images of three cases showing increased IL-26 protein expression are shown. IL-26 was detected in tumor-infiltrating lymphocytes. 40x. Scale bar = 100 µm. Similar results were obtained for the other six TNBC samples. FIG. 44C and FIG. 44D show TNBC tissue specimens that were stained with H&E staining (C) and anti-human EphA3 pAb (D). All immunohistochemistry specimens were counterstained with hematoxylin. Representative images of three cases showing increased EphA3 protein expression are shown. The expression of EphA3 was detected in tumor cells. 40x. Scale bar = 100 µm. Similar results were obtained for the other six TNBC samples.

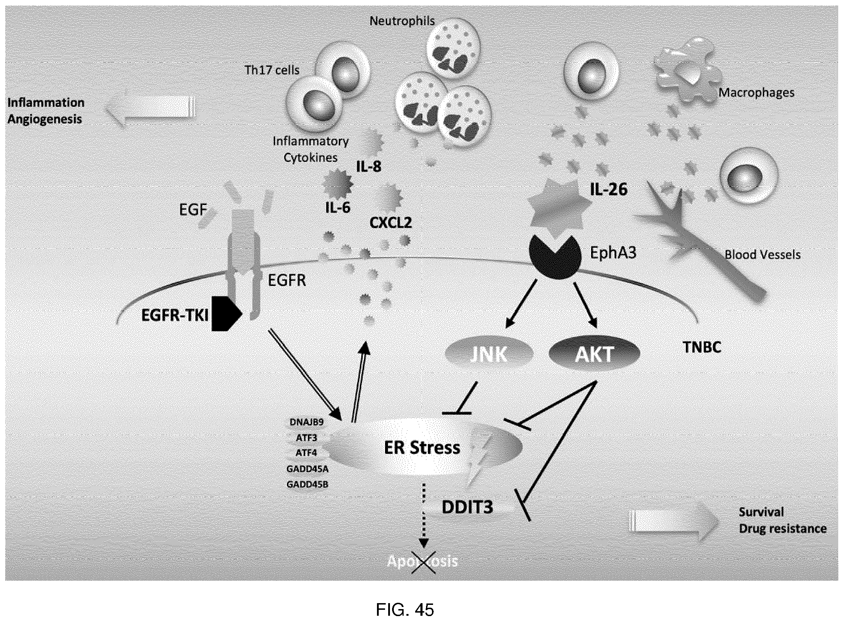

FIG. 45 is a conceptual diagram of a mechanism through interaction with a newly identified IL-26 receptor EphA3. IL-26 suppresses ER stress induced by EGFR-TKI by activating AKT and JNK signaling, which are EGFR-TKI bypass pathways, through interaction with a newly identified IL-26 receptor EphA3, thereby allowing TNBC tumors to survive and become drug resistant. Furthermore, after treatment with EGFR-TKI, production of ER stress-related inflammatory cytokines/chemokines including IL-6, IL-8, and CXCL2 is promoted, whereby neutrophils are recruited to TME, and accumulation of inflammatory cells such as T-cells and macrophages and an inflammatory reaction are further promoted.

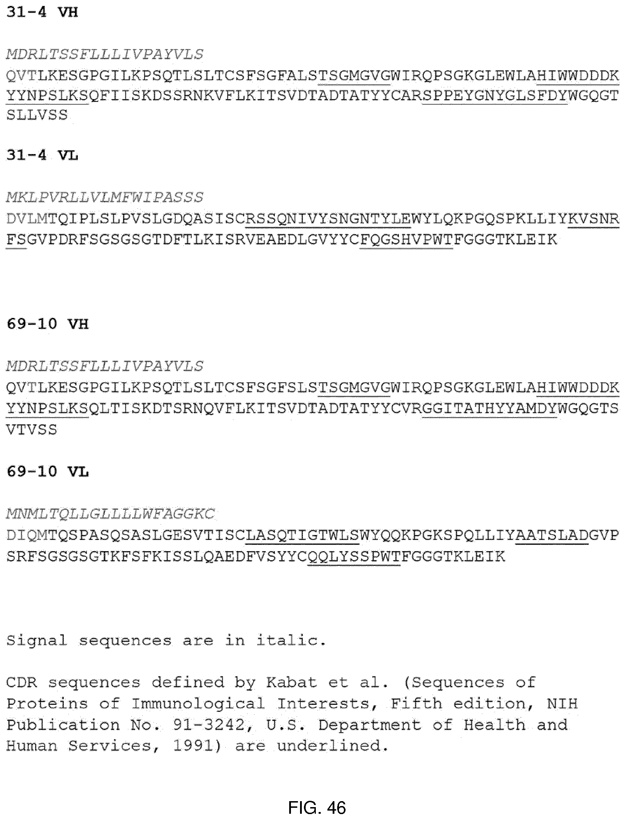

FIG. 46 is a figure showing an amino acid sequence of a mouse antibody of the present invention.

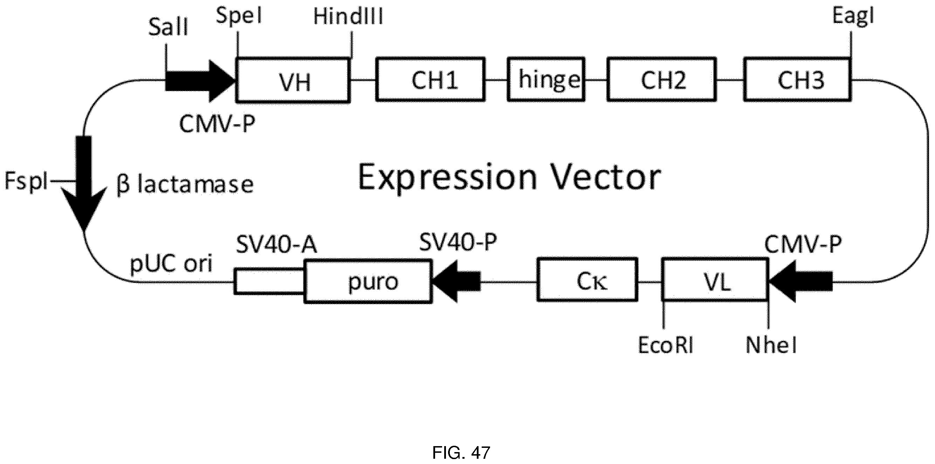

FIG. 47 shows schematic structures of pCh69-10, pHu69-10A, and pHu69-10B expression vectors (collectively referred to as "expression vectors"). The plasmid contains a heavy chain transcription unit starting with the major immediate early promoter and enhancer of human cytomegalovirus (CMV) (CMV-P) to initiate transcription of an antibody heavy chain gene, clockwise from the SalI site on the upper left. The CMV promoter followed by VH exon; genomic sequence of human gamma-1 heavy chain constant region including CH1, hinge, CH2, and CH3 exons via introns; and the CH3 exon followed by a polyadenylation site. After the heavy chain gene sequence, the light chain transcription unit starts at the CMV promoter, followed by the VL exon; genomic sequence including a human kappa chain constant region exon (Cκ), preceded by a portion of an intron; and the Cκ exon followed by the polyadenylation site. A light chain gene is followed by a segment containing the SV40 early promoter (SV40-P), a puromycin N-acetyltransferase gene for puromycin resistance acquisition (puro), and the SV40 polyadenylation site (SV40-A). In addition, the plasmid contains a part of plasmid pUC19 containing a bacterial origin of replication (pUC ori) and a beta-lactamase gene (β-lactamase). Positions of relevant restriction enzyme sites are shown in the figure.

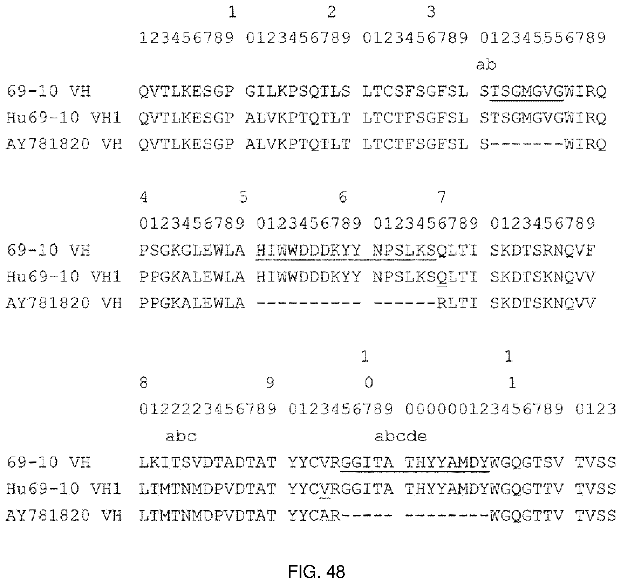

FIG. 48 is an alignment of amino acid sequences of 69-10VH (SEQ ID NO:44), humanized 69-10VH (Hu69-10VH1) (SEQ ID NO:24), and human acceptor AY781820 VH (SEQ ID NO:42). Amino acid residues are indicated by a one-letter code. Numbers above the sequences indicate positions according to Kabat et al. CDR sequences defined by Kabat et al. are shown in an underlined way at 69-10VH. CDR residues of AY781820VH were omitted. The amino acid residues underlined in Hu69-10VH1 were predicted to be important for proper formation of an antigen binding site, thus retaining the corresponding mouse residues at these positions in humanization.

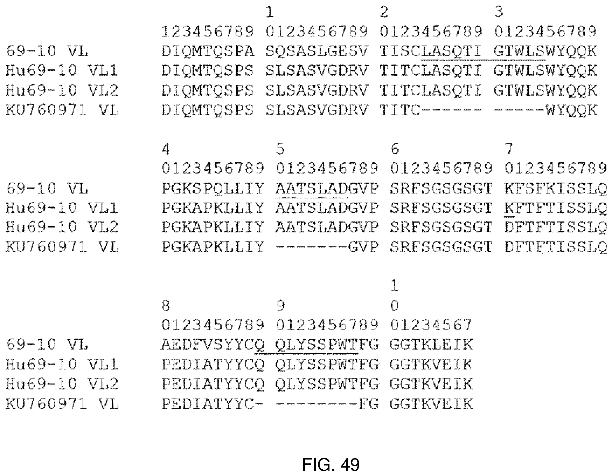

FIG. 49 is an alignment of amino acid sequences of 69-10VL (SEQ ID NO:45), two versions of humanized 69-10VL (Hu69-10VL1 and VL2) (SEQ ID NOs:30 and 35), and human acceptor KU760971 (GenBank Accession Number) VL (SEQ ID NO:43). The amino acid residues are indicated by the one-letter code. The numbers above the sequences indicate positions according to Kabat et al. The CDR sequences defined by Kabat et al. are shown in an underlined way at 69-10VL. The CDR residues for KU760971VL were omitted. The underlined amino acid residues in Hu69-10VL1 were predicted to be important for proper formation of the antigen binding site, thus retaining the mouse residues corresponding to this position in humanization.

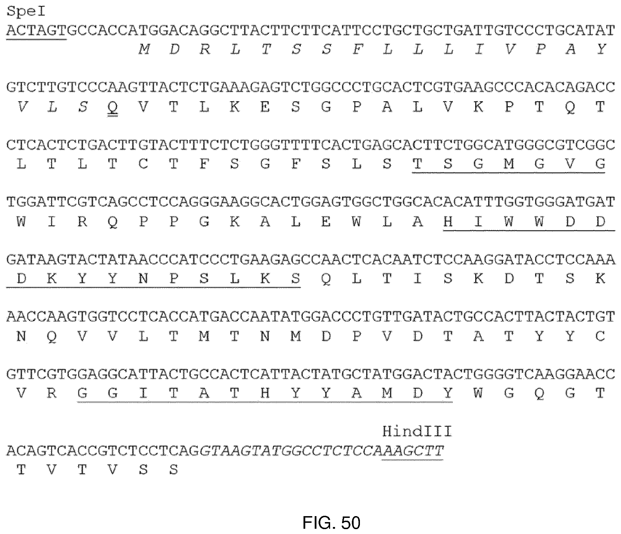

FIG. 50 shows the nucleotide sequence of Hu69-10VH1 gene adjacent to SpeI and HindIII sites (underlined) together with a putative amino acid sequence. The amino acid residues are indicated by the one-letter code. A signal peptide sequence is shown in italics. N-terminal amino acid residues (Q) of the mature VH are indicated by double underlines. CDR sequences as defined by Kabat et al. are shown in an underlined way. Intron sequences are shown in italics.

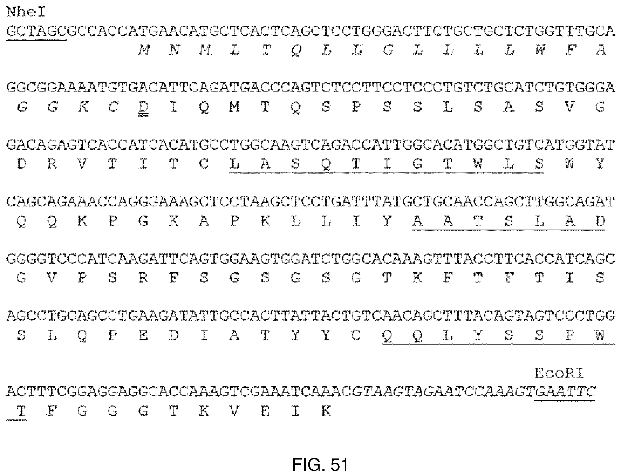

FIG. 51 shows the nucleotide sequence of the Hu69-10VL1 gene adjacent to the NheI and EcoRI sites (underlined), together with a putative amino acid sequence. The amino acid residues are indicated by the one-letter code. The signal peptide sequence is shown in italics. N-terminal amino acid residues (D) of the mature VL are indicated by double underlines. The CDR sequences as defined by Kabat et al. are shown in an underlined way. The intron sequences are shown in italics.

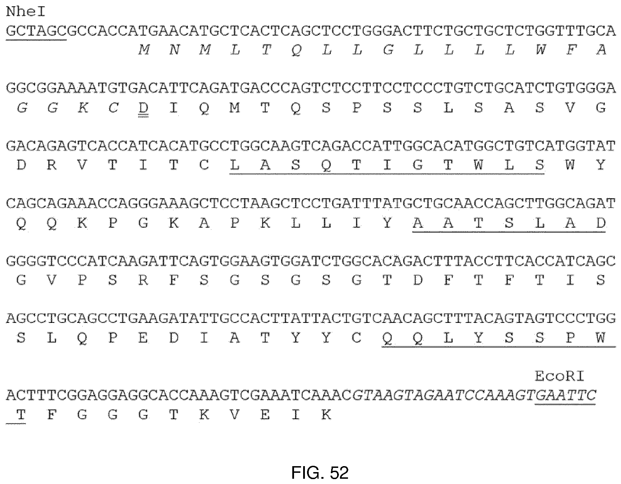

FIG. 52 shows the nucleotide sequence of the Hu69-10VL2 gene adjacent to the NheI and EcoRI sites (underlined), together with a putative amino acid sequence. The amino acid residues are indicated by the one-letter code. The signal peptide sequence is shown in italics. N-terminal amino acid residues (E) of the mature VL are indicated by double underlines. The CDR sequences as defined by Kabat et al. are shown in an underlined way. The intron sequences are shown in italics.

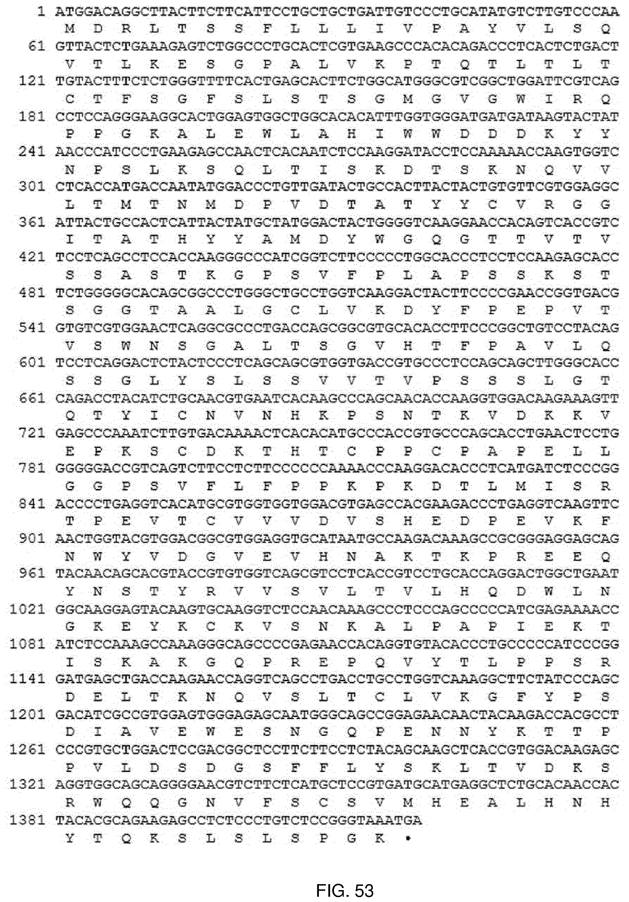

FIG. 53 shows the nucleotide sequence of a coding region of Hu69-10 heavy chain encoded by pHu-10A and pHu69-10B, together with a putative amino acid sequence. Stop codons are indicated by black dots.

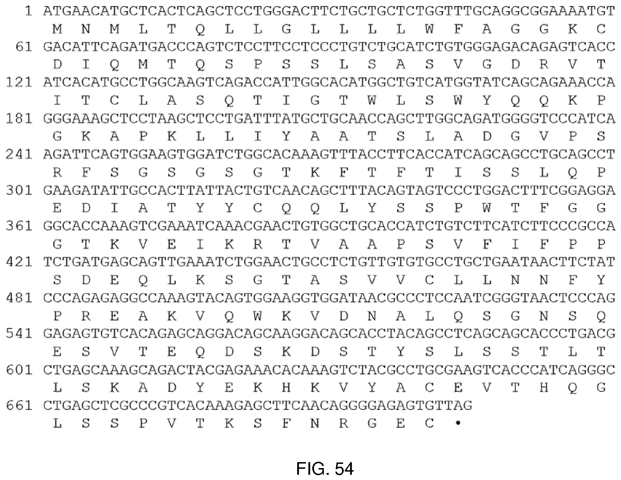

FIG. 54 shows a nucleotide sequence of a coding region of Hu69-10A light chain (including Hu69-10VL1) encoded by pHu69-10A, together with a putative amino acid sequence. The stop codons are indicated by black dots.

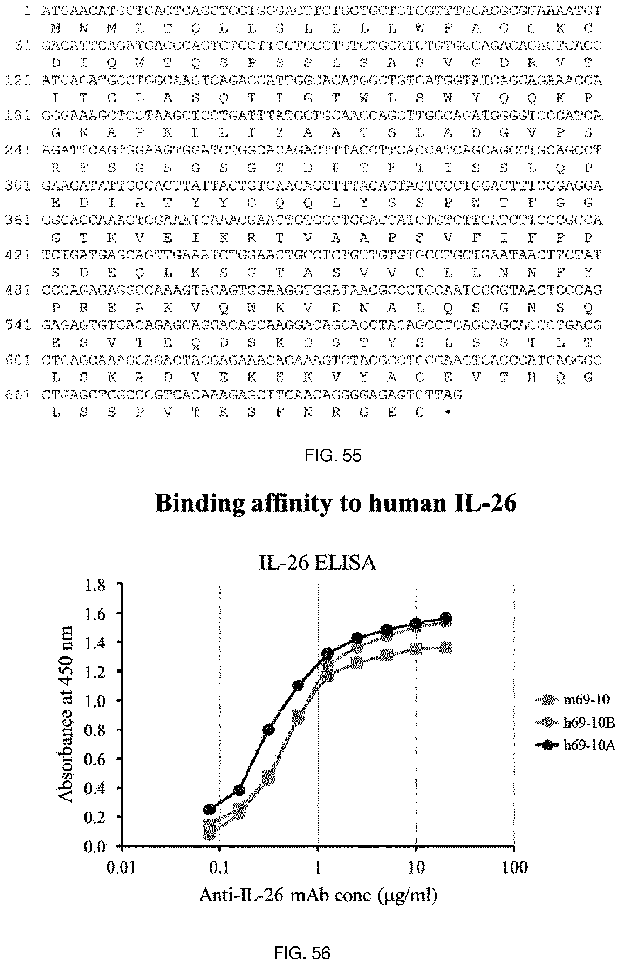

FIG. 55 shows a nucleotide sequence of a coding region of Hu69-10B light chain (including Hu69-10VL2) encoded by pHu69-10B, together with a putative amino acid sequence. The stop codons are indicated by black dots.
FIG. 56 is a figure showing the binding of purified mouse anti-human IL-26 antibody m69-10, humanized anti-IL-26 antibodies Hu69-10A and Hu69-10B to IL-26. Recombinant human IL-26 was immobilized on a plate, and mouse and the humanized anti-IL-26 antibodies were added for binding. Next, a secondary antibody (Horseradish peroxidase (HRP) -conjugated anti-mouse Ig antibody and HRP-conjugated anti-human IgG antibody) was reacted, a substrate was added, and then an absorption value (an absorption wavelength) at 450 nm was measured with a plate reader. In all the antibodies, an increase in the absorption value dependent on an antibody concentration was observed with respect to IL-26 immobilized on the plate. Among them, Hu69-10A established by the present inventors showed stronger binding to IL-26 than an original mouse antibody m69-10. Data is shown as a mean value ± standard deviation of results measured in triplicate for each group. Representative data from three independent experiments with similar results is shown.

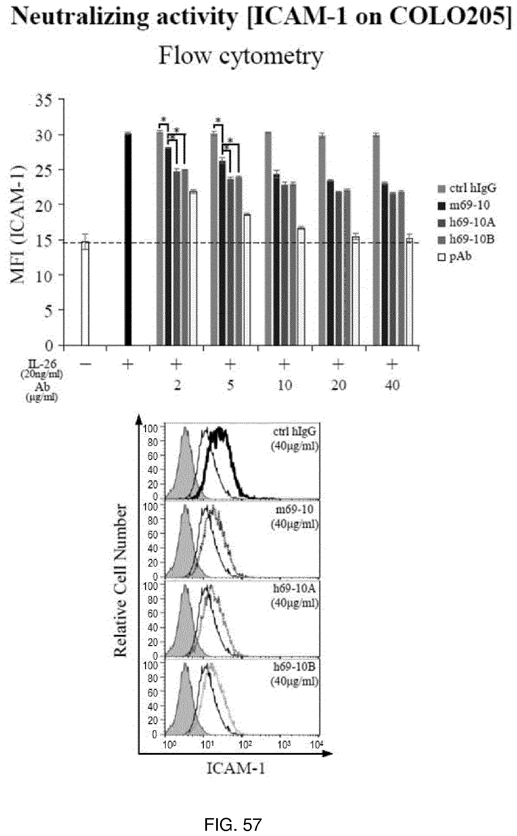

FIG. 57 is a figure showing a neutralizing activity of IL-26 against a human large intestine cancer cell line COLO 205 by the purified mouse anti-human IL-26 antibody m69-10, the humanized anti-IL-26 antibodies Hu69-10A and Hu69-10B, and a commercially available goat anti-human IL-26 polyclonal antibody. The exogenous IL-26 was added to COLO 205 cells in the presence and absence of a control human IgG or various anti-human IL-26 antibodies, and the cells were collected after 24 hours of culture, and the expression of ICAM-1 was measured by flow cytometry. MFI is an abbreviation of mean fluorescence intensity, and indicates a value (= a mean value) obtained by dividing total fluorescence intensity of each cell taken as data by the number of all cells. The higher the fluorescence intensity, the more ICAM-1 molecules are expressed on the cell membrane of one cell, which means that many fluorescently labeled antibodies are bound to them. COLO 205 cells were added with the exogenous IL-26 in the presence of the control human IgG, the purified mouse anti-human IL-26 antibody m69-10, the humanized anti-IL-26 antibodies Hu69-10A and Hu69-10B, or the commercially available goat anti-human IL-26 polyclonal antibody. The expression of ICAM-1 in COLO 205 cells was enhanced by the exogenous IL-26 and partially inhibited by the presence of anti-human IL-26 monoclonal antibodies established by the present inventors (*p < 0.01 with respect to corresponding control human IgG-added group). The humanized anti-IL-26 antibodies Hu69-10A and Hu69-10B showed stronger inhibitory effect at 2 µg/ml and 5 µg/ml than the original mouse antibody m69-10. Data is shown as a mean value ± standard deviation of the results of culturing in triplicate for each group and measuring each independently. Dotted lines show the MFI of ICAM-1 in unstimulated COLO 205 cells. Representative data from three independent experiments with similar results is shown.

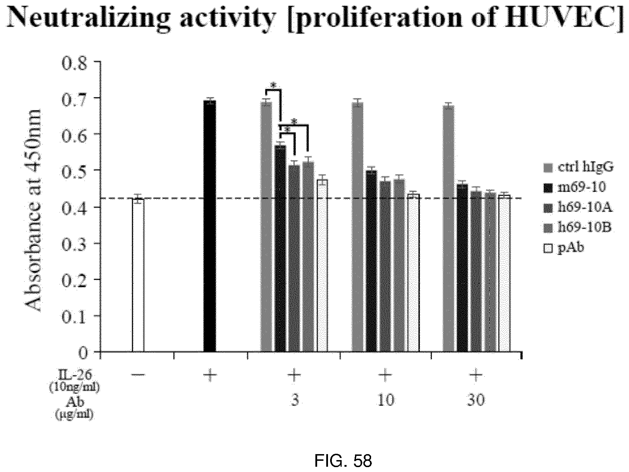

FIG. 58 shows the neutralizing activity of IL-26 against human umbilical vein endothelial cells HUVECs by the commercially available goat anti-human IL-26 polyclonal antibody, the purified mouse anti-human IL-26 antibody m69-10, and humanized anti-IL-26 antibodies Hu69-10A and Hu69-10B. The exogenous IL-26 was added to HUVECs in the presence or absence of the control human IgG or various anti-human IL-26 antibodies. The cell proliferation of HUVECs treated with IL-26 for 48 hours was assessed in the MTT assay. The proliferation of HUVECs was promoted by the exogenous IL-26 and significantly inhibited by the presence of the anti-human IL-26 monoclonal antibody established by the present inventors (* p < 0.01 with respect to the corresponding control human IgG-added group). The humanized anti-IL-26 antibodies Hu69-10A and Hu69-10B showed a stronger inhibitory effect at 3 µg/ml than the original mouse antibody m69-10. The data is shown as a mean value ± standard deviation of results of culturing in quadruplicate for each group and measuring independently for each group. The dotted lines indicate cell proliferation of unstimulated HUVECs. Representative data from three independent experiments with similar results is shown.

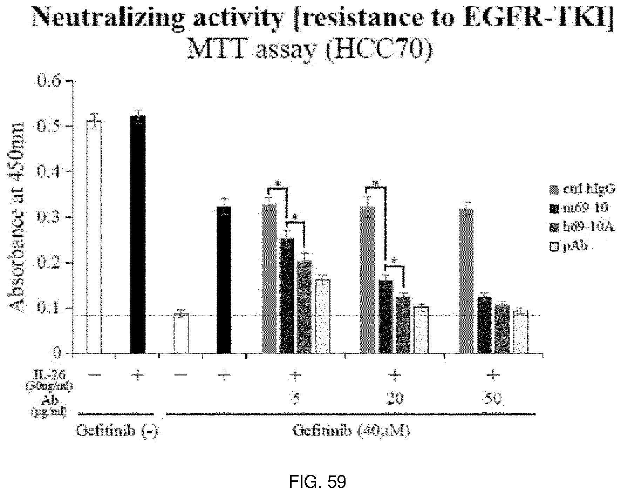

FIG. 59 shows the neutralizing activity of IL-26 against human TNBC strain HCC70 of the commercially available goat anti-human IL-26 polyclonal antibody, the purified mouse anti-human IL-26 antibody m69-10, and humanized anti-IL-26 antibody Hu69-10A. These are graphs showing the results of the MTT assay of HCC70 cells treated with IL-26 (30 ng/ml) and/or gefitinib (40 µM) for 48 hours in the presence of the anti-IL-26 neutralizing mAb or the control human IgG. The blocking of IL-26 with the anti-IL-26 antibody clearly reversed the inhibitory effect of IL-26 on gefitinib-induced suppression of cell proliferation. The humanized anti-IL-26 antibody Hu69-10A showed a stronger inhibitory effect at 5 µg/ml and 20 µg/ml than the original mouse antibody m69-10. The representative data from three independent experiments was presented as a mean value ± standard deviation of triplicate samples. The dotted lines indicate the reference values when gefitinib and the vehicle were added. Similar results were obtained in each experiment.

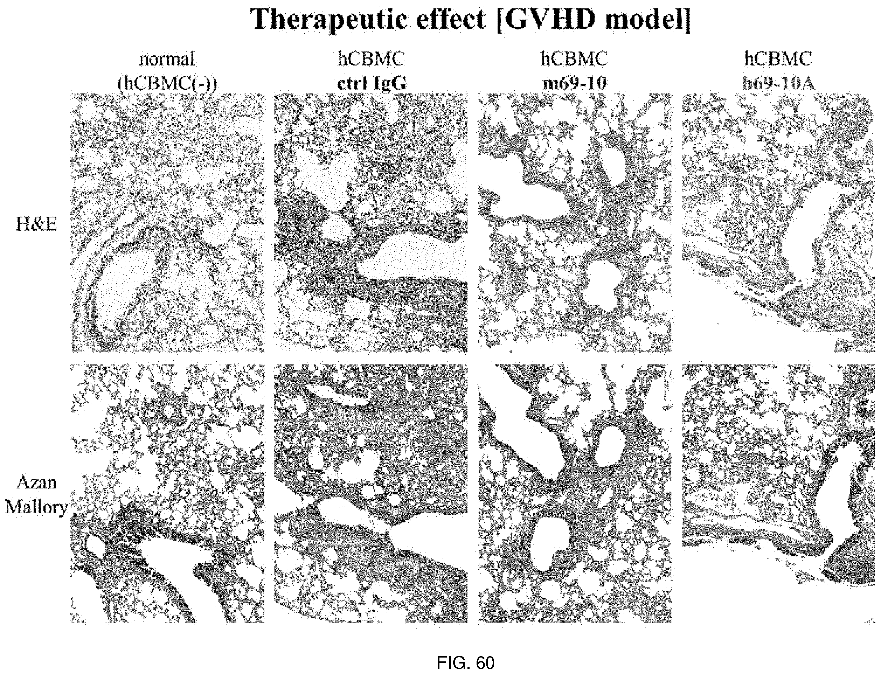

FIG. 60 is a figure showing therapeutic effects of a mouse anti-human IL-26 antibody m69-10 and a humanized anti-IL-26 antibody Hu69-10A in a heterogeneous chronic GVHD model. NOG mice were irradiated at a sublethal dose (200 cGy) and the next day mononuclear cells (CBMC) isolated from human umbilical cord blood (5×10⁶) were transplanted from the tail vein of the mice. The control human IgG or monoclonal antibody 69-10 was intraperitoneally administered twice a week at 200 µg per mouse from day 28 after transplantation when clinical signs/symptoms of mild GVHD began to appear. The data is cumulative results from three independent experiments (the control human IgG group n=10, m69-10 group n=10, Hu69-10A group n=6). Representative results of each group of H & E staining and AZAN-MALLORY staining of the lung at eight weeks after transplantation in the GVHD non-onset control group in which human CBMC is not transplanted, the group in which the control human IgG is administered after CBMC transplantation, the group in which the mouse anti-human IL-26 antibody m69-10 is administered, and the group in which the humanized anti-IL-26 antibody Hu69-10A is administered are shown. In the group to which the control human IgG was administered after CBMC transplantation, marked inflammatory cell infiltration, bronchial stenosis due to fibrogenesis, and collagen deposition around the bronchi and blood vessels were observed, whereas in the group to which the mouse antibody m69-10 was administered, suppression of inflammatory cell infiltration, reduction of fibrogenesis, and reduction of collagen deposition were remarkably observed, and it was shown that in the group to which the humanized antibody Hu69-10A was administered, the therapeutic effect was even stronger. It is 100× of the original size.

### MODE FOR CARRYING OUT THE INVENTION

Accordingly, in one embodiment, the present invention relates to a pharmaceutical composition for use in combination with an anticancer drug for treating cancer in a patient, the pharmaceutical composition containing a drug that reverses resistance to an anticancer drug due to IL-26 as an active ingredient. Furthermore, the present invention relates to a method for producing a pharmaceutical composition for treating cancer, containing a drug that reverses resistance to an anticancer drug due to IL-26 as an active ingredient, for use in combination with an anticancer drug. In addition, the present invention also relates to a drug for reversing resistance to an anticancer drug due to IL-26 for use in a method for treating cancer in combination with a chemotherapeutic agent. Also, the present invention relates to a method for treating cancer in a patient, the method including administering an effective amount of an anticancer drug and a drug for reversing resistance to an anticancer drug due to IL-26 to a patient in need thereof.

As used in the present description, "cancer" and "tumor" are used interchangeably and mean cells that have undergone malignant transformation to be pathogenic for a host organism. Primary cancer cells (that is, cells obtained from the vicinity of the site of malignant transformation) can be readily distinguished from non-cancerous cells by well-established techniques, particularly histological examination. In the present description, the definition of cancer includes not only primary cancers but also any cancer derived from primary cancers, for example, metastatic cancers. Cancers that manifest as solid tumors are detectable based on a tumor mass and can be identified, for example, by CAT scan, MR imaging, X-ray, ultrasound, or palpation, and/or detection of expression of one or more cancer-specific antigens in a sample obtained from a patient. The cancers may be, for example, a hematopoietic tumor that is a tumor such as blood cells. Examples of the cancer in the present description include, but are not limited to, a lung cancer, a non-small cell lung cancer, a small cell lung cancer, a non-Hodgkin's lymphoma, an adrenocortical cancer, an AIDS-related cancer, an AIDS-related lymphoma, a pediatric cerebellar astrocytoma, a pediatric cerebral astrocytoma, a basal cell cancer, a skin cancer (non-melanoma), a biliary tract cancer, an extrahepatic bile duct cancer, an intrahepatic bile duct cancer, a bladder cancer, a cancer of bone or joint, an osteosarcoma and a malignant fibrohistiocytoma, a brain cancer, a brain tumor, a brain stem glioma, a cerebellar astrocytoma, a glioma, a cerebral astrocytoma/malignant glioma, an ependymoma, a medulloblastoma, a supratentorial primitive neuroectodermal tumor, a visual pathway and hypothalamic glioma, a head and neck cancer, a metastatic squamous cell carcinoma, a bronchial adenoma/carcinoid, a carcinoid tumor, a nervous system lymphoma, a cancer of central nervous system, a central nervous system lymphoma, a neuroblastoma, a pediatric syndrome, a Seziary syndrome, an extracranial germ cell tumor, an extragonadal germ cell tumor, an extrahepatic bile duct cancer, a cancer of eye, an intraocular melanoma, a retinoblastoma, a salivary gland cancer, an oral cancer, an oral cavity cancer, an oropharyngeal cancer, an oral cancer, a cancer of tongue, an esophageal cancer, a gastric cancer, a gastrointestinal carcinoid tumor, a gastrointestinal stromal tumor (GIST), a small intestine cancer, a colon cancer, a colorectal cancer, a rectal cancer, an anorectal cancer, an anal cancer, a germ cell tumor, a hepatocyte (liver) cancer, a Hodgkin lymphoma, a laryngeal cancer, a pharyngeal cancer, a hypopharyngeal cancer, a nasopharyngeal cancer, a sinus and nasal cavity cancer, a throat cancer, a pancreatic islet cell tumor (endocrine pancreas), a pancreatic cancer, a parathyroid cancer, a liver cancer, a gallbladder cancer, an appendix cancer, a kidney cancer, a urethral cancer, a transitional cell carcinoma of renal pelvis and ureter and other urinary cancers, a squamous cell carcinoma, a penile cancer, a testicular cancer, a thymoma, a thymoma and thymic carcinoma, a thyroid cancer, a prostatic cancer, an ovarian cancer, an ovarian epithelial cancer, an ovarian low malignancy tumor, an ovarian germ cell tumor, a gestational trophoblastic tumor, a breast cancer, a uterine cancer, a uterine sarcoma, a cervical cancer, an endometrial cancer, a cancer of endometrium, a vaginal cancer, a vulvar cancer, and Wilms' tumor, an acute lymphoblastic leukemia, an acute myelogenous leukemia, a chronic lymphocytic leukemia, a chronic myelogenous leukemia, a multiple myeloma, a chronic myeloproliferative disease, a hairy cell leukemia, a primary central nervous system lymphoma, a chronic myeloproliferative disease, a cutaneous T-cell lymphoma, a lymphoid neoplasm, a macroglobulinemia of Waldenstram, a medulloblastoma, a skin cancer (non-melanoma), a skin cancer (melanoma), a brown cell carcinoma, a Merkel cell skin cancer, a mesothelioma, a malignant mesothelioma, a multiple endocrine tumor syndrome, a myelodysplastic syndrome, a myelodysplasia/maleoproliferative disease, a pineoblastoma and pituitary tumor, a plasmacytoma, a pleura lungs blastoma, a retinoblastoma, a rhabdomyosarcoma, a sarcoma, an Ewing's tumor families, a soft tissue sarcoma, a soft tissue sarcoma, a mycosis fungoides lymphoma, and Kaposi's sarcoma. In the present description, a breast cancer is particularly preferable.

In the present description, cancers or cancer cells are mammalian cancers or mammalian cancer cells, preferably human cancers or human cancer cells.

The cancer in the present description may be HER2-negative, and includes, for example, HER2-negative, ER-positive, and PR-positive cancer and ER-negative, PR-negative, and HER2-negative cancer (triple negative cancer), and is preferably ER-negative, PR-negative, and HER2-negative breast cancer (triple negative breast cancer (TNBC)).

The cancers in the present description may be those expressing EphA3. Human EphA3 is a 110 kDa protein consisting of 983 amino acids, also known as EK4, ETK, HEK, ETK1, HEK4, TYRO4, and belongs to the Ephrin receptor subfamily of the Eph receptor tyrosine kinase (RTK) family (NCBI Reference Sequence: NP_005224.2). A structure of EphA3 is well conserved between humans and mice, with about 96% homology and 88% similarity. EphA3 is composed of an N-terminal globular ligand-binding domain in the extracellular region, a regulatory juxtaposed membrane domain having two tyrosine residues, a tyrosine kinase domain, and a PDZ-binding motif in the intracellular cytoplasmic region.

Whether or not cancer cells express HER2, ER, PR, and EphA3 can be determined by an immunohistochemical (IHC) method, a fluorescence in situ hybridization (FISH) method, a CISH method, an SISH method, a Southern blot method, a PCR method, a Northern blot method, gene amplification by an RT-PCR method, an IHC method, a Western blot method, an ELISA method, a chemical fluorescence immunoassay method (CLIA method), or the like, using an antibody that recognizes these proteins and a nucleic acid complementary to DNA or RNA encoding these proteins.

Furthermore, the cancer to be treated or prevented according to the present invention may be a cancer having a low sensitivity to an anticancer drug or exhibiting resistance to the anticancer drug. The low sensitivity to the anticancer drug may mean that the cancer does not shrink or even proliferates when the anticancer drug is brought into contact with the cancer. Furthermore, showing resistance to an anticancer drug may mean that the disease progresses even when treatment with anticancer drug is performed, or the therapeutic effect is gradually weakened and the cancer recurs due to continuous treatment with anticancer drug. As a method for examining whether or not a cancer has a low sensitivity to the anticancer drug or exhibits resistance to the anticancer drug, for example, the following methods are known: human tumor clonogenic assay (HTCA method) (Rozencweig M et al., Recent Results Cancer Res 1984; 94:1 - 7.), thymidine incorporation assay (TIA method) (Kern DH et al., Cancer Res 1985; 45:5436-41., and Tanigawa N et al., Eur J Cancer 1992; 28:31-4.), succinic dehydrogenase inhibition test (SDI method) (Fruehauf JP et al., Cancer Principle Prac Oncol 1993; 7:12.), 3-[4,5-dimethylethiazol -2-yl]-2,5-diphenyltetrazolium bromide (MTT) assay (Carmichael J et al., Cancer Res 1987; 47:936 -42.), differential staining cytotoxicity (DiSC) assay (Weisenthal LM et al., Oncol Huntingt 1991; 5:93-103.), 3-dimensional agarose -based extreme drug resistance assay (EDRA) (Fruehauf JP et al., Contrib Gynecol Obstet 1994; 19:39-52.), histoculture drug response assay (HDRA method) (Furukawa T et al., Clin Cancer Res 1995; 1:305-11.), Collagen gel Droplet embedded culture. Drug Sensitivity Test (CD-DST method) (Yuuki Takamura et al., Int. J. Cancer: 98, 450-455 (2002)). For example, in the CD-DST method, isolated floating cells obtained by enzymatically treating a tumor tissue are cultured in a culture vessel coated with a collagen gel; a mixture of the obtained tumor cells and a collagen solution is gelled and an anticancer drug is added; after serum-free culture, tumor living cells remaining in the collagen gel drop are stained by Neutral Red staining; mixed fibroblasts are erased on an image by image analysis; and the number of cells of only the remaining cancer cells is measured. Thereby, the sensitivity to the anticancer drug is evaluated from a relative growth ratio between the anticancer drug added group and the non-added group. Alternatively, an anticancer drug is administered to the cancer patients, and the treatment progress is observed. In a case where a low therapeutic effect by the anticancer drug is not recognized, it can be determined that the cancer has a low sensitivity to the anticancer drug or exhibits resistance to the anticancer drug.

The resistance to the anticancer drug in the present description may be caused by IL-26. "IL-26" is a member of the IL-10 cytokine family identified as AK155, and is a molecule composed of 171 amino acids with a calculated molecular weight of about 19 kDa, and present as a 36 kDa homodimer in human serum (Human IL-26 precursor NCBI Reference Sequence: NP_060872.1).

Whether or not the resistance to the anticancer drug is caused by IL-26 can be determined, for example, by performing the sensitivity or the resistance test for the anticancer drug described above using cancer cells of a mouse not expressing IL-26 in the presence or absence of IL-26 (under the condition of addition or non-addition of IL-26), and determining that the resistance to the anticancer drug is caused by IL-26 in a case where the antitumor effect due to the anticancer drug is further attenuated in the presence of IL-26 (under the addition condition) as compared with the case of absence of IL-26 (under the non-addition condition). Alternatively, for a human cancer sample (a cell line or a specimen), the antitumor effect by the anticancer drug is determined in the presence or absence of an IL-26 inhibitor (siRNA molecules such as anti-IL-26 antibody or IL-26 dsRNA or IL-26 shRNA, and the like), and in a case where the antitumor effect by the anticancer drug is stronger in the presence of the IL-26 inhibitor (under the addition condition) than in the absence of the IL-26 inhibitor (under the non-addition condition), it can be determined that the resistance of the cells to the anticancer drug is caused by IL-26. Alternatively, a tumor-bearing model mouse transplanted with cancer is prepared using a human IL-26 transgenic mouse and a wild-type mouse (not expressing IL-26), the anticancer drug is administered to both mice to determine the antitumor effect, and in a case where the antitumor effect by the anticancer drug is low in the human IL-26 transgenic mouse, it can be determined that the resistance to the anticancer drug is caused by IL-26. Alternatively, in combination therewith, the expression level of IL-26 in a cancer tissue or its periphery is measured, and in a case where the expression level of IL-26 is high, it can be determined that the resistance to the anticancer drug is caused by IL-26. Alternatively, in a case where the resistance to the anticancer drug is reversed by administering an IL-26 inhibitor such as the anti-IL-26 antibody, it can be determined that the resistance to the anticancer drug is caused by IL-26. The cancer cells used in these tests may be cancer cells whose resistance to the anticancer drug is already known.

Furthermore, the resistance to an anticancer drug in the present description may be caused by the interaction between IL-26 and EphA3. The interaction between IL-26 and EphA3 refers to an action that contributes to resistance to an anticancer drug by coexistence of IL-26 and EphA3 in a cancer microenvironment, and IL-26 and EphA3 may be bound in the action mechanism thereof. Whether or not the resistance to an anticancer drug is caused by the interaction between IL-26 and EphA3 can be determined by examining whether or not the resistance to an anticancer drug due to IL-26 described above is reversed by inhibition of EphA3. For example, in a case where the sensitivity or the resistance test to the anticancer drug described above is performed in the presence of IL-26 and in the presence or absence of an EphA3 inhibitor (such as an anti-EphA3 antibody) (under the condition of addition or non-addition of an anti-EphA3 antibody), and the antitumor effect of the anticancer drug reduced by IL-26 is restored by addition of the anti-EphA3 antibody, it can be determined that the resistance to the anticancer drug is caused by the interaction between IL-26 and EphA3.

In addition, the resistance to an anticancer drug in the present description may be caused by the phosphorylation of JNK and/or the phosphorylation of AKT due to IL-26. The phosphorylation of JNK and/or the phosphorylation of AKT may be caused by the interaction between IL-26 and EphA3. Whether or not the resistance to the anticancer drug is caused by the phosphorylation of JNK and/or the phosphorylation of AKT due to IL-26 can be determined by examining whether or not the resistance to the anticancer drug due to IL-26 described above is reversed by a phosphorylation inhibitor of JNK and/or a phosphorylation inhibitor of AKT. For example, in a case where a sensitivity or a resistance test to the anticancer drug described above is performed in the presence of IL-26, in the presence or absence of the phosphorylation inhibitor of JNK and/or the phosphorylation inhibitor of AKT, and the antitumor effect of the anticancer drug reduced by IL-26 is restored by the addition of the phosphorylation inhibitor of JNK and/or the phosphorylation inhibitor of AKT, it can be determined that the resistance to the anticancer drug is caused by the interaction between IL-26 and the phosphorylation of JNK and/or the phosphorylation of AKT.

In the present description, the IL-26 may be produced by CD4+ T-cells or macrophages. Whether or not IL-26 is produced by CD4+ T-cells or macrophages can be determined, for example, by examining localization of CD4+ T-cells or macrophages and IL-26 in a tissue sample derived from the cancer patients. When the localization of CD4+ T-cells and IL-26 or macrophage and IL-26 matches, it can both be determined that IL-26 is produced by CD4+ T-cells or macrophages. The localization of IL-26 can be determined using a labeled anti-IL-26 antibody. The localization of CD4+ T-cells can be confirmed by immunostaining using a labeled anti-CD4 antibody. The localization of macrophages can be confirmed by immunostaining using a labeled anti-F4/80 antibody. By changing the color of a dye used for labeling, a plurality of factors can be simultaneously labeled, and colocalization can be confirmed as overlapping of both colors.

In the present description, the "anticancer drug" means a drug effective for treating or preventing cancer, and includes an antibody drug and an artificially synthesized compound having an anticancer effect. Examples of the anticancer drug include nitrogen mustards such as cyclophosphamide, ifosfamide, melphalan, busulfan, and thiotepa; alkylating agents such as nitroureas such as nimustine, ranimustine, dacarbacin, procarbacin, temozolomide, carmustine, streptozotocin, and bendamustine; platinum compounds such as cisplatin, carboplatin, oxaliplatin, and nedaplatin; antimetabolites such as enocitabine, capecitabine, carmofur, cladribine, gemcitabine, cytarabine, cytarabine ocphosphate, decafur, decafur/uracil, decafur/gimeracil/oteracil potassium, doxyfluridine, nelarabine, hydroxycarbamide, 5-fluorouracil (5-FU), fludarabine, pemetrexed, pentostatin, mercaptopurine, and methotrexate; plant alkaloids or microtubule inhibitors such as irinotecan, etoposide, eribulin, sobuzoxane, docetaxel, nogitecan, paclitaxel, vinorelbine, vincristine, vindesine, vinblastine; anticancer antibiotics such as actinomycin D, aclarubicin, amrubicin, idarubicin, epirubicin, dinostatin stimaramer, daunorubicin, doxorubicin, pirarubicin, bleomycin, pepromycin, mitomycin C, mitoxantrone, and liposomal doxorubicin; cancer vaccines such as Cypriucel-T; molecular target drugs such as alemtuzumab, ibritumomab tiuxetan, imatinib, everolimus, erlotinib, elotuzumab, obinutuzumab, ofamutumab, gefitinib, gemtuzumab, gemtuzumab ozogamicin, sunitinib, cetuximab, sorafenib, dasatinib, tamibarotene, trastuzumab, tretinoin, panitumumab, brentuximab, bevacizumab, pertuzumab, bortezomib, mogalizumab, lapatinib, ramucirumab, rituximab, anti-VEGF antibodies, anti-BEGFR antibodies, anti-EGFR antibodies, anti-HER2 antibodies, anti-CD20 antibodies, anti-CD52 antibodies, anti-CD33 antibodies, anti-CCR4 antibodies, anti-CD30 antibodies, anti-SLAMF7 antibodies, and anti-CD26 antibodies; immune checkpoint inhibitors such as nivolumab, pembrolizumab, ipilibumab, anti-PD-1 antibody, anti-PD-L1 antibody, and anti-CTLA-4 antibody; hormonal agents such as anastrozole, exemestane, estramustine, ethinylestradiol, octreotide, chlormadinone, goserelin, tamoxifen, dexamethasone, toremifene, bicalutamide, flutamide, brednisolone, phosphestrol, mitotane, methyltestosterone, medroxyprogesterone, mepithiostane, leuprorelin, and letrozole; biological response modifiers such as interferon α, interferon β, interferon γ, interleukin, ubenimex, dried BCG, and lentinan; and the like.

The artificially synthesized compound having an anticancer effect is preferably a molecular target drug, and is preferably a kinase inhibitor. Examples of the kinase inhibitor include tyrosine kinase inhibitors such as epidermal growth factor receptor (EGFR, HER1, ErbB1), HER2 (ErbB2, EGFR2, ERBB2, CD340, and NEU), HER3 (ErbB3), HER4 (ErbB4), VEGFR (VEGFR1, 2, and 3), RET, PDGFR (PDGFR-α, β), FGFR, KIT, c-KIT, Bcr-Abl, and FLT3; Raf kinase inhibitors (sorafenib, vemurafenib, dabrafenib, and the like); MEK inhibitors such as trametinib; CDK inhibitors (palbociclib, abemaciclib, and the like); anaplastic lymphoma kinase inhibitor (crizotinib, ceritinib, alectinib, lorlatinib, and the like); and janus kinase inhibitors such as ruxolitinib. Examples of the EGFR tyrosine kinase inhibitors include gefitinib, erlotinib, osimertinib, afatinib, dacomitinib, imatinib, dasatinib, bosutinib, vandetanib, sunitinib, axitinib, pazopanib, lenvatinib, lapatinib, nintedanib, nilotinib, and ibrutinib. gefitinib or erlotinib is preferable.

As described in the examples of the present application, since the present inventors have found that the presence of IL-26 attenuates the anticancer effect of an anticancer drug, it is considered that the resistance of cancer cells to the anticancer drug can be reversed by treatment targeting IL-26. In the present description, the "drug that reverses the resistance of IL-26 to the anticancer drug" means a drug that can enhance the sensitivity of cancer cells to the anticancer drug by weakening or losing the resistance to the anticancer drug acquired by the cancer cells due to IL-26. Typically, it is an IL-26 inhibitor. Examples of the IL-26 inhibitor include siRNA molecules such as IL-26 dsRNA or shRNA, IL-26 antisense, anti-IL-26 antibodies, aptamers against IL-26, and the like. Preferably, it is the anti-IL-26 antibody or the aptamer against IL-26, which is capable of inhibiting IL-26 in particular in a cancer microenvironment. Furthermore, since it was shown that IL-26 contributes to the resistance to an anticancer drug through interaction with EphA3, a drug that reverses resistance to the anticancer drug due to IL-26 is a drug that inhibits or reduces interaction between EphA3 and IL-26 in one example. The drug that inhibits or reduces the interaction between EphA3 and IL-26 is not particularly limited as long as it is a substance capable of enhancing the sensitivity to the anticancer drug by inhibiting or reducing the interaction between IL-26 and EphA3, and examples thereof include an antibody that inhibits a binding between EphA3 and IL-26 and soluble EphA3. Therefore, the anti-IL-26 antibody described above may be an antibody that further inhibits the binding of IL-26 to EphA3. Furthermore, as shown in the examples of the present application, since it was shown that the anti-EphA3 antibody restores the anticancer effect of the anticancer drug attenuated by IL-26, the anti-EphA3 antibody is also included in the drug that inhibits or reduces the interaction between EphA3 and IL-26. In addition, since IL-26 dephosphorylates EphA3, an agent that inhibits or reduces the interaction between EphA3 and IL-26 or the anti-IL-26 antibody may have a property of phosphorylating EphA3.

As to whether or not a candidate drug (for example, the anti-IL-26 antibodies) reverses resistance to an anticancer drug due to IL-26, for example, in a case where cancer cells of a mouse not expressing IL-26 is used, the antitumor effect due to the anticancer drug in the presence or absence of a drug to be tested in the presence of IL-26 (under the condition of addition of IL-26) is determined using the cancer cells exhibiting resistance to the anticancer drug in the presence of IL-26 (under the condition of addition of IL-26), and in a case where the antitumor effect due to the anticancer drug is higher in the presence of the drug to be tested than in the absence of the drug to be tested, it can be determined that the drug to be tested reverses the resistance to the anticancer drug due to IL-26. Alternatively, in a case where a drug-resistant human cancer sample (a cell line or a specimen) expressing IL-26 is used, the antitumor effect of the anticancer drug on the sample is determined in the presence or absence of the drug to be tested, and in a case where the antitumor effect of the anticancer drug is stronger in the presence of the drug to be tested than in the absence of the drug to be tested, it can be determined that the drug to be tested reverses the resistance of IL-26 to the anticancer drug. Alternatively, it can be determined that the resistance to the anticancer drug due to IL-26 is reversed when the antitumor effect is high in the mouse to which the drug to be tested is administered, compared with the mouse to which the drug to be tested is not administered by administering the anticancer drug under the conditions of administration and non-administration of the drug to be tested using a cancer-bearing model mouse in which the drug-resistant cancer is transplanted to the human IL-26 transgenic mouse.

The present inventors have already reported anti-IL-26 antibodies. In one example, the anti-IL-26 antibody in the present description is a clone 20-3, 31-4, and 69-10 antibody produced from a hybridoma deposited under Accession Nos. NITE P-02577, NITE P-02578, NITE P-02579, or NITE P-02580, or an antibody having complementarity determining regions of a heavy chain and a light chain thereof, or an antibody having variable regions of a heavy chain and a light chain thereof.

In another embodiment, the anti-IL-26 antibody is an antibody including a heavy chain variable region (each of the first 19 amino acids is a signal sequence, and the mature protein may not contain a signal sequence), including an amino acid sequence described in SEQ ID NO:2 or 12 and a light chain variable region having an amino acid sequence described in SEQ ID NO:4 or 14 (the first 19 or 20 amino acids, respectively, are signal sequences, and the mature protein may not contain a signal sequence), respectively, or an antibody having complementarity determining regions of a heavy chain and a light chain thereof.

In certain embodiments, the anti-IL-26 antibody contains both a heavy chain variable region having an amino acid sequence with at least 80%, 85%, 90%, 95%, 96%, 97%, 98%, or 99% identity to an amino acid sequence described in SEQ ID NO:2 or 12 and a light chain variable region having an amino acid sequence with at least 80%, 85%, 90%, 95%, 96%, 97%, 98%, or 99% identity to an amino acid sequence described in SEQ ID NO:4 or 14.

For example, the anti-IL-26 antibody may be an antibody having CDRH3 described in SEQ ID NO:7, and preferably may be an antibody having CDRH1, CDRH2, and CDRH3 with amino acid sequences described in SEQ ID NOs:5, 6, and 7, respectively. The antibody may further have the amino acid sequences described in SEQ ID NOs:8, 9, and 10 as CDRL1, CDRL2, and CDRL3.

For example, the anti-IL-26 antibody may be an antibody having CDRH3 described in SEQ ID NO:17, and preferably may be an antibody having CDRH1, CDRH2, and CDRH3 having amino acid sequences described in SEQ ID NOs:15, 16, and 17, respectively. The antibody may further have the amino acid sequences described in SEQ ID NOs:18, 19, and 20 as CDRL1, CDRL2, and CDRL3.

Furthermore, the present inventors have succeeded in obtaining the humanized anti-IL-26 antibody that can be administered to humans and exhibits excellent IL-26 binding activity as compared with the mouse antibody. The humanized anti-IL-26 antibody may be an antibody that binds to IL-26 or an antigen-binding fragment thereof. In this antibody, a heavy chain variable region (VH) has an amino acid sequence with 80% or more of identity to the amino acid sequence described in SEQ ID NO:25, and a light chain variable region (VL) has an amino acid sequence with 80% or more of identity to the amino acid sequence described in SEQ ID NO:30, or has an amino acid sequence with 80% or more of identity to the amino acid sequence described in SEQ ID NO:35. Preferably, the humanized anti-IL-26 antibody is an antibody whose VH has the amino acid sequence described in SEQ ID NO:25 and whose VL has the amino acid sequence described in SEQ ID NO:30 or the amino acid sequence described in SEQ ID NO:35, or an antigen-binding fragment thereof. For example, the humanized anti-IL-26 antibody may be an antibody or an antigen-binding fragment thereof. In the antibody, the heavy chain has the amino acid sequence described in SEQ ID NO:37 at positions 20 to 472 and the light chain has the amino acid sequence described in SEQ ID NO:39 at positions 21 to 234 or the amino acid sequence described in SEQ ID NO:41 at positions 21 to 234.

The anti-IL-26 antibody and the anti-EphA3 antibody used in the present description specifically bind to IL-26 and EphA3, respectively. The anti-IL-26 antibody includes an antibody that binds to the same epitope as at least one antibody selected from the 20-3 antibody, the 31-4 antibody, and the 69-10 antibody that are mouse monoclonal antibodies. The anti-IL-26 antibody may be an antibody that inhibits the binding of at least one antibody selected from the 20-3 antibody, the 31-4 antibody, and the 69-10 antibody, which are mouse monoclonal antibodies, to IL-26 in a competition assay.

Methods for determining affinity are known in the art. For example, binding affinity may be determined using a BIAcore biosensor, a KinExA biosensor, a scintillation proximity assay, ELISA, ORIGEN immunoassay (IGEN), fluorescence quenching, fluorescence transfer, and/or yeast display. The binding affinity may also be screened using appropriate bioassays.

One way to determine the binding affinity of an antibody for IL-26 is to measure the affinity of a monofunctional Fab fragment of the antibody. To obtain monofunctional Fab fragments, antibodies, e.g., IgG, can be cleaved with papain or expressed by recombinant techniques. The affinity of an anti-IL-26 Fab fragment of a monoclonal antibody can be determined by a surface plasmon resonance (SPR) system (BIAcore 3000 (trademark), BIAcore, Piscaway, NJ) . SA chips (streptavidin) are used according to the supplier's instructions. Biotinylated IL-26 can be diluted in HBS-EP (100 mM HEPES pH 7.4, 150 mM NaCl, 3 mM EDTA, 0.005% P20) and injected onto the chip at a concentration of 0.005 mg/mL. Using variable flow times across individual chip channels, two ranges of antigen density are achieved: 10 to 20 response units (RU) for detailed kinetic testing and 500 to 600 RU for concentration. The mixture of Pierce elution buffer and 4M NaCl (2 : 1) efficiently removes bound Fab while preserving the activity of IL-26 on the chip for 200 or more injections. HBS-EP buffer can be used for all BIAcore assays as a running buffer. A step-wise diluted solution of purified Fab samples (0.1 to 10× estimated KD) is injected at 100 µL/min for 2 min and a dissociation time up to 30 min is usually tolerated. Concentration of Fab protein can be determined by ELISA and/or SDS-PAGE electrophoresis using known concentrations (determined by amino acid analysis) of standard Fab. A binding rate (kon) and a dissociation rate (koff) of reaction kinetics are simultaneously obtained by fitting the data to a 1 : 1 Langmuir binding model (Lofas & Johnsson, 1990) using the BIA evaluation program. An equilibrium dissociation constant (KD) value is calculated as koff/kon.

The competition assay can be used to determine whether two antibodies bind to the same epitope by recognizing the same or sterically overlapping epitope. Usually, the antigen is immobilized on a multi-well plate, and the performance of the unlabeled antibody to block the binding of the labeled antibody is measured. Common labels for such competition assays are radiolabels or enzyme labels. Furthermore, epitopes to which an antibody binds can be determined by using epitope mapping techniques known to those skilled in the art. For example, whether or not an antibody to be tested competes with at least one antibody selected from 20-3 antibody, 31-4 antibody and 69-10 antibody in binding to IL-26 can be determined by contacting at least one antibody selected from labeled 20-3 antibody, 31-4 antibody and 69-10 antibody with an antigen (IL-26) together with the antibody to be tested. Preferably, as a control, one obtained by contacting at least one antibody selected from 20-3 antibody, 31-4 antibody and 69-10 antibody labeled in the absence of the antibody to be tested with an antigen (IL-26) alone is used. In a case where a labeling amount in the presence of the antibody to be tested is smaller than the labeling amount in the absence of the antibody to be tested by comparing the labeling amount of at least one antibody selected from the labeled 20-3 antibody, 31-4 antibody and 69-10 antibody bound to the antigen in the presence or absence of the antibody to be tested, it can be determined that the antibody to be tested competes with the labeled antibody used.

In the present description, the "antibody" is an immunoglobulin molecule capable of specifically binding to a target such as a carbohydrate, polynucleotide, lipid, or antibody via at least one antigen recognition site located in a variable region of the immunoglobulin molecule. As used in the present description, the term antibody includes intact polyclonal or monoclonal antibodies as well as fragments thereof. It is known that a variable region (in particular, CDRs) of the antibody imparts a binding property, and it is widely known to those skilled in the art that even an antibody fragment that is not a complete antibody can use the binding property. In the present description, the "fragment" or "antigen-binding fragment" of an antibody means a protein or peptide containing a part (partial fragment) of an antibody and retaining the action (immunoreactivity/binding property) of the antibody on the antigen. Examples of such immunoreactive fragments include F(ab') ₂, Fab', Fab, Fab₃, single chain Fv (in the present description hereinafter, referred to as "scFv"), (tandem) bi-specific single chain Fv(sc(Fv)₂), single chain triple body, nanobody, divalent VHH, pentavalent VHH, minibody, (double chain) diabody, tandem diabody, bi-specific tribody, bi-specific bibody, dual affinity retargeting molecule (DART), triabody (or tribody), tetrabody (or [sc(Fv)₂]₂ or (scFv-SA)₄) disulfide-bonded Fv (in the present description hereinafter, referred to as "dsFv"), compact IgG, heavy chain antibody, or polymers thereof (Nature Biotechnology, 29(1): 5-6 (2011); Maneesh Jain et al., TRENDS in Biotechnology, 25(7)(2007): 307-316; and Christoph stein et al., Antibodies (1): 88-123(2012)). In the present description, the immunoreactive fragments may be any of mono-specific, bi-specific (bispecificity), tri-specific (trispecificity), and multi-specific (multispecificity). Furthermore, the fragment of the antibody may include a fragment having a length of at least about 10 amino acids, at least about 25 amino acids, at least about 50 amino acids, at least about 75 amino acids, or at least about 100 amino acids.

Antibodies include any class of antibodies such as IgG, IgA, or IgM (or subclasses thereof) and need not be a particular class. Immunoglobulins are classified into different classes according to the antibody amino acid sequence of constant domains of the heavy chain. There are five main immunoglobulin classes: IgA, IgD, IgE, IgG, and IgM, some of which may be further subdivided into subclasses (isotypes), e.g., IgG1, IgG2, IgG3, IgG4, IgA1, and IgA2. The constant domains of the corresponding heavy chains of different classes of immunoglobulins are referred to as α, δ, ε, γ, and µ, respectively. Subunit structures and three-dimensional structures for different classes of immunoglobulins are well known. Preferably, the antibody may be an IgG antibody, particularly an IgG1 antibody or an IgG2 antibody, or may be a human IgG antibody.

The antibody in the present description can be a monoclonal antibody or a polyclonal antibody, and is preferably a monoclonal antibody. As used in the present description, the term "monoclonal antibody" refers to an antibody obtained from a cell population that produces a substantially homogeneous antibody. In other words, the individual antibodies contained in the cell population are the same except for natural mutants that may be slightly present. The monoclonal antibodies are directed against a single antigenic site and are highly specific. Furthermore, in contrast to typical polyclonal antibodies containing different antigens targeting different determinants (epitopes), each monoclonal antibody targets a single determinant of the antigen. The modifier "monoclonal" indicates characteristics of the antibody as being obtained from a substantially homogeneous population of antibody-producing cells, and is not to be understood as requiring production of the antibody via a particular method. For example, the monoclonal antibodies used according to the present invention may be prepared by recombinant DNA methods as described in US Patent No. 4,816,567. The monoclonal antibodies can also be isolated, for example, from phage libraries generated using the techniques described in McCafferty et al., 1990, Nature, 348: 552-554.

The antibody in the present description can be chimeric antibodies, humanized antibodies, fully human antibodies, or animal antibodies adapted to any animal to be treated other than human, and is preferably a humanized antibody. As used in the present description, a "humanized" antibody is an antibody or fragment thereof (Fv, Fab, Fab', F(ab')₂, or other antigen-binding subsequences of antibodies, and the like) containing a minimal sequence derived from non-human immunoglobulin and a sequence derived from human. Some humanized antibodies are human immunoglobulins (recipient antibodies) in which residues from the complementarity determining region (CDR) of the receptor are replaced with residues from the CDRs of a non-human species (donor antibodies), such as mice, rats, or rabbits, having the desired specificity, affinity, and capacity. Fv framework region (FR) residues of human immunoglobulins may be substituted by corresponding non-human-derived residues. The humanized antibodies include variable regions derived from non-human species (the donor antibodies), such as mice, rats, or rabbits, having the desired specificity, affinity, and/or capacity, although residues of one or more Fv framework regions and/or one or more CDR residues may be substituted by corresponding human residues (that is, residues derived from human antibody sequences). Humanized antibodies can contain residues that are not present in the transferred original CDR or framework sequences for further improvement and optimization of antibody performance. The humanized antibodies most preferably contain at least a portion of a constant region (Fc) or domain of a human immunoglobulin (generally a human immunoglobulin). Some humanized antibodies have modified Fc regions as described in WO 99/58572. Some forms of humanized antibodies have one or more (for example, 1, 2, 3, 4, 5, 6) CDRs altered relative to the original antibody, which are also referred to as one or more CDRs "derived from" one or more CDRs of the original antibody.

In certain embodiments, the antibodies contain one or more constant regions, e.g., human constant regions. The constant regions can be a constant region of the heavy chain and/or a constant region of the light chain. The antibodies may contain a constant region having at least about 80%, at least about 85%, at least about 90%, at least about 95%, at least about 98%, or 100% identity to a human constant region. The antibodies may contain an Fc region, e.g., a human Fc region. The antibodies may contain an Fc region having at least about 80%, at least about 85%, at least about 90%, at least about 95%, at least about 98%, or 100% identity to a human Fc region.

The antibodies described in the present description may be modified, examples of such modifications include functionally equivalent antibodies and variants with enhanced or attenuated activity that do not significantly affect properties of the antibodies. Modifications of antibodies are routine manipulations in the art and need not be described in detail in the present description. Examples of modifications include conservative substitutions of amino acid residues, insertion, addition, or deletion of one or more amino acids that do not significantly degrade functional activity, or the use of chemical analogs. The insertion or addition of amino acid sequences includes addition of an amino acid to the amino and/or carboxyl terminus in length, and insertion within the sequence of a single or more amino acid residues. Examples of terminus insertions include antibodies with N-terminal methionyl residues or fused to epitope tags. Other insertion variants of antibody molecules include fusion of an enzyme or antibody that extends serum half-life of the antibody to the N-terminus or C-terminus of the antibody.

In a substituted variant, at least one amino acid residue of the antibody sequence is removed, and a different residue is inserted at the position. The sites where the utmost effect is obtained by substitutional mutagenesis include CDRs, but FRs are also intended to be changed. The conservative substitutions are shown in Table 1. Where such substitutions result in altered biological activity, more substantial changes may be introduced to screen the products, designated as "exemplary substitutions" in Table 1, or as further described below with respect to amino acid classes.

**[Table 1]**

| Original Residues | Conservative Substitutions | Exemplary Substitutions |
|---|---|---|
| Ala (A) | Val | Val; Leu; Ile |
| Arg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn; Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr: Phe |
| Tyr (Y) | Phe | Trp; Phe; Thr; Ser |
| Val (V) | Leu | Ile; Leu; Met; Phe; Ala; Norleucine |

Substantial modifications in the biological properties of antibodies are achieved by selecting substitutions that significantly alter the effect of modification on (a) the structure of the antibody backbone in the substituted region, e.g., sheet or helix conformation, (b) charges or hydrophobicity of molecules at a target site, or (c) maintenance of volume of a side chain. The residues present in a natural state are classified into the following groups based on common side chain properties:
(1) Hydrophobicity: norleucine, Met, Ala, Val, Leu, and Ile;
(2) Neutral hydrophilicity: Cys, Ser, and Thr;
(3) Acidity: Asp and Glu;
(4) Basicity: Asn, Gln, His, Lys, and Arg;
(5) Residues affecting chain orientation: Gly and Pro; and
(6) Aromatic: Trp, Tyr, and Phe.

Non-conservative substitutions are made by exchanging one member of these classes for another class. More conservative substitutions include exchanging one member of a class for another member of the same class. Amino acid substitutions in the variable region can alter binding affinity and/or specificity. For example, no more than 1 to 5 conservative amino acid substitutions are made within the CDR domain. For example, no more than 1 to 3 conservative amino acid substitutions are made within the CDR3 domain.

Substitution of any cysteine residue not involved in maintaining an appropriate conformation of the antibody (usually substituted with serine) can improve an oxidative stability of the molecule and prevent abnormal crosslinking. Conversely, particularly in a case where the antibody is an antibody fragment such as an Fv fragment, stability of the antibody can be improved by adding a cysteine bond to the antibody.

The antibodies described in the present description also include glycosylated and aglycosylated antibodies as well as antibodies with other post-translational modifications such as, glycosylation, acetylation, and phosphorylation at different sugars. The antibodies are glycosylated at conserved positions in their constant regions (Jefferis and Lund, 1997, Chem. Immunol. 65: 111-128; Wright and Morrison, 1997, TibTECH 15: 26-32). It has been reported that the absence of fucose in the Fc region of antibodies has an effect on antibody-dependent cellular cytotoxicity (ADCC).

In another embodiment, the present invention relates to a pharmaceutical composition for improving the sensitivity of cancer cells to an anticancer drug, the pharmaceutical composition containing a drug that reverses resistance to the anticancer drug due to IL-26 as an active ingredient. In addition, the present invention relates to a method for producing a pharmaceutical composition for improving the sensitivity of cancer cells to the anticancer drug, containing a drug that reverses resistance to an anticancer drug due to IL-26 as an active ingredient. Also, the present invention also relates to a drug for reversing resistance to the anticancer drug due to IL-26 for use in a method for improving the sensitivity of cancer cells to the anticancer drug. Furthermore, the present invention relates to a method for improving the sensitivity of cancer cells to an anticancer drug, the method including administering an effective amount of the anticancer drug and a drug for reversing resistance to the anticancer drug due to IL-26 to a patient in need thereof.

Also, in another embodiment, the present invention relates to a pharmaceutical composition for suppressing proliferation of cancer cells, the pharmaceutical composition containing a drug that reverses resistance to the anticancer drug due to IL-26 as an active ingredient. In addition, the present invention relates to a method for producing a pharmaceutical composition for suppressing proliferation of cancer cells, containing a drug that reverses resistance to the anticancer drug due to IL-26 as an active ingredient. The present invention also relates to a drug for reversing resistance to the anticancer drug due to IL-26 for use in a method for suppressing proliferation of cancer cells. Furthermore, the present invention relates to a method for suppressing the proliferation of cancer cells, the method including administering an effective amount of anticancer drug and a drug for reversing the resistance to the anticancer drug due to IL-26 to a patient in need thereof. The pharmaceutical composition in the present description may contain a drug that reverses resistance to the anticancer drug due to IL-26 as the only active ingredient, or may contain other active ingredients. In the therapeutic method or preventive method of the present description, a drug that reverses resistance to the anticancer drug due to IL-26 may be administered as the only active ingredient, or may be administered together with other active ingredients.

In the present description, the "cancer cells" may be cancer cells having a low sensitivity to the anticancer drug or exhibiting resistance to the anticancer drug.

In another embodiment, the present invention relates to a nucleic acid molecule having a polynucleotide encoding an anti-IL-26 antibody or immunoreactive fragment thereof as described above. Specifically, the nucleic acid molecule of the present invention may be a nucleic acid molecule having a polynucleotide encoding the amino acid described in SEQ ID NO:2 or 12 as VH and having a polynucleotide encoding the amino acid sequence described in SEQ ID NO:4 or 14 as VL. For example, the nucleic acid molecule of the present invention is a nucleic acid molecule having a polynucleotide (for example, it has a base sequence described in SEQ ID NO:1) encoding the amino acid sequence described in SEQ ID NO:2 as VH and a polynucleotide (for example, it has a base sequence described in SEQ ID NO:3) encoding the amino acid sequence described in SEQ ID NO:4 as VL; a polynucleotide encoding the amino acid sequence described in SEQ ID NO:12 as VH (for example, it has a base sequence described in SEQ ID NO:11) and a polynucleotide encoding the amino acid sequence described in SEQ ID NO: 14 as VL (for example, it has a base sequence described in SEQ ID NO:13). Alternatively, the nucleic acid molecule of the present invention may be a nucleic acid molecule containing a polynucleotide encoding a VH having a base sequence described in SEQ ID NO:24 and a polynucleotide encoding a VL having a base sequence described in SEQ ID NO:29 or SEQ ID NO:34. Furthermore, the nucleic acid molecule of the present invention may be a nucleic acid molecule containing a polynucleotide encoding a heavy chain having a base sequence described in positions 58 to 1416 of SEQ ID NO:36 and a polynucleotide encoding a light chain having a base sequence described in positions 61 to 702 of SEQ ID NO:38 or positions 61 to 702 of SEQ ID NO:34.

Furthermore, the present invention includes a vector having the nucleic acid molecule. Such a vector is not particularly limited as long as it is a vector that can be used for expression of an antibody, and can be selected according to a host using an appropriate viral vector or plasmid vector. In another embodiment, the present invention relates to host cells containing the vector. The host cells are not particularly limited as long as it is a host cell that can be used for expression of the antibodies, and examples thereof include mammalian cells (mouse cells, rat cells, rabbit cells, human cells, and the like), yeast, and microorganisms (E. coli and the like).

The "host cells" include an individual cell or cell culture that may or has already been a recipient of the vector that incorporates a polynucleotide insert. The host cells include progeny of a single host cell, and the progeny may not necessarily be identical (morphologically or in genomic DNA complement) to the original parent cell due to natural, accidental, or deliberate mutations. The host cells include cells transfected in vivo with the polynucleotides of the invention.

As used in the present description, "treatment" is an approach for obtaining beneficial or desired clinical results. For purposes of the present invention, beneficial or desired clinical results include, but are not limited to, alleviation of one or more symptoms, diminishment of extent of disease, stabilized (that is, it is not deteriorated) disease state, delay or slowing of disease progression, amelioration or palliation of disease state, and remission (partial or total), whether detectable or undetectable. The "treatment" may also mean prolonging life expectancy relative to expected life expectancy if not receiving treatment.

The "effective amount" is an amount sufficient to achieve beneficial or desired clinical results, including clinical results. The effective amount can be administered in one or more administrations. For the purpose of the present invention, the effective amount of a drug for reversing the resistance to the anticancer drug due to IL-26 or a drug for reversing the resistance to the anticancer drug due to IL-26 and an anticancer drug described in the present description is an amount sufficient to reverse resistance of the anticancer drug, delay the progression of a state related to tumor growth, or reduce a tumor size. The effective amount of the drug that reverses the resistance of the IL-26 to the anticancer drug and the other anticancer drug may vary or depend on other factors such as the patient medical history, and the drug that reverses the resistance of the IL-26 to the anticancer drug used and the type (and/or amount) of the anticancer drug used in combination, among others.

The pharmaceutical compositions in the present description may optionally contain pharmaceutically acceptable additives. When the "pharmaceutically acceptable additive" is combined with an active ingredient, the active ingredient is capable of maintaining biological activity, the "pharmaceutically acceptable additive" is non-reactive with the subject's immune system when delivered, and includes any material that is non-toxic to the subject. Examples include, but are not limited to, phosphate buffered saline, water, emulsions such as oil/water emulsions, and any standard pharmaceutical carrier such as various types of wetting agents. Preferred diluents for spray or parenteral administration are phosphate buffered saline or saline (0.9%). Compositions containing such carriers are formulated by well-known conventional methods (see, e.g., Remington's Pharmaceutical Sciences, 18th edition, A. Gennaro ed., Mack Publishing Co., Easton, PA, 1990 and Remington, The Science and Practice of Pharmacy 20th Ed., Mack Publishing, 2000).

In certain embodiments, the antibody is expressed in any organism or host cell derived from any organism, including but not limited to bacteria, yeast, plants, insects, and mammals. Specific types of cells include, but are not limited to, Drosophila melanogaster cells, Saccharomyces cerevisiae and other yeasts, E. coli, Bacillus subtilis, SF9 cells, HEK-293 cells, Neurospora, BHK cells, CHO cells, COS cells, Hela cells, fibroblasts, Schwann cell lines, immortalized mammalian bone marrow and lymphoid cell lines, Jurkat-cells, mast-cells and other endocrine and exocrine cells, and neuronal cells.

Various protein expression systems, vectors, and cell media useful for the production of antibodies are known to those skilled in the art. See, for example, WO 03/054172, WO 04/009823, and WO 03/064630, the entirety of which is incorporated in the present description by reference. In certain embodiments, a glutamine synthase (GS) expression system is used for expression of the antibody.

In one embodiment, the present invention may be a complex in which a drug that reverses resistance to the anticancer drug due to IL-26 is bound to an anticancer drug.

In another embodiment, the present invention relates to a combination drug of a drug for reversing the resistance to an anticancer drug due to IL-26 and an anticancer drug. Therefore, the present invention is a pharmaceutical composition for treating cancer, including a pharmaceutical composition containing (i) an anticancer drug and (ii) a drug that reverses resistance to an anticancer drug due to IL-26. The drug that reverses resistance to the anticancer drug due to IL-26 and the anticancer drug can be contained in the same preparation or in separate preparations. The drug that reverses resistance to the anticancer drug due to IL-26 can be administered simultaneously with the anticancer drug, sequentially, after administration of the anticancer drug or before administration of the anticancer drug. The pharmaceutical composition that is a combination drug may contain only (i) an anticancer drug and (ii) a drug that reverses resistance to an anticancer drug due to IL-26, or may contain other active ingredients.

Furthermore, in another embodiment, the present invention relates to a kit for treating cancer, containing (i) an anticancer drug and (ii) a drug that reverses resistance to the anticancer drug due to IL-26. In the present description, the kit can include an outer box, a container, a diluent, a turbid solution, and/or instructions on a preparation method and an administration method. In the kit, different components among (i) the anticancer drug and (ii) the drug reversing the resistance to the anticancer drug due to the IL-26 may be packaged in separate containers and included in one kit, or only one component among (i) the anticancer drug and (ii) the drug reversing the resistance to the anticancer drug due to the IL-26 may be included in the kit, and another component may be provided separately from the kit.

The pharmaceutical composition of the present invention is useful for various applications including a therapeutic method and a method for lysing malignant cells, but is not limited thereto. The present invention also provides a method for inhibiting the proliferation of cancer cells, wherein the inhibition of the proliferation of cancer cells contains any observable level of inhibition, including partial or complete inhibition of proliferation. In certain embodiments, the cell proliferation is inhibited by at least about 10%, at least about 25%, at least about 50%, at least about 75%, at least about 90%, at least about 95%, at least about 98%, or about 100%. This method may be performed in vitro or in vivo. In certain embodiments, the method for inhibiting proliferation of cancer cells includes contacting the cells with a pharmaceutical composition described in the present description. Generally, the cells are contacted with a sufficient amount of a pharmaceutical composition to inhibit the cell proliferation.

The humanized anti-human IL-26 antibody or the antigen-binding fragment thereof of the present invention is useful as a therapeutic agent for various diseases caused by inhibiting the activity of human IL-26 and enhancing the IL-26 activity. Here, examples of diseases associated with IL-26 include refractory immune disorders and cancers. Specific target diseases include diseases selected from acute or chronic graft-versus-host disease, post-transplant rejection, autoimmune diseases, atopic dermatitis, cancers, idiopathic pulmonary hypertension, chronic obstructive pulmonary disease, idiopathic pulmonary fibrosis, bronchiolitis obliterans, viral hepatitis, liver cirrhosis, nephrosclerosis, chronic glomerulonephritis, and idiopathic nephrotic syndrome. Here, examples of the autoimmune diseases include diseases selected from rheumatoid arthritis, systemic lupus erythematosus, psoriasis, psoriatic arthritis, polymyositis, dermatomyositis, scleroderma, Sjogren's syndrome, vasculitis syndrome, mixed connective tissue disease, autoimmune hepatitis, primary biliary cirrhosis, primary sclerosing cholangitis, autoimmune pancreatitis, ulcerative colitis, Crohn's disease, idiopathic thrombocytopenic purpura, autoimmune hemolytic anemia, autoimmune neutropenia, type I diabetes, pemphigus, pemphigoid, frequent miscarriage, causative diseases, and autoimmune optic neuritis. In addition, examples of the cancers include head and neck cancer, esophageal cancer, gastric cancer, colon cancer, rectal cancer, liver cancer, gallbladder/bile duct cancer, pancreatic cancer, lung cancer, breast cancer, ovarian cancer, bladder cancer, prostate cancer, bone/soft part sarcoma, malignant lymphoma, leukemia, multiple myeloma, cervical cancer, skin cancer, brain tumor, and the like.

The methods (including therapeutic methods) described in the present description may be made by a single direct injection at a single time point or multiple time points to a single site or multiple sites. The administration may also be administered to multiple sites at about the same time. Frequency of administration is determined and adjusted over a course of treatment, and is based on results desired to be achieved. In some cases, sustained release formulations of the pharmaceutical compositions of the present invention may be suitable. Various formulations and devices for achieving sustained release are well known in the art.

The subject to be treated includes mammals such as human, bovine, equine, canine, feline, porcine, and ovine, and is preferably human.

The pharmaceutical composition of the present invention may adopt any oral or parenteral preparation as long as it is a preparation that can be administered to a patient. Examples of the composition for parenteral administration include injections, nasal drops, suppositories, patches, ointments, and the like. It is preferably an injection. Examples of dosage forms of the pharmaceutical composition of the present invention include a liquid preparation and a lyophilized preparation. In a case where the pharmaceutical composition of the present invention is used as an injection, additives including solubilizing agents such as propylene glycol, ethylenediamine, and the like; buffering agents such as phosphate; isotonizing agents such as sodium chloride, glycerin, and the like; stabilizers such as sulfite; preservatives such as phenol; and soothing agents such as lidocaine, can be added as necessary (refer to "Handbook of Pharmaceutical Excipients Fifth Edition" APhA Publications, Yakuji Nippo Limited). In addition, when the pharmaceutical composition of the present invention is used as an injection, examples of storage containers can include an ampoule, a vial, a prefilled syringe, a pen-type syringe cartridge, a drip bag, and the like.

In another embodiment, the present invention relates to a method for improving the responsiveness to the anticancer drug in cancer patients having a low sensitivity to the anticancer drug or exhibiting resistance to the anticancer drug, the method including inhibiting IL-26 in the cancer patients. The method may include inhibiting or reducing the interaction between EphA3 and IL-26.

Furthermore, in another embodiment, the present invention is a method for treating cancer in cancer patients having a low sensitivity to the anticancer drug or exhibiting resistance to the anticancer drug, the method including inhibiting IL-26 in the cancer patients and administering the anticancer drug to the patients. The method may include inhibiting or reducing the interaction between EphA3 and IL-26. In the present method, the order of the inhibition of IL-26 and the administration of the anticancer drug in the cancer patients does not need to be this order, and either may precede. In other words, the anticancer drug may be administered after the inhibition of IL-26, IL-26 may be inhibited after the administration of the anticancer drug, or both may be performed simultaneously or sequentially.

In these methods of the present invention, the inhibition of IL-26 in the cancer patients is preferably an inhibition of IL-26 in a microenvironment of the cancer to be treated. In addition, preferably, the inhibition of IL-26 is performed by administering a pharmaceutical composition containing a drug that reverses resistance to the anticancer drug due to IL-26 of the present invention as an active ingredient.

The pharmaceutical composition of the present invention may be administered to a mammal by injection (for example, systemically, intravenously, intraperitoneally, subcutaneously, intramuscularly, intraportally, or into cancer tissues) or by other methods that ensure delivery to bloodstream in an effective form (for example, injection).

The effective dose and schedule for administering the pharmaceutical compositions of the present invention are empirically determined, and such methods of determination are within the common general knowledge in the art. Those skilled in the art will appreciate that the dose of the pharmaceutical composition of the present invention to be administered varies depending on, for example, a mammal inoculating the pharmaceutical composition of the present invention, a route of administration, a specific type of agent used, and other agents administered to the mammal. A typical dosage per day of the pharmaceutical composition of the present invention may range from about 1 µg/kg body weight to 100 mg/kg body weight or more per day, depending on the aforementioned factors. In general, any of the dosages below may be used: at least about 50 mg/kg body weight; at least about 10 mg/kg body weight; at least about 3 mg/kg body weight; at least about 1 mg/kg body weight; at least about 750 µg/kg body weight; at least about 500 µg/kg body weight; at least about 250 µg/kg body weight; at least about 100 µg/kg body weight; at least about 50 µg/kg body weight; at least about 10 µg/kg body weight; a dosage of at least about 1 µg/kg body weight, or more, is administered.

The pharmaceutical composition of the present invention may be further administered in combination with one or more therapeutic agents other than the anticancer drug in an effective amount to a mammal. The pharmaceutical compositions of the present invention may be administered sequentially or simultaneously with one or more other therapeutic agents. After administration of the pharmaceutical composition of the present invention to a mammal, physiological conditions of the mammal can be monitored in various ways well known to those skilled in the art.

in the present description hereinafter, the examples will be shown to describe the present invention in more detail, but the present invention is not limited thereto. In addition, all documents cited throughout the present application are incorporated herein by reference as they are.

### Materials and methods)

### (1) Cells and reagents

Mouse breast cancer E0771 cells (mouse TNBC cell lines) were purchased from CH3 Biosystems (Amherst, NY). Human mammary gland cancer cell lines MDA-MB-468 (human TNBC cell line), HCC70 (human TNBC cell line), and T47D (ER/PR-positive, HER2-negative cell line) were obtained from ATCC. The human large intestine cancer cell line COLO 205 was purchased from RIKEN BioResource Center (Tsukuba, Japan). Human umbilical vein endothelial cells HUVECs were purchased from LONZA (Walkersville, MD, USA) and cultured in EGM medium (LONZA). For human umbilical cord blood mononuclear cells, mononuclear cells isolated from human umbilical cord blood by a Ficoll density gradient method were purchased from RIKEN BioResource Center. Recombinant human IL-26 dimers for cell stimulation were purchased from R&D Systems (Minneapolis, MN). The recombinant human IL-26 dimers were labeled with Alexa Fluor 488 using a protein labeling kit (Thermo Fisher Scientific, Waltham, MA) and used for immunofluorescence staining. Recombinant human and mouse EphA3 Fc chimeric proteins were purchased from R&D Systems. EGFR-TKI and the inhibitor used in this example are shown in Table 2. A negative control siRNA (oligonucleotide sequence not disclosed) was purchased from Qiagen (Hilden, Germany).

**[Table 2]**

| EGFR-TKI and inhibitor list | | | |
|---|---|---|---|
| Product name | The primary target of the inhibition | ^{∗} Conc | Company |
| Gefitinib (ZD1839) | EGFR | 5-40 µM | Selleck Chemicals (Houston, TX, USA). |
| Erlotinib (CP358774) | EGFR | 30, 50 µM | Selleck Chemicals |
| wortmannin | Akt | Low: 0.375, Mid: 0.75, High: 1.5 µM | Merck Millipore |
| SP600125 | JNK | Low: 0.3, Mid: 1.0, High: 3.0 µM | Tocris Biosystems |

### 2) Antibodies

As the anti-IL-26 monoclonal antibody, clones 69-10, 20-3, 31-4, and 2-2 mAbs described in Hatano R et al., MAbs (2019) 1-15, and JP 2009-99492 A were used. Hybridomas producing clones 69-10, 20-3, 31-4, and 2-2 mAbs have been deposited with National Institute of Technology and Evaluation Patent Microorganisms Depositary (NPMD) under Accession Nos. NITE P-02577, NITE P-02578, NITE P-02579, and NITE P-02580, respectively, in Kazusakamatari 2-5-8, Kisarazu-shi, Chiba, Japan on November 21, 2017.

Other antibodies used in this experiment are shown in Table 3.

**[Table 3]**

| **Antibody list** | | |
|---|---|---|
| **Cell proliferation assay** | | |
| | Mouse IgG_{l,κ} isotype control mAb (clone MG 1-45) | BioLegend |
| | goat anti-mouse EphA3 pAb | R&D Systems |
| | anti-human IL-26 mAbs (clone 2-2, 20-3, 31-4, 69-10) | Original (mAbs 2019) |
| | goat anti-human IL-26 pAb | R&D Systems |

| **Immunofluorescence staining** | | |
|---|---|---|
| | mouse anti-human/mouse EphA3 mAb (clone D-2) | Santa Cruz Biotechnology (Santa Cruz, CA) |
| | Biotin-conjugated anti-human IL-26 mAb (clone 69-10) | Original *(*mAbs 2019) |
| | rat anti-mouse CD4 mAb (clone GK1.5) | Thermo Fisher Scientific |
| | rat anti-mouse CDS mAb (clone EP1150Y) | abcam |
| | rat anti-mouse F4/80mAb (clone CI:A3-1) | abcam |
| | Alexa Fluor 647-conjugated donkey anti-rat IgG pAb | Thermo Fisher Scientific |
| | Alexa Fluor 488-conjugated streptavidin | Thermo Fisher Scientific |
| | Alexa Fluor 647-conjugated goat anti-mouse IgG pAb | Thermo Fisher Scientific |

| **Western blot analysis** | | |
|---|---|---|
| | rabbit anti-Akt pAb | Cell Signaling Technology (Danvers, MA) |
| | rabbit anti-phospho-Akt (Ser473) pAb | Cell Signaling Technology |
| | rabbit anti-STAT3 pAb | Cell Signaling Technology |
| | rabbit anti-phospho-STAT3 (Tyr705) pAb | Cell Signaling Technology |
| | rabbit anti-JNK/SPAK pAb | Cell Signaling Technology |
| | rabbit anti-phospho-JNK/SPAK (Thr183/Tyr185) mAb (clone 81E11) | Cell Signaling Technology |
| | mouse anti-human/mouse EphA3 mAb (clone D-2) | Santa Cruz Biotechnology |
| | mouse anti-human/mouse EphA3 (Y596) mAb (clone EPR3434(N)) | abeam |
| | rabbit anti-β-Actin mAb (clone 13E5) | Cell Signaling Technology |
| | HRP-Linked sheep anti-mouse IgG | GE Healthcare |
| | HRP-Linked Donkey anti-rabbit IgG | GE Healthcare |

| **Flow cytometry** | | |
|---|---|---|
| | rabbit anti-mouse IL-20RA pAb | LifeSpan BioSciences (Seattle. WA) |
| | APC anti-mouse IL-10RB mAb (clone 547324) | R&D Systems |
| | APC anti-human IL-20RA mAb (clone 173714) | R&D Systems |
| | PE anti-human IL-10RB mAb (clone 90220) | R&D Systems |
| | rabbit IgG. polyclonal - Isotype Control | abeam |
| | APC rat IgG2a, Isotype Control (clone RTK2758) | BioLegend |
| | APC mouse IgG1, Isotype Control (clone MOPC-21) | BioLegend |
| | PE mouse IgG1, Isotype Control (clone MOPC-21) | BioLegend |
| | PE anti-rabbit IgG | BioLegend |

| **Immunohistochemistry** | | |
|---|---|---|
| | rabbit anti-human IL-26 mAb (clone EPR22268-141) | abcam |
| | rabbit anti-human EphA3 pAb | Gene Tex (San Antonio. TX) |
| | HRP-Linked Goat anti-rabbit IgG | abeam |

### 3) Mice

The human IL-26 transgenic mouse (hIL-26Tg mouse) from Thomas

Provided by Dr. Aune, it was crossed with C57BL/6 mouse in an animal facility at Juntendo University. A female hIL-26Tg mouse of 8-12 weeks old and littermate control mice were used. NOD/Shi-scid, IL-2RγKO Jic (Cg-PrkdcscidI12rgtm1Sug/Jic) mice (in the present description hereinafter, NOG mice), ♀ were purchased from Central Institute for Experimental Animals (Kawasaki, Japan). All mice used in this experiment were housed under specific sterile equipment in a microisolator cage. Animal experiments were performed in accordance with protocols approved by the Institutional Animal Care and Use Committee.

### 4) MTT assay

Human and mouse breast cancer cells (1×10⁴) were cultured in 10% FCS containing RPMI1640 medium on a 96-well flat bottom plate (Corning, Tewksbury, MA) at 37°C for 24 hours, and then stimulated with IL-26 for 48 hours in the presence or absence of EGFR-TKI (gefitinib or erlotinib) at various concentrations, a signal inhibitor, and a neutralizing antibody. HUVECs were seeded in a 96-well flat bottom plate at 5×10³ cells/well in a 2% FBS-containing EGM medium in which growth factors were reduced by half, and cultured at 37°C for 12 hours. Thereafter, the medium was aspirated, and a recombinant human IL-26 dimer was adjusted to a final concentration of 10 ng/mL in a 2% FBS-containing EGM medium containing no growth factors, and added to the cells. The stimulation was started in the presence or absence of control human IgG and anti-human IL-26 antibodies, and the cells were cultured for 48 hours. Thereafter, a concentration of 20 µl or 1/10 of a 5 mg/ml MTT solution (Tetrazolium monosodium salt, WST-8) was added to each well, and the plate was allowed to stand for 1 hour. The absorbance at 450 nm/595 nm was measured using Microplate Reader (Bio-Rad, Hercules, CA) and data was analyzed with Microplate Manager 6 software (Bio-Rad).

### 5) Cell proliferation assay

Human and mouse breast cancer cells (1×10⁴) were cultured in RPMI1640 medium containing 10% FCS for 24 hours at 37°C on a 96-well flat bottom plate (Corning), and then stimulated with IL-26 (30 ng/ml) in the presence or absence of various concentrations of gefitinib and a neutralizing antibody. Cell proliferation was measured as cell confluence using IncuCyte ZOOM (Essen Bioscience, Ann Arbor, MI).

### 6) Western blotting

To analyze the phosphorylation of AKT, ERK, p38, JNK, STAT3, and EphA3, E0771 cells and HCC70 cells in 100 mm dishes were stimulated with IL-26 (30 ng/ml) in the presence or absence of gefitinib (10 µM) and an anti-EphA3 polyclonal antibody (pAb) (10 µg/ml) for a specified period of time. After stimulation, the cells were lysed with a 2% protease inhibitor mixture (Sigma-Aldrich) and/or RIPA buffer added with 1×PhosSTOP (Roche Diagnostics, Tokyo, Japan), and a lysate was separated in a reduced state by SDS-PAGE (15 µg/lane). Immunoblots were performed using an anti-phosphorylated AKT antibody, an anti-phosphorylated ERK antibody, an anti-phosphorylated p38 antibody, an anti-phosphorylated JNK antibody, an anti-phosphorylated STAT3 antibody, and an anti-phosphorylated EphA3 antibody that recognize both human and mouse antigens. For reprobing, the membrane was immersed in a stripping buffer. After the stripping procedure, the membranes were re-probed with anti-pan-AKT, anti-pan-ERK, anti-pan-p38, anti-pan-JNK, anti-pan-STAT3, and anti-pan-EphA3 antibodies recognizing both human and mouse antigens. Images were taken using a C-Digit Blot scanner (MS Techno Systems, Inc., Japan).

### 7) Flow cytometry

E0771 cells and HCC70 cells were washed with PBS containing 1% FBS and 0.1% sodium azide, and then stained with fluorochrome-labeled antibodies (anti-IL-20RA antibody and anti-IL-10RB antibody) at 4°C for 30 minutes. The cells were incubated with purified rabbit anti-human/mouse EphA3 antibody for 30 min at 4°C, followed by staining with PE-conjugated donkey anti-rabbit IgG (BioLegend) for 25 min at 4°C. The flow cytometry was performed on a two-laser FACSCalibur (BD Biosciences) and data was analyzed on FlowJo software (BD Biosciences).

### 8) ICAM-1 expression analysis of COLO205

COLO205 was seeded at 5×10⁴ cells/well in a 96-well flat bottom plate, and stimulated with recombinant human IL-26 monomer (20 ng/mL) in the presence or absence of control human IgG or anti-human IL-26 antibody. After 24 hours of culture, the cells were collected and washed with ice-cold PBS (FCM buffer) containing 1% FBS and 0.1% sodium azide. Thereafter, a solution prepared by diluting a PE-labeled mouse anti-human ICAM-1 monoclonal antibody (clone 15.2, catalog number 50-0549-T100) of Bay bioscience (Kobe, Japan) or a PE-labeled mouse IgG_{1,κ} isotype control monoclonal antibody (clone MOPC-21, catalogue number 400114) of BioLegend with FCM buffer to 5 µg/mL, was added at 50 µL/sample, and the mixture was allowed to stand at 4°C for 25 minutes. Thereafter, the cells were washed with ice-cold FCM buffer, then measured with FACSCalibur, and the obtained data was analyzed with FlowJo.

### 9) Microarray analysis

E0771 cells were treated with the exogenous IL-26 (30 ng/ml) for 6 or 24 hours. Total RNA was isolated and DNA microarray analysis was performed using 3D-Gene mouse mRNA oligo chip (Toray Industries, Inc., Japan). A heat map (magnification >= 2.0) of changes in 960 genes with variable expression between IL-26 treated E0771 cells and control vehicle treated E0771 cells was constructed by the hierarchical cluster analysis using Cluster 3.0 software, and the results were displayed in the TreeView program. The data described in this patent application is provided at the NCBI Gene Expression Omnibus and can be accessed from accession number GSE147804 of the GEO series. HCC70 cells were treated with the exogenous IL-26 (30 ng/ml) in the presence or absence of gefitinib (40 µM) for 3 hours. Total RNA was subjected to DNA microarray analysis using 3D-Gene human mRNA oligo chip. A heat map of 943 genes differentially expressed (magnification >= 2.0) between HCC70 cells treated with vehicle, IL-26, gefitinib and vehicle, and gefitinib and IL-26 was created and displayed in the same manner as described above. The gene category portions identified by microarray Gene Ontology (GO) enrichment analysis were displayed as GO terms in descending order of correlation coefficient values.

### 10) In vivo evaluation of IL-26 in TNBC transplantation model

E0771 cells (5×10⁵) with 50% Matrigel were injected subcutaneously into the flank of hIL-26Tg mice or control mice. Tumor measurements were performed using caliper and volume was calculated using an equation (v = width × width × (length/2)). gefitinib was suspended in H₂O with 1% tween (registered trademark) 80 for oral administration (50 mg/kg). Ten days after injection of E0771 cells, the oral administration once a day was performed 5 times a week. As a control, 1% tween (registered trademark) 80 containing H₂O without Gefiinib was administered. The monoclonal antibody (mAb) treatment was performed by diluting anti-IL-26 mAb (clone 69-10) (Hatano R et al., MAbs (2019) 1-15, and JP 2009 -99492 A) or mouse IgG1 isotype control to 1 mg/ml with sterile PBS, and by intraperitoneal injection of 200 µl (200 µg) once a day twice a week starting on day 10 after E0771 cell injection. Mice were sacrificed until subcutaneous tumor size reached 1600 mm³.

### 11) Heterogeneous chronic GVHD model

On day -1, NOG mice were irradiated with a sublethal dose (100 cGy). On day 0, 5×10⁶ human umbilical cord blood mononuclear cells were transplanted from the tail vein of NOG mice. Survival of the mice was assessed daily and body weight was measured twice a week. In an antibody treatment experiment, the control human IgG or anti-human IL-26 monoclonal antibody (the mouse antibody m69-10 and the humanized antibody h69-10A) was administered from day +28 of transplantation when clinical signs/symptoms of mild GVHD began to appear. The antibody solution was adjusted to a concentration of 1 mg/mL with sterile PBS, and 200 µL (200 µg) of the antibody solution per mouse was intraperitoneally injected twice a week. On day +56 after transplantation, the mice were sacrificed, and the skin, lung, liver, and large intestine were collected.

### 12) Histological assessment

NOG mice were dissected, and then the skin, lung, liver, and large intestine on the back were excised, fixed in 10% formalin solution (Wako) at room temperature, and embedded in paraffin. Thereafter, a section having a thickness of 5 µm was prepared and placed on a glass slide, and stained with hematoxylin and eosin (H&E). In addition, in order to confirm the proliferation of collagen fibers, Azan-Mallory staining was performed. Images of the optical microscope were taken with an Olympus BX41 microscope (OLYMPUS, Tokyo, Japan) connected with an Olympus digital camera DP25, and CellSens software was used.

### 13) Immunofluorescence analysis

E0771 and HCC70 cells (1×10⁵) were cultured for 24 hours in RPMI1640 medium with 10% FCS on Lab-Tek chamber slides (Thermo Fisher Scientific). The cultured cells were stimulated with Alexa Fluor 488 labeled IL-26 (30 ng/ml) in the presence or absence of recombinant human and mouse EphA3-Ig for 1 hour. HCC70 cells were stimulated with IL-26 (30 ng/ml) in the presence or absence of gefitinib (40 µM), a signal inhibitor, and a neutralizing antibody for 24 hours. Subcutaneous tumor samples obtained from mice were fixed in 4% paraformaldehyde (Thermo Fisher Scientific) and embedded in OCT compound (Tissue-Tek (registered trademark), Sakura Finetek, Japan). Cells and slides were immunostained with each antibody and observed using a Zeiss inverted microscope and Axiovision 2.0 program (Carl Zeiss, Germany). The fluorescence intensity was quantified with Image-J software (NIH). The images were captured using a 100×400x objective.

### 14) JC-1 staining

Mitochondrial stability was evaluated using the mitochondrial membrane potential (ΔΨm) assay kit (Cayman Chemical, Ann Arbor, MI, USA) utilizing JC-1 staining. HCC70 cells (1×10⁴ cells) were stimulated with IL-26 (30 ng/ml) in the presence or absence of gefitinib (40 µM), a signal inhibitor, and a neutralizing antibody in 96-well flat bottom plate (Corning) for 24 hours. After stimulation, the cells were stained with JC-1 stain. ΔΨm was evaluated with a fluorescence microscope (Nikon, Japan) at wavelengths of 530 nm and 590 nm, respectively. ΔΨm depolarization showed green fluorescence. Stained cells were quantified using Image-J software (NIH). Images were captured using a 40x objective.

### 15) Quantitative real time PCR (qRT-PCR)

E0771 cells, HCC70 cells, and MDA-MB-468 cells were cultured in RPMI1640 medium containing 10% FCS on a 6-well plate (Corning), and then stimulated with IL-26 (30 ng/ml) in the presence or absence of gefitinib for 3, 6, or 12 hours. The cells were harvested and total RNA was extracted. cDNA was synthesized using PrimeScript II first-strand cDNA synthesis kit (TaKaRa Bio, Japan) and oligo (dT) primers. The mRNA levels were measured by 7500 System SDS software (Applied Biosystems, Foster City, CA) and normalized to hypoxanthine phosphoribosyltransferase expression levels. The sequences of the primers used in this experiment are shown in Table 4.

**[Table 4]**

| **The primer sequences for RT-PCR** | | |
|---|---|---|
| Target gene (GenBank accession number) | | Oligo sequence |
| Mouse EphA3 (NM_001362452.1) | Sense | 5'-ggaatgctccgtgggatag |
| | Antisense | 5'-gctggctgaggtgaacttg |
| Mouse HPRT (NM_013556) | Sense | 5'- tgaagtactcattatagtcaagggca |
| | Antisense | 5'- ctggtgaaaaggacctctcg |

### 16) Tissue samples and immunohistochemistry

Human TNBC tissue arrays were purchased from Abcam (ab214756, UK). Hatano R et al., PLoS One; Histological and immunohistochemical methods were performed according to 14(6): e0218330 (2019). Histological studies were performed at the Department of Mammology, Juntendo University School of Medicine (Tokyo, Japan) after approval by the Institutional Review Board at Juntendo University School of Medicine. A pathological examination was performed by two experienced pathologists. In the specimens subjected to IHC, the ER state and the PgR state were semi-quantitatively determined, and when 1% or more of the nuclei of the cancer cells showed staining, they were determined to be positive. HER2 was determined to be positive when 10% or more of the tumor cells showed strong staining of the entire cell membrane. Triple-negative (ER/PgR-HER2-) status was classified by IHC staining in a case where the sample was ER (< 1%), PgR (< 1%), and HER2 (< 10%).

### 17) Statistics

Data was analyzed by two-tailed Student's t-test for two-group comparisons and one-way analysis of variance test using Tukey for multiple comparison tests. Data shows a mean value ± standard deviation of triplicate samples of representative in vitro experiments or a mean value ± standard error of three independent in vivo experiments. Significance was analyzed using MS Excel (Microsoft), and a value of p < 0.01 was considered significant and described in the drawings and the description of the figures.

### Example 1) Expression of IL-26 in tumor-infiltrating lymphocytes (TILs) in clinical specimens of TNBC

IL-26 expression in clinical specimens was assessed by immunohistochemical staining. The results are shown in FIG. 1. IL-26 was clearly detected in TNBC TILs (FIG. 1A). Although IL-26 positive TILs were observed in all subtypes (FIG. 1B), the proportion of IL-26 positive TILs was higher in TNBC and HER2 types than in luminal tumors (FIG. 1C). These data suggest that stromal immune cells produce IL-26 in TNBC, as well as in HER2 and luminal subtype breast cancer.

### Example 2) Activation of EGFR-TKI bypass pathway in human and mouse TNBC with the exogenous IL-26

Since IL-26 is present in humans but not in rodents including mice, roles of IL-26 in human disease including cancers, has not yet been determined. The present inventors have previously shown an important role of IL-26 in pulmonary fibrosis or psoriasis models utilizing hIL-26Tg mice (Ohnuma K et al. (2015), supra; Itoh T et al. (2019), supra; Hatano R et al., MAbs 2019: 1-15.; Ohnuma K et al. (2016), supra). IL-26 has been reported to play a role in gastric cancer and hepatocellular carcinoma using human samples (You W et al. (2013), supra; Xi ZF et al. (2019), supra; Xue T et al., J BUON 2019, 24(1): 215-220). However, the roles of IL-26 in the pathophysiology of breast cancer was unknown.

Using the mouse TNBC cell line E0771, the human TNBC cell line HCC70 (Crosby EJ et al., Oncoimmunology 2018, 7 (5): e 1421891.; Sameni M et al., Clin Cancer Res 2016, 22 (4): 923 -934.) and MDA-MB468, and the human breast cancer cell line T47D, the effect of the exogenous IL-26 in combination with EGFR-TKI treatment on cell proliferation was evaluated.

In both E0771 (FIGS. 2A to 2C) and HCC70 (FIGS. 3A to 3C) cells, cell proliferation was suppressed by gefitinib treatment, and the exogenous IL-26 significantly inhibited the cell proliferation suppression induced by gefitinib. In addition, the suppression of cell proliferation of the human TNBC cell lines MDA-MB468 and T47D by gefitinib was also inhibited by treatment with the exogenous IL-26 (FIGS. 4A to 4D).

The suppression of cell proliferation of E0771 by gefitinib was significantly suppressed in a dose-dependent manner by an addition of the exogenous IL-26 (FIGS. 5A and 5B). Furthermore, the exogenous IL-26 significantly inhibited the suppression of gefitinib-induced cell proliferation even in the high dose of gefitinib (FIG. 5C). On the other hand, IL-26 treatment alone did not affect cell proliferation of E0771 (FIGS. 2A, 2B and 2C). In addition, four different anti-IL-26 neutralizing mAbs, clones 2-2, 20-3, 31-4, and 69-10, reversed the inhibitory effect of IL-26 on gefitinib-induced suppression of cell proliferation (FIG. 5D).

As a result of studying signaling pathway mediators, it was found that the exogenous IL-26 activated AKT and JNK phosphorylation but did not activate STAT3 in E0771 cells (FIG. 5E). Furthermore, the addition of the exogenous IL-26 resulted in activation of AKT and JNK in spite of the treatment with gefitinib (FIG. 5F). In addition, inhibiting both AKT and JNK significantly reduced IL-26 stimulated proliferation and inhibiting either AKT or JNK partially reduced cell proliferation (FIG. 6).

Results similar to those obtained with mouse TNBC cells were also obtained with human TNBC cells (FIGS. 7, 8, and 9).

Furthermore, the exogenous IL-26 also significantly suppressed the TNBC cell proliferation inhibitory effect by erlotinib, another EGFR-TKI, at a level similar to that seen with gefitinib (FIG. 10). These results indicate that the exogenous IL-26 inhibits the proliferation inhibitory effect of EGFR-TKIs including gefitinib and erlotinib on TNBC. These findings also indicate that IL-26 induces increased the phosphorylation of AKT and JNK, thus activating the EGFR-TKI-associated bypass pathway, resulting in tumor growth of both mouse and human TNBC.

To characterize the inhibitory effect of IL-26 on gefitinib-mediated suppression, cell proliferation assays were performed under anti-IL-26 mAb treatment. As anti-IL-26 mAbs, anti-IL-26 mAbs that recognize 4 different epitopes previously established (clone 2-2, 20-3, 31-4, and 69-10) were used (Hatano et al. (2019) supra). As shown in FIG. 11, three different anti-IL-26 mAbs, clones 20-3, 31-4 and 69-10, reversed the effect of IL-26 on gefitinib-induced suppression of cell proliferation. Furthermore, a mixture of four different anti-IL-26 mAbs clearly abolished the effect of IL-26 on gefitinib-induced suppression of cell proliferation, which was comparable to the effect of the anti-IL-26 polyclonal antibody (pAb) (FIG. 11). From the above, these data showed that the exogenous IL-26 activated the EGFR-TKI bypass pathway in TNBC, and that the activation was inhibited by the anti-IL-26 antibody.

### Example 3) Identification of new functional receptors for IL-26 in TNBC

The present inventors next elucidated the signaling pathway of IL-26 in TNBC. IL-26 has been reported to activate phosphorylation of STAT1 and STAT3 via IL-20RA and IL-10RB heterodimers, which function as IL-26 receptors, in various immune cells, fibroblast keratinocytes, and gastric cancer cells (Ohnuma et al., (2015) and You W et al., (2013) supra; and Fujii M et al., World J Gastroenterol 2017, 23(30): 5519-5529.; and Hor S et al. (2004), supra). However, by flow cytometry using TNBC cell lines E0771, HCC770, MDA-MB468, and human breast cancer cell line T47D, expression of IL-10RB was detected, while expression of IL-26RA (Sheikh F et al. (2004), supra) as a main IL-20 binding chain was not detected (FIG. 12).

Furthermore, unlike the results previously reported for the activation of STAT3 by IL-26 (You W et al. (2013), supra), addition of the exogenous IL-26 to E0771 and HCC770 cells did not lead to the phosphorylation of STAT3 but did lead to the phosphorylation of AKT and JNK (FIGS. 5 and 7).

IL-26 stimulation did not induce STAT3 phosphorylation in E0771 cells, suggesting the presence of novel receptors/signaling pathways other than IL-20RA/IL-10RB-STAT3. To identify novel interacting proteins of IL-26 in TNBC cells, a pathway analysis by DNA microarray of IL-26 treated E0771 cells was performed (FIG. 13). A heat map (magnification >= 2.0) of 960 genes differentially expressed between IL-26 treated and control solvent treated E0771 cells was constructed by the hierarchical cluster analysis and the results were displayed in the TreeView program. According to this analysis, a significant reduction in the expression level of EPhA3 and altered downstream signaling of EphA3 have become clear, indicating an involvement of the EphA3 receptor signaling pathway in IL-26 treated E0771 cells.

### Example 4) Inhibition of binding of IL-26 to EphA3 due to anti-IL-26 mAb

Expression of the EphA3 receptor was also detected in E0771, HCC70, MDA-MB468 and T47D (FIG. 14 and FIG. 15).

The present inventors then investigated whether EphA3 mediates IL-26 stimulation of TNBC cells. The blocking of the EphA3 receptor by the anti-EphA3 receptor pAb significantly inhibited the proliferation of E0771 cells via IL-26 under EGFR-TKI treatment in a dose-dependent manner of the anti-EphA3 receptor pAb (FIG. 16). Immunocytochemical analysis showed that the exogenous IL-26 colocalized with the cell surface EphA3 (FIGS. 17 and 18). It was revealed that the colocalization by the EphA3/IL-26 interaction was inhibited by addition of soluble EphA3-Ig (FIG. 19). It was revealed that the colocalization of the exogenous IL-26 with the cell surface EphA3 is significantly inhibited by the addition of anti-IL-26 mAb clones 31-4 and 69-10 (FIG. 20). Clone 20-3 partially inhibited the colocalization of IL-26 with EphA3, while the commercial available anti-IL-26 mAb 2 clone was shown to hardly inhibit the binding of IL-26 to the cell membrane surface, i.e. EphA3 (FIG. 20).

A similar result was observed in the human breast cancer cell line HCC70, and the exogenous IL-26 was colocalized with the cell surface EphA3 of HCC70 (FIG. 21). The blocking of the EphA3 receptor with anti-EphA3 pAb or soluble EphA3-Ig under EGFR-TKI treatment conditions resulted in significant inhibition of cell proliferation via IL-26 at the same level as the anti-IL-26 antibodies (FIG. 22). These results indicate that the established anti-IL-26 mAb acts on inhibition of binding to EphA3 by binding to the binding domain to EphA3 on IL-26. In other words, these results strongly indicate that EphA3 is a new functional receptor for IL-26 in TNBC.

Furthermore, genetic ablation of EphA3 expression by siRNA resulted in significant inhibition of cell proliferation via IL-26 under EGFR-TKI treatment conditions (FIG. 23). These data suggest that IL-26 does not interact with the known receptor IL-20RA on TNBC cells, but interacts with EphA3.

### Example 5) Activation of EGFR-TKI bypass pathway by interaction of IL-26 with EphA3 receptor

EphA3 contains RTK activity and is converted into a downstream signaling pathway following the participation of its well-known ligand, Ephrin A5 (Janes PW et al., Growth Factors (2014) 32(6): 176-189. and Pasquale EB, Nat Rev Cancer (2010) 10(3): 165-180). The present inventors have found that IL-26 induced dephosphorylation of EphA3 in the E0771 cell line (FIG. 24A). IL-26 also inhibited the suppression of AKT and JNK phosphorylation via gefitinib (lanes 1 to 2 in FIG. 24B). Furthermore, this inhibition due to IL-26 was lost by anti-EphA3 receptor pAb (lane 4 in FIG. 24B) treatment and was comparable to the phosphorylation levels of AKT and JNK obtained with gefitinib alone (lane 1 in FIG. 24B) and in combination with gefitinib and anti-EphA3 receptor pAb (lane 3 in FIG. 24B). Similar results were obtained with the HCC70 cell line (FIGS. 25 and 26). The addition of the exogenous IL-26 to HCC70 cells resulted in dephosphorylation of EphA3, activation of AKT and JNK phosphorylation, but such changes were not observed after stimulation with Ephrin A5, which is a known ligand of EphA3 (FIG. 26). The dephosphorylation of EphA3 and activation of AKT as well as the phosphorylation of JNK due to IL-26 stimulation were inhibited by treatment with anti-EphA3 pAb (FIG. 26). From the above, it was shown that the interaction between IL-26 and EphA3 induces an increase in the phosphorylation of AKT and JNK not only in mouse TNBC but also in human TNBC, thus activating the EGFR-TKI-related bypass pathway and leading to tumor growth. These findings indicate that binding of IL-26 to the cell surface EphA3 RTK of TNBC cells leads to the dephosphorylation of EphA3 and increased the phosphorylation of AKT and JNK, leading to subsequent tumor growth resulted by activation of EGFR-TKI-related bypass pathways.

### Example 6) Inhibition of EGFR- TKI-induced endoplasmic reticulum stress signaling pathway in TNBC cells by interaction of IL-26 with EphA3

To identify key regulators of tumor growth downstream of the IL-26/EphA3 interaction in TNBC, pathway analysis by DNA microarray of HCC70 cells treated with IL-26 and gefitinib was performed (FIG. 27). A significant reduction in the expression levels of endoplasmic reticulum stress-related molecules was evident, indicating involvement of the endoplasmic reticulum stress signaling pathway in HCC70 cells treated with IL-26 and gefitinib. To confirm these in silico findings, roles of IL-26/EphA3 in the EGFR-TKI-associated endoplasmic reticulum stress pathway was examined. To this end, the expression levels of markers of endoplasmic reticulum stress signaling, DDIT3, ATF3, ATF4, in TNBC cells treated with IL-26 and gefitinib were analyzed. The mRNA expression levels of DDIT3, ATF3 and ATF4 were significantly increased by gefitinib treatment, whereas they were clearly reduced by treating IL-26 with gefitinib (FIG. 28). Similarly, in the other TNBC cell lines, MDA-MB-468 and E0771, the mRNA expression levels of DDIT3, ATF3 and ATF4 were significantly increased by gefitinib treatment and significantly decreased by IL-26 treatment with gefitinib (FIG. 29).

Immunocytochemical staining revealed a marked increase in expression of DDIT3 at the protein level by gefitinib treatment and a significant decrease in expression by IL-26 treatment with gefitinib (FIG. 30). The gefitinib treatment significantly reduced mitochondrial activity, whereas IL-26 treatment with gefitinib significantly inhibited the reduction in mitochondrial activity (FIG. 31). On the other hand, a decrease in the mRNA expression level of DDIT3 by IL-26 treatment with gefitinib was significantly inhibited by treatment with AKT inhibitor alone, JNK inhibitor alone, or EphA3 pAb, but this inhibition was more obvious by treatment with both AKT and JNK inhibitors and comparable to anti-IL-26 mAb treatment (upper figure in FIG. 32). Similarly, also in the immunocytochemical staining, the decrease in expression of DDIT3 at the protein level by treatment with IL-26 together with gefitinib was significantly inhibited by treatment with an AKT inhibitor, a JNK inhibitor, EphA3 pAb, and an anti-IL-26 mAb (lower figure of FIG. 32). Furthermore, the inhibitory effect of reduction in mitochondrial activity due to treatment with IL-26 together with gefitinib was significantly inhibited by treatment with the AKT inhibitor alone, the JNK inhibitor alone, or EphA3 pAb, but this inhibition was more obvious by treatment with both AKT and JNK inhibitors and comparable to anti-IL-26 mAb treatment (FIG. 33).

Thus, both the AKT and JNK inhibitors partially increased the expression level of DDIT3 and mitochondrial membrane depolarization in IL-26 treated HCC70 cells in the presence of gefitinib. The combination of the AKT inhibitor and the JNK inhibitor almost completely reversed the inhibitory effect of IL-26 on gefitinib-induced DDIT3 expression and the mitochondrial membrane depolarization to administration levels of gefitinib alone. Both anti-EphA3 pAb and anti-IL-26 mAb reversed the effect of IL-26 on HCC70 cells treated with gefitinib, and the inhibitory effect of anti-IL-26 mAb was stronger than that of anti-EphA315 pAb. These results indicated that the phosphorylation of AKT and JNK plays an important role in endoplasmic reticulum stress response and induces cell survival and proliferation in TNBC treated with IL-26 in the presence of gefitinib.

Furthermore, gefitinib treatment significantly increased the mRNA expression levels of other endoplasmic reticulum stress-related cytokines/chemokines, including IL-6, IL-8 and CXCL2, in HCC70, MDA-MB-468 and E0771 cells, whereas IL-26 treatment with gefitinib significantly reduced the expression levels thereof (FIG. 34). These results also showed that IL-26 reduced endoplasmic reticulum stress induced in TNBC by EGFR-TKI.

### Example 7) Action of IL-26 on promoting TNBC tumor growth in in vivo EGFR-TKI-treated mouse model

In order to confirm the above in vitro obtained findings in vivo, an in vivo tumor growth assay of TNBC after gefitinib treatment was performed. A mouse syngeneic transplantation model of TNBC using hIL-26Tg mice was established. E0771 tumors grew in hIL-26Tg and its littermates control mice (tumor photographs in the upper panel of FIGS. 35A and 35B) and tumor growth in control mice was suppressed after gefitinib treatment (tumor photographs in the upper panel of FIGS. 35A and 35B). On the other hand, tumor suppression by gefitinib was significantly inhibited in hIL26-Tg mice (the following tumor photographs in the lower panels of FIG. 35A and FIG. 35B). These data indicate that IL-26 does not directly affect tumor growth and plays an important role in TNBC EGFR-TKI resistance.

To further confirm the inhibitory effect of IL-26 on tumor growth inhibition via gefitinib, a tumor growth assay was performed by administering anti-IL-26 mAb to hIL-26Tg mice transplanted with TNBC. As shown in FIG. 36A and FIG. 36B, tumor growth in TNBC transplanted into hIL-26Tg mice administered with anti-IL-26 mAb and gefitinib was significantly reduced compared to that seen with gefitinib alone (FIG. 36A). Treatment with anti-IL-26 mAb alone had no effect on tumor growth (not illustrated). From the above, these results strongly suggest that IL-26 brings about EGFR-TKI resistance of TNBC and that neutralization of IL-26 due to anti-IL-26 mAb effectively restores the tumor inhibitory effect of EGFR-TKI therapy.

The present inventors have previously reported that CD4+ effector T-cells infiltrating inflammatory lesions secrete IL-26, resulting in exacerbation of cGVHD or psoriasis (Ohnuma K et al. (2015); Itoh T et al. (2019), supra). Next, the cells from which IL-26 was generated in the tumor microenvironment (TME) were determined. Immunohistochemical assays of tumors developed in the tumor transplantation model of hIL-26Tg mice were performed. CD4+ T-cells and F4/80+ macrophages in the TME expressed IL-26 (FIG. 37 and FIG. 38), whereas CD8+ T-cells did not express IL-26 (not illustrated). In the TME of hIL-26Tg mice administered gefitinib, PECAM-positive vascular endothelial cells were significantly increased compared to control mice (FIG. 39), and in response to KC and MIP-2 (FIG. 34) induced by endoplasmic reticulum stress signaling, neutrophil levels were increased in the TME (FIG. 40). In addition, cells with phosphorylated AKT and JNK were increased in hIL-26Tg mice receiving gefitinib (FIGS. 41 and 42). These data indicate that TNBC cells are exposed to CD4+ T-cells infiltrated with TME and IL-26 secreted by macrophages, and acquire resistance to EGFR-TKI therapy in association with the phosphorylation of AKT and JNK of ER stress response.

### Example 8) Expression of IL-26 in tumor infiltrating lymphocytes of clinical specimens of TNBC and expression of EphA3 in tumor cells

In order to confirm whether IL-26 producing immune cells are present in clinical tissues of TNBC patients, immunohistochemical staining of clinical specimens was performed. Similar to the results of tumor samples obtained from in vivo models using hIL26-Tg mice (FIG. 37), IL-26 was clearly detected in tumor infiltrating lymphocytes (TIL) in the TME of human clinical tissue samples (FIG. 44 A). Furthermore, EphA3 was expressed in TNBC cells of human clinical tissue samples (FIG. 44B). Data from these clinical samples suggests that the exposure of TNBC cells to IL-26 secreted by TILs and that EGFR-TKI resistance is indeed induced by the interaction of IL-26 and EphA3 in TNBC in the patients, based on the findings from the above experiments.

Based on the above experimental results, FIG. 45 shows a schematic diagram of the inhibitory effect of IL-26 on tumor suppression via EGFR-TKI in TNBC cells. Through binding of ligands such as EGF, EGFR is activated by transautophosphorylation of receptors and recruitment of signaling proteins, leading to involvement of downstream signaling cascades including ERK, JNK, PI3K/AKT, which results in cell survival and proliferation (Wee P et al., Cancers (Basel) (2017) 9(5)). EGFR-TKI inactivates downstream signaling including ERK, JNK, and AKT by inhibiting EGFR phosphorylation, and inhibits EGF-mediated cell proliferation and survival (Wee P et al. (2017), and Hammerman PS et al., Clin Cancer Res (2009) 15(24): 7502-7509). In TNBC cells, IL-26 binds to EphA3 on the cell surface and causes dephosphorylation of EphA3 while phosphorylating downstream factors AKT and JNK. This IL-26/EphA3 mediated EGFR-TKI bypass pathway recruitment was subsequently found to cause cell proliferation and tumor growth.

The results showed that IL-26 activated the EGFR-TKI-related bypass pathway and that the blocking of IL-26 overcomes EGFR-TKI resistance of TNBC. In addition, the inventors have discovered that EphA3 is a new functional receptor for IL-26 in TNBC and that binding of IL-26 leads to cell proliferation signaling events that are located downstream of EphA3. These findings reveal the importance of IL-26 in EGFR-TKI resistance in TNBC, which is the basis of new therapeutic strategies for TNBC and other EGFR-TKI-resistant cancers such as NSCLC and CRC using a combination of anti-IL-26 mAb and anti-EGFR agents.

### Example 9) Acquisition of humanized 69-10 antibodies

### (1) Sequence of the variable region of the mouse 69-10 monoclonal antibody

To obtain the entire VH and VL sequences of the mouse 69-10 monoclonal antibody including a signal peptide region, mouse 69-10 hybridoma cells were grown in GIT medium (FUJIFILM Wako Pure Chemical Corporation, Osaka, Japan) with 5% BriClone (Boca Scientific, Westwood, MA) in a 37°C, 7.5% CO₂ incubator. Total RNA was extracted from approximately 10⁷ hybridoma cells using the TRI Reagent (Molecular Research Center, Cincinnati, OH) following the supplier's protocol. cDNA primed with oligo dT was synthesized using the SMARTer RACE cDNA amplification kit (Clontech, Mountain View, CA) according to the Supplier's protocol. The 69-10 heavy chain and light chain variable region cDNAs were amplified by polymerase chain reaction (PCR) with Phusion DNA polymerase (Thermo Fisher Scientific, Waltham, MA) using a Universal Primer A Mix provided in the SMARTer RACE cDNA Amplification Kit as 3' primer and 5' primer that anneal to mouse gamma-1 and kappa chains, respectively. The sequence of the 3' primer for PCR amplification of the heavy chain variable region (VH) was 5'-GCCAGTGGATAGACAGATGG-3' (SEQ ID NO:46). The sequence of the 3' primer for PCR amplification of the light chain variable region (VL) was 5'-GATGGATACAGTTGGTGCAGC-3' (SEQ ID NO:47). Amplified VH and VL cDNA was cloned into the pJet 1.2/blunt vector (Thermo Fisher Scientific) for sequencing. Several heavy chain and light chain clones were sequenced and unique sequences homologous to typical mouse heavy chain and light chain variable regions were identified.

### 2) Construction of expression vector of chimeric 69-10 IgG1 antibody

The gene encoding the mouse 69-10VH (Ch69-10VH) was designed as an exon containing a splice donor signal after the coding region, a SpeI site at the 5' end, and a HindIII site at the 3' end. Similarly, the gene encoding the mouse 69-10VL (Ch69-10VL) was designed as an exon containing a splice donor signal after the coding region, a NheI site at the 5' end, and an EcoRI site at the 3' end. Codons rarely used in mammals were replaced by corresponding codons frequently used in the Ch69-10VH and VL genes. Furthermore, potential splice donor signals of the coding region were eliminated so as not to alter the amino acid sequence.

After digestion of the Ch69-10VH and VL genes with SpeI and HindIII (for VH) or NheI and EcoRI (for VL), Ch69-10VH and VL gene fragments were cloned into mammalian expression vectors carrying human gamma-1 and kappa constant regions for the production of a chimeric 69-10 IgG1/kappa antibody (Ch69-10-IgG1). A schematic diagram of the obtained expression vector is shown in FIG. 47.

### 3) Humanized V gene design

The design of humanized 69-10VH and VL amino acid sequences is described in Kakushita et al., (2005) Methods; 36: 69-83. A three-dimensional molecular model of the mouse 69-10 variable region was constructed using a proprietary algorithm from the company JN Biosciences (Mountain View, CA).

The Human VH sequences homologous to the 69-10VH framework were retrieved in the GenBank database, and a VH sequence encoded by the human AY781820 cDNA (AY781820VH) (GenBank Accession No.) (Dahlke, I. et al. (2006) J. Allergy Clin.Immunol.; 117: 1477-1483) was selected as an acceptor for humanization. There were no somatic hypermutations in the framework regions in AY781820VH. The CDR sequence of 69-10VH was first grafted at the corresponding position of AY781820VH. The amino acid residues of AY781820VH were then replaced with the corresponding residues of mouse 69-10VH at framework positions 66 and 93. The amino acid sequences of the resulting humanized VH, Hu69-10VH1, and 69-10 and AY781820VH sequences are shown in FIG. 48.

Based on a homology search with the 69-10VL framework sequence, the human V region encoded by the KU760971 cDNA (KU760971VL) (GenBank Accession No.) (Jardine, J. G. et al., (2016) Science; 351: 1458-1463) was selected as an acceptor for humanization. KU760971VL had no somatic hypermutations in the framework regions. The CDR sequences of 69-10VL was transplanted into the corresponding location of KU760971VL. At framework amino acid residue position 70, the corresponding residue of mouse 69-10VL was used in humanization. The amino acid sequence of the resulting humanized VL (Hu69-10VL1) is shown in FIG. 49 together with the 69-10 and KU760971VL sequences.

While a lysine residue at position 70 in mouse 69-10VL was predicted to play an important role in the formation of the antigen binding site, a more detailed analysis of the 69-10 variable region molecular model suggested that an aspartic acid residue at the corresponding position in the human receptor KU760971VL could be accepted for proper formation of the antigen binding site. Accordingly, a second humanized VL (Hu69-10VL2) was designed and the lysine residue at position 70 of Hu69-10VL1 was further replaced with an aspartic acid residue. The amino acid sequence of Hu69-10VL2 is shown in FIG. 49.

### 4) Construction of expression vectors for humanized 69-10 IgG1 antibodies

The gene encoding Hu69-10VH1 was designed as an exon containing a signal peptide, a SpeI site at the 5' end, a HindIII site at the 3' end, and a splice donor signal (FIG. 50). The signal peptide sequence and splice donor signal of the Ch69-10VH gene were used for Hu69-10VH1.

Each gene encoding Hu69-10VL1 and VL2 was similarly designed as an exon containing a signal peptide, a NheI site at the 5' end, an EcoRI site at the 3' end, and a splice donor signal (FIGS. 51 and 52). The signal peptide sequence and splice donor signal of the Ch69-10VL gene were used for Hu69-10VL1 and VL2.

The VH gene of pCh69-10 was first replaced by Hu69-10VH1 between the SpeI and HindIII sites. Next, the VL gene of this intermediate expression vector was replaced with HuVL1 and HuVL2 between the NheI site and the EcoRI site to generate pHu69-10A and pHu69-10B, respectively. Schematic structural diagrams of pHu69-10A and pHu69-10B are similar to FIG. 47. pHu69-10A expresses Hu69-10A-IgG1/kappa antibody (Hu69-10A-IgG1). pHu69-10B expresses Hu69-10B IgG1/kappa antibody (Hu69-10B-IgG1).

### 5) Expression and purification of Ch69-10-IgG1, Hu69-10A-IgG1 and Hu69-10B-IgG1 antibodies

In order to obtain a cell line stably producing each of Ch69 -10- IgG 1, Hu69-10A-IgG1 and Hu69-10B-IgG1 antibodies, the expression vectors pCh69-10, pHu69-10A and pHu69-10B were individually introduced into chromosome of the Chinese hamster ovary cell line CHO-K1 (ATCC, Manassas, VA). CHO-K1 cells were grown in SFM4CHO medium (HyClone, Logan, UT) at 37°C in a 7.5% CO₂ incubator. Stable transfection into CHO-K1 cells was performed by electroporation. Prior to transfection, each expression vector was cut with FspI to be linear. Linear plasmid of 20 µg was transfected into to 2.5×10⁶ cells, suspended in SFM4CHO medium, and the cells were appropriately diluted before seeding in 96-well plates. After 48 hours, 10 µg/ml of puromycin was added to isolate stable transfectants.

About 10 days after the start of selection, antibody production in the culture supernatant of the transfectants in 96-well plates was assayed by sandwich ELISA. ELISA plates were coated overnight with 100 µl/well of 1/2,000 diluted goat anti-human IgG, Fcy-specific polyclonal antibody (Jackson ImmunoResearch, West Grove, PA) in PBS, washed with a wash buffer (PBS with 0.05% Tween (registered trademark) 20) and blocked with 200 µl/well of ELISA buffer (PBS with 2% skim milk and 0.05% Tween (registered trademark) 20). After washing with wash buffer, 100 µl/well of the test sample appropriately diluted with ELISA buffer was applied to the ELISA plate. Appropriate humanized IgG1/kappa antibodies were used as standards. ELISA plates were incubated for 1 hour at room temperature and washed with the wash buffer before adding 100 µl/well of 1/2,000 diluted HRP-conjugated goat anti-human kappa chain polyclonal antibody (Bethyl Laboratories, Montgomery, TX) to detect bound antibodies. After incubation at room temperature for 0.5 hours and washing with the washing buffer, 100 µl/well of ABTS substrate (Sigma-Aldrich, St. Louis, MO) was added for color development, and 100 µl/well of 2% oxalic acid was added to stop the color development. The absorbance was measured at 405 nm.

CHO-K1 stable transfectants producing each of Ch69-10-IgG1 (clone 1H8), Hu69-10A-IgG1 (clone 1G10), and Hu69-10B-IgG1 (clone 1G5) at high levels were grown in SFM4CHO (500 ml) in roller bottles until cell viability became < 50%. After centrifugation and filtration, the culture supernatant was loaded onto a Protein A column (HiTrap MabSelect SuRe, GE Healthcare, Piscataway, NJ). The column was washed with PBS before the antibody was eluted with 0.1 M Glycine-HCl buffer (pH 3.0) containing 0.1 M NaCl. After neutralization with 1 M Tris-HCl (pH 8.0), the buffer of eluted antibody was exchanged into PBS by dialysis. The antibody concentration was determined by measuring the absorbance at 280 nm (1.4 OD = 1 mg/ml). A yield was 45 mg for Ch69-10-IgG1 (3.9 mg/ml), 86 mg for Hu69-10A-IgG1 (5.8 mg/ml), and 21 mg for Hu69-10B-IgG1 (2.5 mg/ml).

The purified Ch69-10-IgG1, Hu69-10A-IgG1, and Hu69-10B-IgG1 antibodies, and the mouse 69-10 antibody were analyzed by SDS-PAGE. Analysis under reducing conditions showed that each of these antibodies was composed of a heavy chain with a molecular weight of about 50 kDa and a light chain with a molecular weight of about 25 kDa. Purity of each antibody appeared to be ≥ 95%.

Reliability of heavy chain and light chain produced in CHO-K1 stable transfectants expressing Ch69-10-IgG1 (clone 1H8), Hu69-10A-IgG1 (clone 1G10), and Hu69-10B (clone 1G5) was confirmed by cDNA sequencing. Total RNA was extracted from these cells using TRI reagent. cDNA primed with oligo dT was synthesized using ProtoScript II First Strand cDNA synthesis kit (New England Biolabs, Ipswich, MA) according to manufacturer's protocol. Primers used for PCR and sequencing are as follows.
CMV2: GAACCGTCAGATCGCCTGGAGACG (SEQ ID NO:48)
JNT026: TGAAAGATGAGCTGGAGGAC (SEQ ID NO:49)
JNT082: CTTTCTTGTCCACCTTGGTG (SEQ ID NO:50)
JNT097: GCTGTCCTACAGTCCTCAG (SEQ ID NO:51)
JNT098: ACGTGCCAAGCATCCTCG (SEQ ID NO:52)
KT437: CTGAGGTCACATGCGTGG (SEQ ID NO:53)

The coding region of a gamma heavy chain was amplified by PCR using CMV2 and JNT098 as primers, or amplified by two separate PCR reactions using CMV2 and JNT082 as the first primer set and JNT097 and JNT098 as the second primer set together with Phusion DNA polymerase (Thermo Fisher Scientific). PCR fragments were gel-purified and sequenced using CMV2, JNT097, JNT098, and KT437 as primers. Similarly, the coding region of a kappa light chain was amplified using CMV2 and JNT026. Gel purified DNA fragments were sequenced using CMV2 and JNT026 as primers. The nucleotide sequences of the regions encoding the heavy chain and light chain of the obtained Ch69-10-IgG1, Hu69-10A-IgG1, and Hu69-10B-IgG1 completely corresponded to the amino acid sequences encoded by the pCh69-10 vector, the pHu69-10A vector, and the pHu69-10B vector. The nucleotide sequences of the heavy chains encoded by the pHu69-10A vector and the pHu69-10B vector are shown in FIG. 53, the nucleotide sequence of the light chain encoded by the pHu69-10A vector is shown in FIG. 54, and the nucleotide sequence of the light chain encoded by the pHu69-10B vector is shown in FIG. 55.

### 6) Characterization of humanized 69-10 IgG1 antibodies

The antigen binding of purified Ch69-10-IgG1, Hu69-10A-IgG1, and Hu69-10B-IgG1 was analyzed by ELISA. ELISA plates were coated overnight at 4°C with the addition of 0.5 µg/ml human IL-26 (recombinant human IL-26/AK155 monomer, Cat # 1375-IL/CF, R&D Systems, Minneapolis, MN) at 100 µl/well in 0.2 M sodium carbonate buffer (pH 9.4). Wells were washed with the wash buffer and blocked with the ELISA buffer. Subsequently, 100 µl/well of test antibody (mouse 69-10, Ch69-10-IgG1, Hu69-10A-IgG1, or Hu69-10B-IgG1) serially diluted 2-fold in RPMI medium starting at 5 µg/ml was added to measure a binding with IL-26. After incubation at room temperature for 1 hour and washing the plates with a wash buffer, a 1/2,000-diluted HRP-conjugated goat anti-human kappa chain polyclonal antibody (Bethyl Laboratories) (for Ch69-10-IgG1, Hu69-10A-IgG1 and Hu69-10B-IgG1) or a 1/2,000-diluted HRP-labeled goat anti-mouse kappa chain polyclonal antibody (Bethyl Laboratories) (for mouse 69-10) in ELISA buffer was added at 100 µl/well to detect bound antibodies. After incubation at room temperature for 30 min and washing with the wash buffer, 100 µl/well of ABTS substrate (Sigma Aldrich) was added to start color development and 100 µl/well of 2% oxalic acid was added to stop the color development. The absorbance was measured at 405 nm.

The results of the ELISA analysis are shown in FIG. 56. EC50 values calculated using GraphPadPrism (GraphPad Software, San Diego, CA) were 340 ng/ml for mouse 69-10, 299 ng/ml for Ch69-10-IgG1, 304 ng/ml for Hu69-10A-IgG1, and 396 ng/ml for Hu69-10B-IgG1.

### Example 10) Inhibitory Effect of IL-26 on human large intestine cancer line COLO205 by humanized 69-10 IgG1 antibody

The expression of ICAM-1 on the cell membrane of COLO205 was enhanced by the exogenous IL-26 stimulation, and both the mouse anti-human IL-26 neutralizing mAb m69-10 and the humanized anti-IL-26 neutralizing mAb h69-10A and h69-10B partially inhibited the enhanced expression of ICAM-1 (* p < 0.01 compared to the corresponding control human IgG-added group). The goat anti-human IL-26 polyclonal antibody from R&D Systems almost completely inhibited the activity of IL-26.

In the test using COLO205 cells, none of the anti-human IL-26 monoclonal antibodies could completely inhibit the activity of IL-26 in one clone, but all of the antibodies inhibited the ICAM-1 expression enhancing action of COLO205 due to IL-26 in an antibody dose-dependent manner. At antibody doses of 2 µg/mL and 5 µg/mL, it was shown that the humanized antibodies h69-10A and h69-10B have a significantly higher inhibitory effect on IL-26 than the original mouse antibody m69-10 (FIG. 57).

### Example 11) Inhibitory Effect of IL-26 on human umbilical vein endothelial cell HUVECs by humanized 69-10 IgG1 Antibody

The present inventors have found that proliferation and tube formation of vascular endothelial cells are remarkably enhanced as a function of IL-26. Therefore, in addition to an evaluation system using COLO205 cells, the neutralizing activity of the humanized anti-IL-26 antibody was evaluated using human umbilical vein endothelial cells HUVECs. As shown in FIG. 58, proliferation of HUVECs was promoted by IL-26 stimulation. In this evaluation system using HUVECs, the inhibitory effect of IL-26 in the monoclonal antibody 69-10 1 clone was more obviously observed than in the evaluation system using COLO205 cells, and it was shown that the level was not inferior to that of the commercially available goat anti-human IL-26 polyclonal antibody. At an antibody dose of 3 µg/mL, it was shown that the humanized antibodies h69-10A and h69-10B have a significantly higher inhibitory effect on IL-26 than the original mouse antibody m69-10 (FIG. 58).

### Example 12) Inhibitory effect of IL-26 on human TNBC cell line HCC70 due to humanized 69-10 IgG1 antibody

suppression of cell proliferation of HCC70 due to gefitinib was significantly inhibited by the addition of the exogenous IL-26. All mouse-type anti-human IL-26 neutralizing mAb m69-10, the humanized anti-IL-26 neutralizing mAb h69-10A, and the commercially available goat anti-human IL-26 polyclonal antibodies reversed the inhibitory effect of IL-26 on gefitinib-induced suppression of cell proliferation in an antibody dose-dependent manner. At antibody doses of 5 µg/mL and 20 µg/mL, it was shown that the humanized antibody h69-10A has a significantly higher inhibitory effect on IL-26 than the original mouse antibody m69-10.

From these results, it was shown that the humanized anti-IL-26 antibody newly established by the present inventors has higher binding activity to IL-26 than the original mouse antibody m69-10, and the inhibitory effect of IL-26 is significantly higher than that of the original mouse antibody m69-10 in any test for evaluating the neutralizing activity of IL-26 using any of COLO205 cells, HUVEC, and HCC70 cells in vitro.

### Example 13) Therapeutic Effect of humanized 69-10 IgG1 antibody in heterologous chronic GVHD model

Since a novel humanized anti-IL-26 monoclonal antibody exhibiting excellent neutralizing activity could be obtained by an in vitro neutralizing activity evaluation test, therapeutic effects in animal experiments on refractory immune disorders and cancer were examined using this antibody.

The present inventors established a heterogeneous chronic GVHD model in which NOG mice, severe immunodeficient mice, were irradiated with sublethal doses of radiation, followed by transplantation of naive human umbilical cord blood mononuclear cells, in which most of the contained T-cells were not sensitized with antigen, and revealed that IL-26 enhances fibroblast collagen production and is crucial for lung fibrosis (J Immunol. 2015; 194(8): 3697-712). Therefore, using the same model, administration of a novel humanized anti-IL-26 monoclonal antibody h69-10A established by the present inventors was started from day 28 after transplantation when mild clinical signs/symptoms of GVHD such as hair disorder and weight loss began to be observed, and the therapeutic effect on chronic GVHD was examined.

In the control human IgG administration group, body weight loss started from the fourth week of transplantation, and all mice died from the fifth week to the ninth week (data not shown). On the other hand, in the mouse anti-human IL-26 monoclonal antibody m69-10 and humanized anti-IL-26 monoclonal antibody h69-10A administration group, body weight loss was clearly reduced, and the number of survival days was significantly prolonged (data not shown). As a result of analyzing engraftment of donor (human) lymphocytes in the recipient (mouse) body, engraftment to the same extent as that in the control human IgG administration group was observed even in the IL-26 antibody administration group (data not shown). From this, it was shown that a remarkable prolongation of the number of days of survival and reduction of body weight loss due to the administration of the IL-26 antibody were not caused by inhibiting the engraftment of donor lymphocytes.

Next, a histopathological evaluation of GVHD target organs was performed. In the group to which the control human IgG was administered after umbilical cord blood mononuclear cell transplantation, hair loss, skin atrophy, distortion of spine, decrease in momentum, and the like were observed from appearance at the eighth week of transplantation. In terms of skin histology, inflammatory cell infiltration, thickening of epidermal layer, skin atrophy, fat reduction, and hair follicle falling were observed. In terms of lungs, expansion of subepithelial fibrous tissue, inflammatory cell infiltration around bronchus, and blockage of bronchiole were observed. In terms of liver, perivascular infiltration by inflammatory cells was observed in the tissue surrounding the bile duct (data not shown). In the large intestine, which is one of the main target organs of acute GVHD, no remarkable lesion findings were observed in this model (data not shown). On the other hand, in the group to which the IL-26 monoclonal antibody 69-10 was administered, hair disorder was slightly suppressed even in appearance, and inflammatory cell infiltration around bronchioles and proliferation of collagen fibers were also clearly reduced, indicating that the level was close to that of the non-GVHD onset group (FIG. 60). It was shown that the humanized antibody h69-10A had a higher therapeutic effect than the original mouse antibody m69-10 (FIG. 60).

These results suggested that the novel humanized anti-IL-26 neutralizing monoclonal antibody established by the present inventors is also useful as a novel therapeutic approach for various chronic inflammatory diseases including chronic GVHD, autoimmune diseases, and cancers.

## Claims

1. A pharmaceutical composition for use in combination with an anticancer drug, the pharmaceutical composition comprising, as an active ingredient, a drug that reverses resistance to the anticancer drug due to IL-26 in a cancer.

2. The pharmaceutical composition according to claim 1, wherein the cancer is breast cancer.

3. The pharmaceutical composition according to claim 2, wherein the breast cancer is a human epidermal growth factor receptor 2 (HER2) negative cancer.

4. The pharmaceutical composition according to claim 2 or 3, wherein the cancer is breast cancer that is estrogen receptor (ER) negative, progesterone receptor (PR) negative, and human epidermal growth factor receptor 2 (HER2) negative (triple negative breast cancer (TNBC)).

5. The pharmaceutical composition according to any one of claims 1 to 4, wherein the cancer is a cancer expressing EphA3.

6. The pharmaceutical composition according to any one of claims 1 to 5, wherein the cancer is a cancer having a low sensitivity to the anticancer drug or exhibiting resistance to the anticancer drug.

7. The pharmaceutical composition according to claim 6, wherein the low sensitivity or the resistance to the anticancer drug is caused by IL-26 or phosphorylation of AKT and/or JNK due to IL-26.

8. The pharmaceutical composition according to claim 7, wherein the low sensitivity or the resistance to the anticancer drug is due to an interaction between IL-26 and EphA3.

9. The pharmaceutical composition according to claim 7 or 8, wherein IL-26 is produced by CD4+ T-cells or macrophages.

10. The pharmaceutical composition according to any one of claims 1 to 9, wherein the anticancer drug is a tyrosine kinase inhibitor.

11. The pharmaceutical composition according to claim 10, wherein the tyrosine kinase inhibitor is an epidermal growth factor receptor (EGFR) tyrosine kinase inhibitor.

12. The pharmaceutical composition according to claim 11, wherein the EGFR tyrosine kinase inhibitor is a drug selected from gefitinib, erlotinib, afatinib maleate, osimertinib mesylate, and dacomitinib hydrate.

13. The pharmaceutical composition according to any one of claims 1 to 12, wherein the drug that reverses the resistance to the anticancer drug due to IL-26 is a drug that inhibits or reduces the interaction between ephrintype-Areceptor3 (EphA3) and IL-26.

14. The pharmaceutical composition according to claim 13, wherein the drug that inhibits or reduces the interaction between EphA3 and IL-26 is an antibody that inhibits the binding between EphA3 and IL-26.

15. The pharmaceutical composition according to any one of claims 1 to 12, wherein the drug that reverses the resistance to the anticancer drug due to IL-26 is an anti-IL-26 antibody.

16. The pharmaceutical composition according to claim 15, wherein the anti-IL-26 antibody is an antibody produced from a hybridoma deposited as Accession Nos. NITEP-02577, NITEP-02578, NITEP-02579, or NITEP-02580, an antibody having complementarity determining regions of a heavy chain and a light chain thereof, or an antibody having variable regions of a heavy chain and a light chain thereof.

17. The pharmaceutical composition according to claim 15, wherein the anti-IL-26 antibody is an antibody containing a heavy chain variable region including an amino acid sequence described in SEQ ID NO:2 or 12 and a light chain variable region including an amino acid sequence described in SEQ ID NO:4 or 14, respectively, or an antibody having complementarity determining regions of a heavy chain and a light chain thereof.

18. The pharmaceutical composition according to any one of claims 1 to 12, wherein the drug that reverses the resistance to the anticancer drug due to IL-26 is an anti-EphA3 antibody.

19. The pharmaceutical composition according to any one of claims 14 to 18, wherein the antibody is a humanized antibody or a fragment thereof.

20. The pharmaceutical composition according to any one of claims 1 to 12, wherein the drug that reverses the resistance to the anticancer drug due to IL-26 is a soluble EphA3.

21. The pharmaceutical composition according to any one of claims 1 to 20, being an agent for improving a sensitivity of cancer cells to the anticancer drug.

22. The pharmaceutical composition according to any one of claims 1 to 21, being a proliferation inhibitor for cancer cells.

23. The pharmaceutical composition according to claim 21 or 22, wherein the cancer cells have a low sensitivity to the anticancer drug or exhibit the resistance to the anticancer drug.

24. The pharmaceutical composition according to any one of claims 1 to 23, wherein the pharmaceutical composition is administered simultaneously with the anticancer drug, after administration of the anticancer drug, or before administration of the anticancer drug.

25. A kit for treating cancer, comprising (i) an anticancer drug and (ii) a drug that reverses resistance to an anticancer drug due to IL-26.

26. A pharmaceutical composition for treating cancer, comprising: (i) an anticancer drug; and (ii) a drug that reverses resistance to the anticancer drug due to IL-26.

27. The pharmaceutical composition according to claim 26, wherein (i) the anticancer drug and (ii) the drug that reverses the resistance to the anticancer drug due to IL-26 are contained in the same preparation.

28. A method for improving responsiveness to an anticancer drug in cancer patients having a low sensitivity to the anticancer drug or exhibiting resistance to the anticancer drug, the method comprising inhibiting IL-26 in a cancer microenvironment of the cancer patients.

29. The method according to claim 28, comprising inhibiting or reducing the interaction between EphA3 and IL-26 by inhibiting IL-26.

30. A method for treating cancer in cancer patients having a low sensitivity to an anticancer drug or exhibiting resistance to an anticancer drug, the method comprising inhibiting IL-26 of the cancer patients and administering an anticancer drug to the cancer patients.

31. The method according to claim 30, comprising inhibiting or decreasing the interaction between EphA3 and IL-26 by inhibiting or decreasing IL-26.

32. An antibody or an antigen-binding fragment thereof, wherein a heavy chain variable region (VH) has an amino acid sequence with 80% or more of identity to an amino acid sequence described in SEQ ID NO:25, and a light chain variable region (VL) has an amino acid sequence with 80% or more of identity to an amino acid sequence described in SEQ ID NO:30 or an amino acid sequence with 80% or more of identity to an amino acid sequence described in SEQ ID NO:35, and the antibody binds to IL-26.

33. An antibody or an antigen-binding fragment thereof, wherein the VH has the amino acid sequence described in SEQ ID NO:25 and the VL has the amino acid sequence described in SEQ ID NO:30 or the amino acid sequence described in SEQ ID NO:35.

34. The antibody or the antigen-binding fragment thereof according to claim 33, wherein a heavy chain has an amino acid sequence described in SEQ ID NO:37 at positions 20 to 472, and a light chain has an amino acid sequence described in SEQ ID NO:39 at positions 21 to 234, or an amino acid sequence described in SEQ ID NO:41 at positions 21 to 234.

35. A nucleic acid molecule comprising a polynucleotide encoding the antibody or the antigen-binding fragment thereof according to any one of claims 32 to 34.

36. The nucleic acid molecule according to claim 35, comprising a polynucleotide encoding a VH having a base sequence described in SEQ ID NO:24 and a polynucleotide encoding a VL having a base sequence described in SEQ ID NO:29 or SEQ ID NO:34.

37. The nucleic acid molecule according to claim 36, comprising a polynucleotide encoding a heavy chain having a base sequence described in SEQ ID NO:36 at positions 58 to 1416 and a polynucleotide encoding a light chain having a base sequence described in SEQ ID NO:38 at positions 61 to 702 or SEQ ID NO:34 at positions 61 to 702.

38. A vector comprising the nucleic acid molecule according to any one of claims 35 to 37.

39. A host cell having the vector according to claim 38.

40. A method for producing the antibody or the antigen-binding fragment thereof according to any one of claims 32 to 34, comprising culturing the host cell according to claim 39.

41. A pharmaceutical composition comprising the antibody or the antigen-binding fragment thereof according to any one of claims 32 to 34 as an active ingredient.

42. The pharmaceutical composition according to claim 19, wherein the anti-IL-26 antibody is the antibody or the antigen-binding fragment thereof according to any one of claims 32 to 34.

43. The pharmaceutical composition according to claim 41, being a therapeutic agent or preventive agent for refractory immune disorders or cancer.
